(19) Europäisches Patentamt
European Patent Office
Office européen des brevets

(11) **EP 4 699 542 A1**

(12) **EUROPEAN PATENT APPLICATION**
published in accordance with Art. 153(4) EPC

(43) Date of publication:
**25.02.2026 Bulletin 2026/09**

(21) Application number: **24792162.0**

(22) Date of filing: **20.04.2024**

(51) International Patent Classification (IPC):
**A61B 7/04** $^{(2006.01)}$

(52) Cooperative Patent Classification (CPC):
**A61B 7/04**

(86) International application number:
**PCT/CN2024/088985**

(87) International publication number:
**WO 2024/217579 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.04.2023 JP 2023070122**

(71) Applicant: **Silicon & System Co., Limited
San Po Kong, Kowloon, Hong Kong (HK)**

(72) Inventors:
• **TADAKI, Yoshitaka
  Musashimurayama-shi Tokyo 208-0023 (JP)**
• **UMEMURA, Shin-ichiro
  Sendai-shi Miyagi 984-0053 (JP)**
• **OGAYA, Kaoru
  Musashimurayama-shi Tokyo 208-0023 (JP)**
• **TAKEMOTO, Yoshiaki
  Musashimurayama-shi Tokyo (JP)**

(74) Representative: **Murgitroyd & Company
165-169 Scotland Street
Glasgow G5 8PL (GB)**

(54) **ELECTRONIC STETHOSCOPE**

(57) The present disclosure introduces an electronic stethoscope capable of achieving a high Signal-to-Noise Ratio (SN ratio) and enhanced auscultatory accuracy. The device comprises a housing 92b, within which are positioned audible range sensors $X_{v2}$, $X_{v4}$, and $X_{w1}$, exhibiting a mechanical resonance frequency within the audible range, and incorporating a drum-shaped detection component designed to detect characteristic audible range signals. Additionally, a signal processing circuit 97, situated within the storage cavity, processes signals outputted from the audible range sensors $X_{v2}$, $X_{v4}$, and $X_{w1}$.

FIG. 16

## Description

### CROSS-REFERENCE TO RELATED APPLICATIONS

This application is a bypass continuation application of PCT application no.: PCT/CN2024/088985. This application claims priorities from PCT Application PCT/CN2024/088985, filed April 20, 2024, and from Japanese patent application 2023070122, filed April 21, 2023, the contents of which are incorporated herein in the entirety by reference.

### TECHNICAL FIELD

The present disclosure pertains to an electronic stethoscope designed to selectively detect signals within the audible range characteristic of biological origin (hereinafter referred to as "bio-audible range signals").

### BACKGROUND

In 1816, R.T. Laennec, unaccustomed to placing his ear directly on a young woman's chest, rolled up a piece of paper and auscultated her, which is considered the world's first stethoscope. Subsequently, in 1828, P.A. Piorry improved the sound collection section into a horn shape; the diaphragm stethoscope was invented in 1894, followed by the bell stethoscope in the 1940s. Differences in sound transmission characteristics between diaphragm and bell types of stethoscopes is also reported in Hashiguchi et al., "The Transmission Characteristics of Stethoscopes," Research Report of the Faculty of Engineering, Yamanashi University, No. 37, pp. 45-49, Dec. 1986 (hereinafter referred to as non-patent document 1).

Although the characteristics of acoustic stethoscopes have been studied and improved over time, their accuracy often depends on the skill of the physician, thus posing challenges in terms of objectivity. Published in 1933, the classification of heart murmur intensity by S.A. Levine, which is currently adopted by medical practitioners, comprises six categories. The first category refers to sounds that are inaudible to beginners, while the second category denotes sounds that can be heard as murmurs if listened carefully. With the decreasing use of stethoscopes among younger doctors, one of the reasons for the neglect of basic clinical practices such as inquiry, visual inspection, and auscultation is said to be the difficulty in detecting subtle heart sounds.

On the other hand, since approximately 1976, various electronic stethoscopes have been proposed and developed (see JP 53-30187U). Some of these electronic stethoscopes have been marketed. As shown in FIG. 12, generally speaking, the design of electronic stethoscopes aims to selectively detect characteristic auditory signals within the primary examination frequency range of approximately 20Hz-710Hz emitted by the diagnostic subject (biological body). However, the electroacoustic transducers used in traditional electronic stethoscopes sacrifice higher receiving sensitivity due to their mechanical resonance frequency being outside the primary examination frequency range, emphasizing simplicity in frequency characteristics. Lower receiving sensitivity can cause signal distortion and noise generation, thereby interfering with clinicians' assessments of patients, and the sensitivity in amplifying and reproducing specific biological sounds is also limited. Therefore, traditional electronic stethoscopes have not yet been fully accepted by doctors and other medical professionals.

Within the primary inspection frequency range of approximately 20Hz-710Hz, the audible signals that require detection encompass characteristic audible signals indicative of physiological parameters (such as respiratory sounds indicating respiratory rate and heart sounds) as well as ambient noise originating from one or multiple noise sources (ambient noise). For instance, ambient noise can stem from external noise sources, including computers, medical equipment, motors, pumps, fans, alarms, and other electronic circuits; it can also arise from the noise of others, such as family members visiting the patient or medical staff in close proximity to the patient; and it can be noise from vehicles and helicopters. Depending on the specific circumstances, ambient noise may include noise from the patient's environment that does not reflect any measured physiological parameters. Ambient noise from the patient includes the patient's voice and coughing. Consequently, the electronic stethoscope generates all detected audible signals.

The signal-to-noise ratio (SN ratio) of electronic stethoscopes is typically diminished by the presence of ambient noise, irrespective of the noise source. In certain scenarios, this reduction in SN ratio can render it challenging to differentiate physiological sound components from noise elements within the signal, thereby impeding the provision of precise measurement outcomes. The underlying cause of this issue is the low receiving sensitivity of the audible electroacoustic transducers employed in conventional electronic stethoscopes, attributable to the mechanical resonance frequency being outside the primary examination frequency range. In emergency settings, such as helicopters in flight or ambulances, the noise detected by the electronic stethoscope may significantly exceed the target characteristic auditory signals, exacerbating this problem.

### SUMMARY

### Technical issues

In light of the aforementioned issues, the objective of the present disclosure is to introduce an electronic stethoscope that, even in environments where the audible range signals as sound sources are relatively weak compared to noise levels, can achieve a high signal-to-noise ratio (SN ratio) across a broad frequency range encompassing the primary examination frequency band, thereby providing high objectivity and auditory accuracy.

### Technological Solutions

To achieve the aforementioned objectives, the first embodiment of the present disclosure is characterized by: (a) a housing; (b) an audible range sensor, which is housed within the storage cavity of the housing, possesses a mechanical resonance frequency within the audible range, and incorporates a drum-shaped detection unit designed to detect characteristic audible range signals; (c) the stethoscope is equipped with a signal processing circuit designed to process signals outputted from the audible range sensor, and is positioned within the storage cavity. Additionally, the second embodiment of the present disclosure is characterized by: (p) a housing; (q) multiple audible range sensors of varying sizes stored within the storage cavity of the housing; (r) an electronic stethoscope equipped with a signal processing circuit situated within the storage cavity, which performs signal processing on signals outputted from multiple audible range sensors individually. In the electronic stethoscope described in the second aspect, each of the multiple audible range sensors incorporates a drum-shaped detection section, possessing a mechanical resonance frequency within the audible range.

### Beneficial effects

In accordance with the present disclosure, even in environments where the audible range signal, serving as the sound source, is relatively weak compared to the noise level, it is possible to achieve a high signal-to-noise ratio (SN ratio) across a wide frequency range within the audible spectrum, including the primary diagnostic frequency band. This, in turn, provides an electronic stethoscope with enhanced objectivity and auditory accuracy.

### BRIEF DESCRIPTION OF THE DRAWINGS

FIG. 1A is an aerial schematic illustrating the technical concept of the electronic stethoscope in the first embodiment of the present disclosure;

FIG. 1B is an aerial view illustrating the Y-shaped tube structure of the electronic stethoscope in the first embodiment depicted in FIG. 1A;

FIG. 2 shows the arrangement of the audible range sensor (electroacoustic transducer) with built-in amplification components in the bottom view of the sound collection section (thoracic cavity) of the electronic stethoscope in the first embodiment shown in FIG. 1A;

FIG. 3A is a schematic cross-sectional view illustrating the situation where the sound-collecting section of the electronic stethoscope in the first embodiment is firmly pressed onto the diagnostic subject, viewed from the direction of IIIA-IIIA in FIG. 2;

FIG. 3B is a schematic cross-sectional view of the sound collection section of the electronic stethoscope in the first embodiment, viewed from the direction of IIIA-IIIA in FIG. 2, illustrating the condition of weak contact with the diagnostic subject;

FIG. 4 is a schematic plan view of the audible range sensor utilized in the electronic stethoscope of the first embodiment;

FIG. 5 is a cross-sectional view of the audible range sensor utilized in the electronic stethoscope of the first embodiment, as viewed from the V-V direction depicted in FIG. 2;

FIG. 6A is an equivalent circuit diagram centered around the audible range sensor utilized in the electronic stethoscope of the first embodiment, when the output is presented in the form of a signal source follower;

FIG. 6B is an equivalent circuit diagram of a source follower configuration similar to that shown in FIG. 6A, but with one side of the central lower electrode grounded through a sensitivity adjustment resistor;

FIG. 6C is an equivalent circuit diagram centered around the audible range sensor utilized in the electronic stethoscope of the first embodiment, where the current signal is sourced from the drain region side;

FIG. 6D shows the large signal equivalent circuit diagram of the internal capacitance and other components of the element, compared with the physical structure of the insulated gate semiconductor component utilized in the partial audible range sensor of the electronic stethoscope in the first embodiment shown in FIG. 5.

FIG. 7 is a cross-sectional view illustrating the improved structure of the audible range sensor utilized in the electronic stethoscope of the first embodiment;

FIG. 8 is a graph illustrating the frequency dependence of the sensitivity characteristics of the sound sensor utilized in the electronic stethoscope of the first embodiment, when the sensitivity is adjusted to a 100MQ resistor grounded;

FIG. 9A is a graph illustrating the frequency dependence of noise that poses a problem in electronic stethoscopes;

FIG. 9B is the sound pressure spectrum of heart sounds heard through a traditional stethoscope, as described in Non-patent Document 1;

FIG. 10A is a graph illustrating the frequency dependence of the sensitivity characteristics of the audible range sensor utilized in the electronic stethoscope of the first embodiment, under two conditions: with a peripheral lower electrode diameter of 2.4mm and a direct current bias voltage of 11V, and with a diameter of 1.2mm and a direct current bias voltage of 45V;

FIG. 10B is a graph illustrating the frequency dependence of the sensitivity characteristics of the audible range sensor utilized in the electronic stethoscope of the first embodiment, under two different conditions: a peripheral lower electrode diameter of 2.4mm with a direct current bias voltage of 4V, and a diameter of 1.2mm with a direct current bias voltage of 16V;

FIG. 11A shows that in the drum-shaped component where the lower electrode is divided into a peripheral lower electrode and a central lower electrode structure, the graph depicts the frequency dependence of the sensitivity characteristics of the audible range sensor utilized in the electronic stethoscope of the first embodiment, as the sensitivity adjustment resistance varies from 100MΩ→50MΩ→20MΩ→10MΩ→5MΩ→2MΩ;

FIG. 11B shows that for the purpose of comparison, in a drum-shaped component that employs a structure similar to the previous cMUT (capacitive micro-machined ultrasonic transducer) without segmenting the lower electrode, the frequency dependence of sensitivity characteristics in the drum-shaped element is illustrated as the sensitivity adjustment resistor varies from 20MQ →10MΩ→5MΩ→2MΩ→1MΩ→500kΩ;

FIG. 12 is a schematic diagram illustrating the frequency bands of audible range signals corresponding to various characteristics of the auscultation targets in the electronic stethoscope of the first embodiment;

FIG. 13A is a schematic diagram illustrating a specific example of the sensitivity adjustment resistor used to regulate the sensitivity characteristics of an electronic stethoscope in the first embodiment;

FIG. 13B is a schematic diagram illustrating another specific example of the sensitivity adjustment resistor used for adjusting the sensitivity characteristics of the electronic stethoscope in the first embodiment;

FIG. 14 shows the relationship between receiving sensitivity and frequency for each thickness as the thickness of the top protective film increases incrementally;

FIG. 15 is a bottom view illustrating the arrangement of the audible range sensor in the sound collector of the electronic stethoscope according to the second embodiment of the present disclosure;

FIG. 16 is a schematic cross-sectional view illustrating the installation of the sound collection unit of the electronic stethoscope in the second embodiment on the diagnostic subject, viewed from the direction of IV-IVA in FIG. 15;

FIG. 17 is a schematic aerial view illustrating an example of the use of the electronic stethoscope in the second embodiment through wireless transmission;

FIG. 18 is a bottom view illustrating the arrangement of the audible range sensor in the sound collection section of the electronic stethoscope according to the third embodiment of the present disclosure;

FIG. 19A is a schematic cross-sectional view of the sound collection section of the electronic stethoscope from the XIX-XIX direction in FIG. 18, excluding the central suction assist device, according to the third embodiment;

FIG. 19B is a schematic cross-sectional view of the sound collection section of the electronic stethoscope according to the third embodiment, with an adsorption assist unit installed at the center of FIG. 19A;

FIG. 19C is a schematic aerial view of the suction assist device positioned at the center of FIG. 19B;

FIG. 20 is another example bottom view of the audible range sensor arrangement in the sound collection section of the electronic stethoscope according to the third embodiment;

FIG. 21 is another example bottom view of the audible range sensor arrangement in the sound collection section of the electronic stethoscope in the third embodiment;

FIG. 22A is a bottom view illustrating the arrangement of the audible range sensor in the sound collection section of the electronic stethoscope, which is the fourth embodiment of the present disclosure;

FIG. 22B is a cross-sectional view of the sound collection section of the electronic stethoscope from the 4th embodiment, viewed from the direction of IIXIIB-IIXIIB in FIG. 22A;

FIG. 23A is a bottom view illustrating the arrangement of audible range sensors within the sound collection unit of the electronic stethoscope in the first variant of the fourth embodiment;

FIG. 23B is a cross-sectional view of the sound collection section of the electronic stethoscope in the first variant of the fourth embodiment, as viewed from the direction of IIXIIIB-IIXIIIB in FIG. 23A;

FIG. 24A is a bottom view illustrating the arrangement of the audible range sensor within the sound collection unit of the electronic stethoscope in the second variant of the fourth embodiment;

FIG. 24B is a cross-sectional view of the sound collection section of the electronic stethoscope in the second variation of the fourth embodiment, as viewed from the direction of IIXIVB-IIXIVB in FIG. 24A;

FIGS. 25A-B are schematic conceptual diagrams illustrating the preferred arrangement of audible range sensors in the sound collection section of an electronic stethoscope, according to other embodiments of the present disclosure.

## DESCRIPTION OF THE EMBODIMENTS

### Embodiments of the present disclosure

Hereinafter, the first to fourth embodiments of the present disclosure will be exemplarily described with reference to the accompanying drawings. In the following description of the drawings, identical or similar components are denoted by identical or similar symbols. However, it should be noted that the drawings are schematic illustrations, and the relationship between thickness and planar dimensions, as well as the size ratios of various components, may differ from the actual situation. Therefore, specific thicknesses, dimensions, and sizes should be further judged in conjunction with the technical concepts comprehensible in the following description. Of course, there are also parts with different dimensional relationships and proportions between the drawings.

Electronic stethoscopes incorporate audible range sensors (audible range electroacoustic transducers) that utilize built-in amplification components. In these built-in amplification component-based audible range sensors, the "first main electrode region" of the insulated gate semiconductor component, which includes the amplification component, refers to the semiconductor region serving as the source or drain region in field-effect transistors (FET), static induction transistors (SIT), or equivalent novel transistor structures. In insulated gate bipolar transistors (IGBT) or semiconductor components with novel structures equivalent to IGBT, it denotes the semiconductor region functioning as the emitter or collector region. For semiconductor components with structures equivalent to insulated gate thyristors or novel structures of insulated gate thyristors, such as MIS-controlled static induction thyristors (SI thyristors), it signifies the semiconductor region acting as the anode or cathode region. The "second main electrode region" in FET, SIT, etc., denotes the semiconductor region serving as the source or drain region, distinct from the aforementioned first main electrode region; in IGBT and similar structures, it refers to the semiconductor region acting as the emitter or collector region, separate from the first main electrode region. In cases such as MIS-controlled SI thyristors, it designates the semiconductor region serving as the anode or cathode region, which is not the aforementioned first main electrode region.

Therefore, if the "first main electrode region" is the source region, the "second main electrode region" is the drain region, and the "main current" will flow between the first and second main electrode regions. If the "first main electrode region" is the emitter region, the "second main electrode region" is the collector region. If the "first main electrode region" is the anode region, then the "second main electrode region" is the cathode region. By exchanging the bias relationship, in the case of MISFET, the functions of the "first main electrode region" and the "second main electrode region" can be interchanged. Furthermore, when referring to the "main electrode region" in this context, it is an inclusive expression, meaning it refers to either the first main electrode region or the second main electrode region, depending on what is technically appropriate.

In the following description, the terms "upper" and "lower" or "upper" and "lower" are used to define the "upper electrode" and "lower electrode", as well as the directions "right" and "left". These terms, along with the use of "upper" and "lower" to define directions, are solely for the sake of convenience and do not restrict the technical concept of the present disclosure. For instance, if an object is observed by rotating it 90°, the upper and lower will naturally switch to the left and right. If the object is observed by rotating it 180°, the upper and lower will be inverted. In the subsequent explanation, the case where the first conductivity type is p-type and the second conductivity type is n-type will be exemplified. However, the conductivity types can also be selected in the opposite relationship, that is, the first conductivity type is n-type and the second conductivity type is p-type. The "+" and "-" following "n" and "p" respectively indicate that the impurity density of the semiconductor region is relatively higher or lower than that of the semiconductor region without "+" and "-". Nevertheless, even for semiconductor regions marked with the same "+" and "-" or those not marked with "+" and "-", it does not necessarily mean that the impurity density of each semiconductor region is strictly identical in a strict sense.

The first to fourth embodiments presented below illustrate the method embodying the technical concept of the present disclosure, as well as the apparatus utilized in this method. The technical concept of the present disclosure does not specify the materials, shapes, structures, arrangements, or other constituent elements, nor does it dictate the steps of the method. Instead, it is as follows. The technical concept of the present disclosure is not limited to the contents described in the first to fourth embodiments, and various modifications can be made within the technical scope defined by the specific matters of disclosure stated in the claims.

### First Embodiment

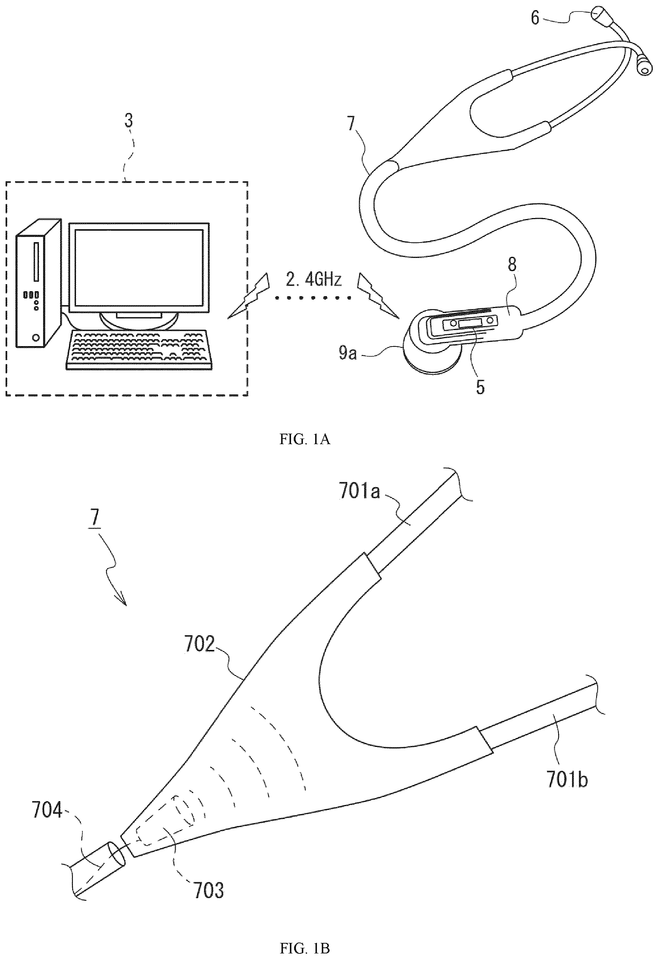

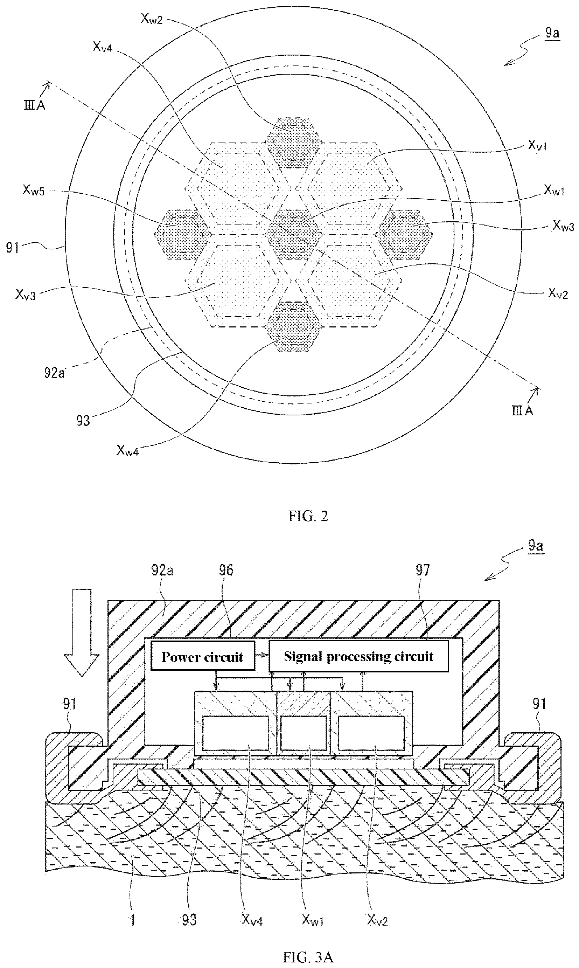

As illustrated in FIG. 1A, the electronic stethoscope in the first embodiment of the present disclosure comprises a sound collection component (chest piece) 9a, an acoustic tube 7, and an external ear attachment component (earpiece) 6. Although two external ear attachments 6 are depicted in FIG. 1A, it is also feasible to use only one external ear attachment 6. As shown in FIGS. 2 and 3A, among others, the sound collection section 9a may incorporate audible range sensors (audible range electroacoustic transducers) $X_{v1}$, $X_{v2}$, $X_{v3}$, $X_{v4}$, $X_{w1}$, $X_{w2}$, $X_{w3}$, $X_{wj}$, $X_{w4}$, and $X_{w5}$, which are separate components. The audible range sensors $X_{v1}$-$X_{v4}$ and $X_{w1}$-$X_{w5}$ can be configured as semiconductor elements with amplification capabilities, designed to amplify audible range characteristic signals indicative of diagnostic subject features directly input to the gate electrode.

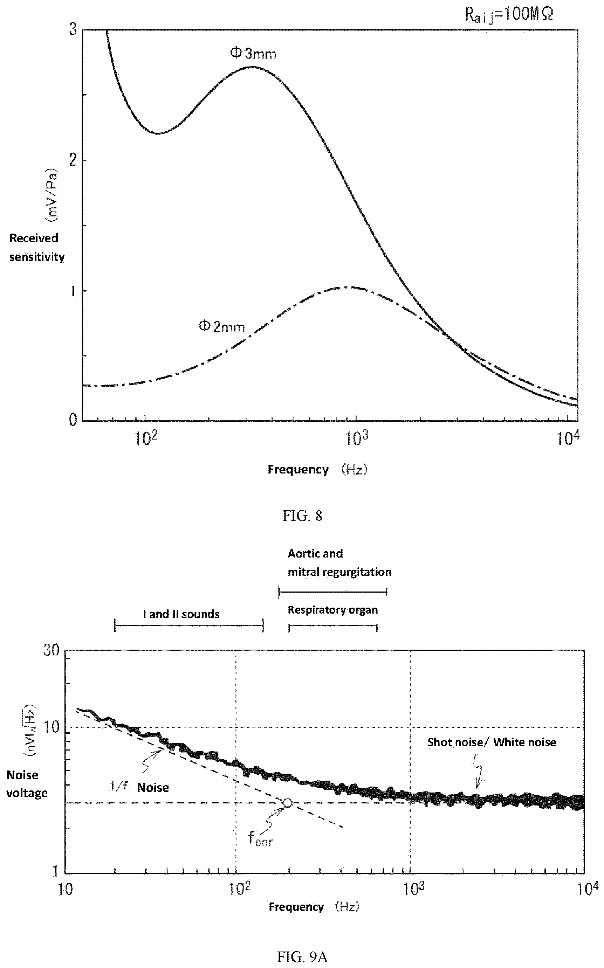

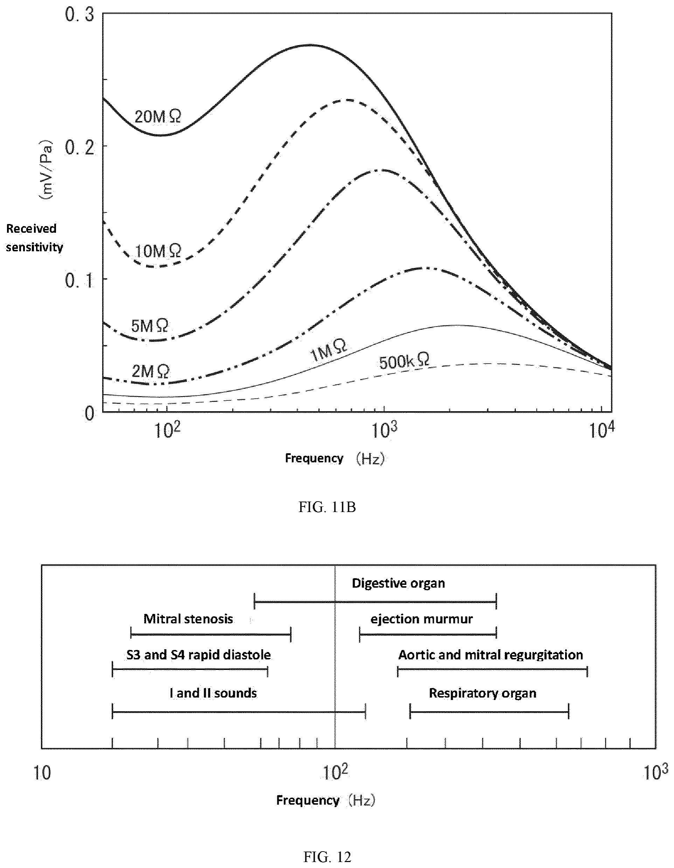

As illustrated in FIGS. 8, 10A, 10B, and 11A, each of the audible range sensors $X_{v1}$-$X_{v4}$ and $X_{w1}$-$X_{w5}$ possesses a drum-shaped detection area. Due to the mechanical resonance frequency within the audible range, the sensitivity to receive characteristic audible frequency range signals is high. In FIG. 2, the large hexagons represent the audible range sensors $X_{v1}$-$X_{v4}$, while the small hexagons represent $X_{w1}$-$X_{w5}$. The inner hexagons within their respective areas depict the planar patterns defining the vibration cavities of the drum-shaped detection components. As shown in FIG. 12, by setting the mechanical resonance frequency of the drum-shaped detection components within the audible range, a higher receiving sensitivity can be achieved. For instance, within the primary inspection frequency band of approximately 20Hz to approximately 710Hz, which falls within the audible frequency range, this frequency band encompasses the audible range. Notably, when the audible frequency range sensors $X_{v1}$-$X_{v4}$ and $X_{w1}$-$X_{w5}$ are integrated with insulated gate semiconductor components (such as MOS transistors that amplify characteristic audible frequency range signals) into a unified structure, the detected voltage signals can be directly amplified by the same functionality as the insulated gate semiconductor components, thereby achieving extremely high receiving sensitivity.

The sound collector 9a can be positioned in contact with or in close proximity to the biological sample under examination, such as the patient's chest, abdomen, arm, or leg (quadriceps) or any other body part of the patient. The audible range sensors $X_{v1}$-$X_{v4}$ and $X_{w1}$-$X_{w5}$ function similarly to insulated gate bipolar transistors, amplifying audible range signals related to one or more biological processes or analogous processes occurring within the patient's body. For instance, as illustrated in FIG. 12, the audible range sensors $X_{v1}$-$X_{v4}$ and $X_{w1}$-$X_{w5}$ can amplify various audible range signals generated by the patient's heartbeat, breathing, blood flow, digestion, and other physiological processes.

The characteristic audible frequency range signals detected/amplified by audible range sensors of varying sizes, $X_{v1}$-$X_{v4}$ and $X_{w1}$-$X_{w5}$, undergo frequency characteristic smoothing processing through signal processing (digital processing) within the signal processing circuit 97 depicted in FIG. 3A and elsewhere. Following D/A conversion, the smoothed audible frequency characteristic signals can be introduced into the external ear attachment component 6 via the speaker structure within the acoustic tube 7 shown in FIG. 1B. The acoustic tube 7 may comprise a hollow tube, which can be filled with air. The acoustic tube 7 can be flexible. FIG. 1B illustrates the Y-shaped tube 702 of an electronic stethoscope, which includes a speaker structure. In FIG. 1B, a single speaker 703 is positioned appropriately within the Y-shaped tube 702, allowing the sound produced by the speaker 703 to propagate through two ear canals, 701a and 701b.

The placement of the loudspeaker structure within the electronic stethoscope should be chosen to minimize or reduce the propagation of frictional sounds within the sound tube 7. Consequently, the loudspeaker 703 can be installed within the external ear attachment 6 of the headset. The conductor 704 connected to the loudspeaker 703 extends from the coupling point of the sound tube 7 through the lumen of the main tube of the sound tube 7 to the signal processing circuit 97 of the sound collector 9a.

Although omitted in FIG. 3A, the sound collection section 9a of the electronic stethoscope in the first embodiment can be equipped with a built-in communication circuit, transmitting the information signals processed by the signal processing circuit 97 to various information systems 3. As shown in FIG. 1A, by providing the communication circuit, the information signals processed by the signal processing circuit 97 can be transmitted in real-time to the information system 3 for convenient visualization, allowing heart rate and heart rate waveform to be displayed on the display screen of the information system 3. Furthermore, such information can be stored in various institutional databases or cloud-based medical systems, such as those associated with clinics, hospitals, or clinic or hospital networks. In other words, the information system 3 can record, display, slow-play back, and store characteristic sound signals, such as biological sound signals, as information received in real-time by the information system 3, and further transmit the information to other information systems.

The communication between the sound collector 9a and the information system 3 can be conducted wirelessly using electromagnetic waves in the 2.4GHz frequency band, for instance. According to Article 38-2-2, Paragraph 1, Item 1 of the Radio Law, "advanced low-power data communication systems in the 2.4GHz frequency band" are exempt from the requirement for a radio station license. Therefore, electromagnetic waves in the 2.4GHz frequency band with an antenna power below 10mW are a convenient frequency band for use within clinics and hospitals. Additionally, the fifth-generation mobile communication systems (5G communication) stipulated by the International Telecommunication Union (ITU) can also be utilized. 5G communication serves as an example, and 6G or 7G communication is also conceivable.

Institutional information systems interested in various biological information such as heartbeat, breathing, blood flow, and digestion of patients can access information processed by the signal processing circuit 97 through the communication circuit embedded in the sound collection component 9a. For instance, the information system 3 can be equipped with artificial intelligence, which enables machine learning on the information acquired by the electronic stethoscope in the first embodiment, thereby achieving highly objective auscultation. A display and control unit 5 is located on the side of the sound collection unit 9a's casing, and is connected to the sound tube 7 via the display and control unit 5. The switch for the power circuit, adjustment of the sensitivity adjustment resistor $R_{aij}$, adjustment of the amplifier gain, control of the communication circuit, adjustment of the volume, switching of modes, and display of signal processing information are all managed by the display/operation unit 5.

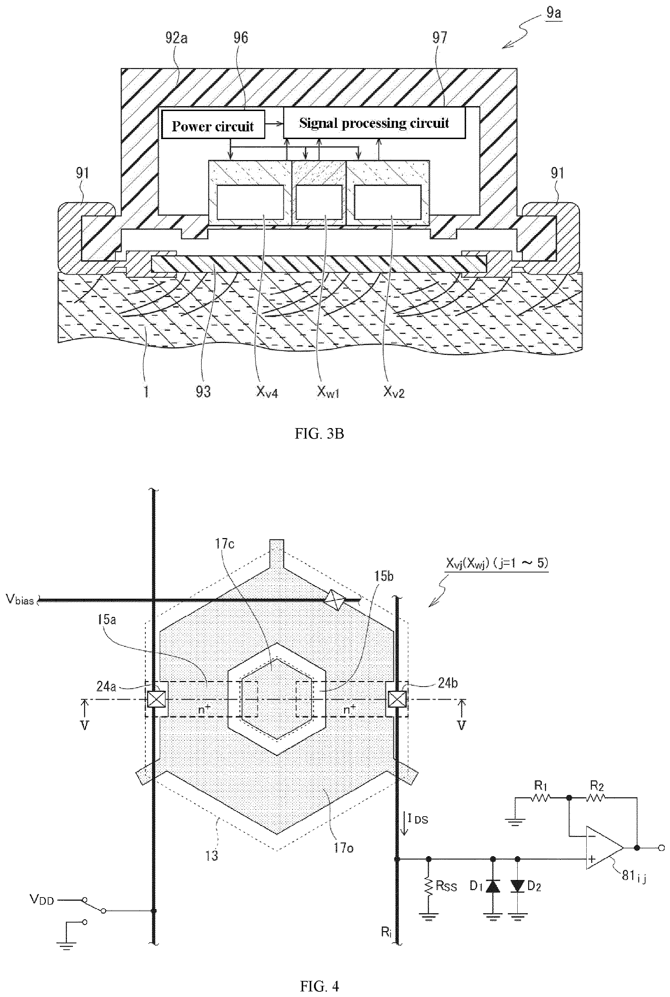

The reception sensitivity of the audible range sensors $X_{v1}$-$X_{v4}$ and $X_{w1}$-$X_{w5}$, which are integrated into the sound collector 9a of the electronic stethoscope according to the first embodiment, is contingent upon the dimensions such as the inner diameter and diagonal diameter occupied by the audible range sensors, as illustrated by the difference between the solid line and the dashed curve in FIG. 8. As depicted in FIG. 2, the sound collector 9a of the electronic stethoscope in the first embodiment features a first audible range sensor $X_{w1}$ positioned centrally within the element array section. This sensor comprises a hexagonal drum-shaped detection section with a first diagonal diameter, which is arranged with nine hexagons. FIG. 4 presents a detailed structural example of the planar pattern of the first audible range sensor $X_{w1}$. The second audible range sensor $X_{v1}$, the third audible range sensor $X_{v2}$, the fourth audible range sensor $X_{v3}$, and the fifth audible range sensor $X_{v4}$ each possess a second diagonal diameter longer than the first diagonal diameter, and they are arranged around the first audible range sensor $X_{w1}$. As shown in FIG. 2, the planar patterns of the second audible range sensor $X_{v1}$, the third audible range sensor $X_{v2}$, the fourth audible range sensor $X_{v3}$, and the fifth audible range sensor $X_{v4}$ also include an inner hexagonal drum-shaped detection area.

Furthermore, a sixth audible range sensor $X_{w2}$ is positioned between the fifth audible range sensor $X_{v4}$ and the second audible range sensor $X_{v1}$, with its first diagonal diameter being shorter than its second diagonal diameter. Similarly, the first diagonal diameter of the seventh audible range sensor $X_{w3}$ is situated between the second audible range sensor $X_{v1}$ and the third audible range sensor $X_{v2}$, while the first diagonal diameter of the eighth audible range sensor $X_{w4}$ is located between the third audible range sensor $X_{v2}$ and the fourth audible range sensor $X_{v3}$. Additionally, a ninth audible range sensor $X_{w5}$, featuring a first diagonal diameter, is placed between the fourth audible range sensor $X_{v3}$ and the fifth audible range sensor $X_{v4}$. As illustrated in FIG. 4, the planar pattern of the sixth audible range sensor $X_{w2}$, the seventh audible range sensor $X_{w3}$, the eighth audible range sensor $X_{w4}$, and the ninth audible range sensor $X_{w5}$ also encompasses an inner hexagonal drum-shaped detection area.

As illustrated in FIG. 2, the inner hexagon of the dual hexagonal pattern, the housing 92a of the electronic stethoscope in the first embodiment, features an array of drum-shaped detection sections with varying diagonal diameters. The diagonal diameter of the first audible range sensor $X_{w1}$ is smaller than that of the second to fifth audible range sensors $X_{v1}$-$X_{v4}$. The drum-shaped detection section of the first audible range sensor $X_{w1}$ is positioned in the center of the housing 92a, while the second to fifth audible range sensors $X_{v1}$-$X_{v4}$ are arranged around the first audible range sensor $X_{w1}$, each featuring a drum-shaped detection section. As can be observed from FIG. 8 and elsewhere, the audible range sensor arrays $X_{v1}$-$X_{v4}$, denoted by inner dashed lines in FIG. 2 and featuring drum-shaped detection sections with larger diagonal diameters, differ from the audible range sensor arrays $X_{w1}$-$X_{w5}$, which have drum-shaped detection sections with smaller diagonal diameters, in terms of their mechanical resonance frequencies. Consequently, by deliberately shifting the mechanical resonance frequencies of the individual drum-shaped detection sections within the array, it is possible to expand the frequency band of the electronic stethoscope in the first embodiment, thereby enhancing the overall receiving sensitivity.

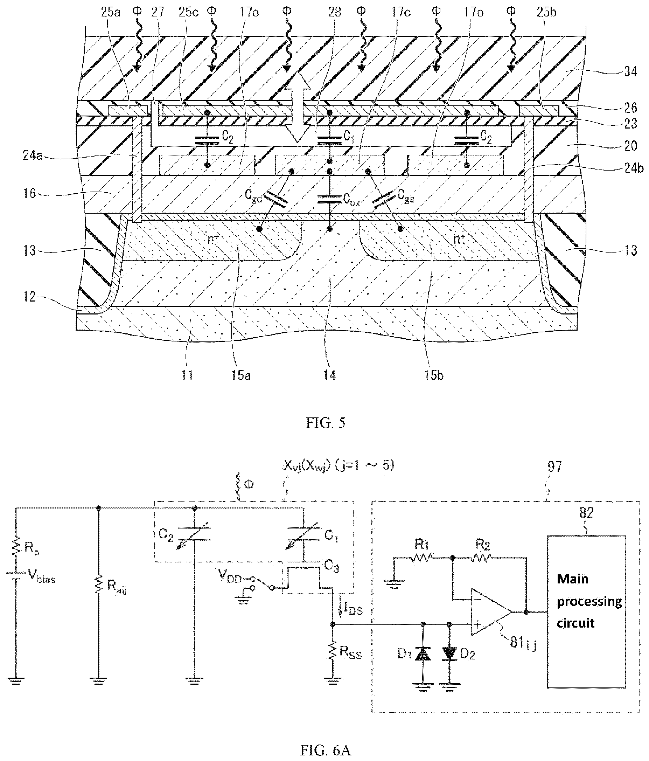

FIGS. 3A and 3B are cross-sectional views taken along the direction of IIIA-IIIA in FIG. 2, illustrating the structure of the electronic stethoscope in the first embodiment, where the fifth audible range sensor $X_{v4}$ is located on the left side of the central first audible range sensor $X_{w1}$, and the third audible range sensor $X_{v2}$ is located on the right side. FIG. 5 and other sections display detailed cross-sectional structural examples of the first audible range sensor $X_{w1}$, the fifth audible range sensor $X_{v4}$, and the third audible range sensor $X_{v2}$. The vibration cavity 28 includes a drum-shaped detection section. In FIGS. 3A and 3B, the hollowed-out inner square area of the first audible range sensor $X_{w1}$ schematically indicates the vibration cavity corresponding to the inner hexagon located within the small hexagonal area representing the first audible range sensor $X_{w1}$ in FIG. 2. In FIG. 2, the hollowed-out areas within the fifth audible range sensor $X_{v4}$ and the third audible range sensor $X_{v2}$ are represented by squares, schematically indicating the vibration cavities corresponding to the inner hexagons located within the larger hexagonal areas representing the fifth audible range sensor $X_{v4}$ and the third audible range sensor $X_{v2}$, respectively. However, this is merely a schematic illustration, and the actual aspect ratio of the vibration cavity differs from the highly exaggerated aspect ratio of the white rectangle in FIGS. 3A and 3B. For instance, the white rectangle is a flat rectangle with a width of approximately 1.2-2.4mm and a height of approximately $2\mu$m, with its aspect ratio almost obscuring its height.

As illustrated in FIG. 3A, the sound collection unit 9a of the electronic stethoscope in the first embodiment features a housing 92a. The housing 92a is surrounded by an outer skin, at least a portion of which is a flat plate, forming a storage cavity within the outer skin for housing audible range sensors $X_{v1}$-$X_{v4}$ and $X_{w1}$-$X_{w5}$, among others. The shape of the housing 92a depicted in FIG. 3A is nearly cylindrical, except for the lower brim. Consequently, the primary components consist of an arc-shaped surface serving as the lateral surface of the cylinder, a planar surface acting as the upper surface (top) of the cylinder, and another planar surface constituting the lower surface (bottom) of the cylinder, which collectively form the outer skin of the cylinder. In the housing 92a shown in FIG. 3A, the flat plate-like portion corresponds to the upper and lower surfaces of the cylinder.

The lower surface of the housing 92a features a step similar to that of a traditional acoustic stethoscope, yet the central portion of the lower surface remains flat. In the electronic stethoscope of the first embodiment, the "signal input surface" is defined as the flat region of the lower surface, where the characteristic audible range signals are inputted. A groove is selected on the lower surface opposite the signal input surface within the housing 92a as the sensor mounting component. The first audible range sensor $X_{w1}$, the third audible range sensor $X_{v2}$, and the fifth audible range sensor $X_{v4}$ are fixed to this sensor mounting component, with the lower portions of their respective side surfaces stored within the inner wall of the sensor mounting component. Conversely, one side (lower portion) of the plate-like outer shell of the housing 92a serves as the signal input surface, facing the sensor mounting portion. Additionally, within the storage cavity formed by the housing 92a, a power circuit 96 and a signal processing circuit 97 are housed. The power circuit 96 provides the necessary electrical energy for the nine audible range sensors $X_{v1}$-$X_{v4}$ and $X_{w1}$-$X_{w5}$, as well as the signal processing circuit 97.

The signal processing circuit 97 receives signals from audible range sensors $X_{v1}$-$X_{v4}$ and $X_{w1}$-$X_{w5}$ and performs signal processing. As illustrated in FIGS. 6A to 6C, the signal processing circuit 97 comprises amplifiers $81_{ij}$ (i=v, w; j=1-4 or 5) dedicated to each audible range sensor $X_{v1}$-$X_{v4}$ and $X_{w1}$-$X_{w5}$, as well as a main processing circuit 82 shared among audible range sensors $X_{v1}$-$X_{v4}$ and $X_{w1}$-$X_{w5}$. This circuit inputs outputs from respective amplifiers $81_{ij}$ and processes the signals. These sensors are equipped with the main processing circuit 82. The main processing circuit 82 receives respective outputs from audible range sensors $X_{v1}$-$X_{v4}$ and $X_{w1}$-$X_{w5}$ through amplifiers $81_{ij}$ acting as preamplifiers. After A/D conversion, it utilizes digital technology to perform frequency characteristic smoothing processing on the output signals of audible range sensors $X_{v1}$-$X_{v4}$ and $X_{w1}$-$X_{w5}$ of different sizes. The main processing circuit 82 further processes the signals, such as removing noise signals, and then outputs them as pure characteristic audible range signals.

FIG. 2 illustrates nine audible range sensors, $X_{v1}$-$X_{v4}$ and $X_{w1}$-$X_{w5}$ installed within the sensor's fixed component. These sensors are designed to detect and amplify characteristic audible range signals indicative of diagnostic object features, ensuring adequate signal strength for detection despite the relatively small diameter of the housing 92a. Currently, the maximum diameter of many sound collection components (thoracic components) on the market typically ranges from 45mm to 50mm, with pediatric models having a maximum diameter of 37mm to 40mm.

However, by reducing the number of the nine audible range sensors, $X_{v1}$-$X_{v4}$ and $X_{w1}$-$X_{w5}$, as shown in FIG. 2, it is possible to decrease the outer diameter of the sound collection component 9a to unprecedented dimensions, thereby achieving excellent audibility.

There are no specific restrictions on the material of the shell 92a, which can be soft plastics such as polyvinyl chloride (PVC). Alternatively, it can be hard plastics or metals such as aluminum (Al) or titanium (Ti). To enhance adhesion to the surface of the diagnostic object 1, it is preferable that the material of the shell 92a is an elastic material such as silicone rubber. The shape of the shell 92a shown in FIG. 3A is merely an example and is not limited to a cylindrical shape. The shell 92a can be a hemispherical sphere or any other three-dimensional shape, as long as it is enclosed by an outer skin that comprises at least a portion of a flat plate-like section, forming a storage cavity within the outer skin. In the case of a hemispherical sphere, the hemispherical sphere is continuous with the plane serving as the lower surface (base) of the hemispherical sphere, forming a storage cavity. In the case of a hemispherical sphere shell, the only flat plate-like section is the lower surface. Therefore, the "signal input surface" can be defined as a portion of the hemispherical sphere's lower surface, where signals within the audible range are inputted. The hemispherical sphere connected to the plane serving as the lower surface does not necessarily have a uniformly curved surface and can include uneven portions.

In the sound collection section 9a of the electronic stethoscope presented in the first embodiment depicted in FIG. 3A, a flexible and elastic buffer film 93 is situated on the underside of the casing 92a. To endow the buffer film 93 with flexibility and elasticity, thereby enabling it to function as a diaphragm on the bottom surface of the casing, a buffer film holder 91 is installed on the lower portion of the outer surface of the casing 92a. This section serves to affix the periphery (outer edge) of the buffer film 93, thereby preserving its flexibility and airtightness. Subsequently, the buffer film (casing diaphragm) 93 is arranged at the lower part of the casing 92a, positioned to obstruct the signal input surface. The buffer film 93 constitutes an air layer, which is sealed within a designated volume between the signal input surfaces of the casing 92a, akin to a conventional acoustic stethoscope.

By constituting a sealed air layer, the buffer film 93 functions as a vibrating film within the air layer, akin to an acoustic stethoscope. That is, when utilizing the electronic stethoscope of the first embodiment, as depicted in FIG. 3A, upon contact of the buffer film 93 with the surface of the diagnostic object 1, characteristic audible signals are transmitted to the signal input surface via the air layer as the signal transmission medium. The characteristic audible range signals transmitted to the signal input surface vibrate the respective upper electrodes of the audible range sensors $X_{v1}$-$X_{v4}$ and $X_{w1}$-$X_{w5}$, which serve as part of the drum-like detection assembly, through the signal input surface. As shown in FIG. 5 and other figures, an example of the detailed cross-sectional structure of each audible range sensor $X_{v1}$-$X_{v4}$ and $X_{w1}$-$X_{w5}$ is provided, where the upper electrode 25c is presented as one layer within a multilayer film that includes the drumhead (vibrating film) of the drum.

Furthermore, a protrusion is situated at the lower part of the housing 92a, encircling the signal input surface area within the lower section of the housing 92a. When the sound collection component 9a is firmly pressed onto the surface of the diagnostic object 1 in the direction indicated by the arrow in FIG. 3A, until an indentation is left on the surface, the periphery of the buffer film (housing vibration film) 93 will bend, and the center of the buffer film 93 will approach the signal input surface. The contact between the buffer film 93 and the protrusion prevents the sensitivity of the electronic stethoscope in the first embodiment from decreasing as in an acoustic stethoscope, as the volume of the air layer maintained between the buffer film (housing vibration film) 93 and the signal input surface is smaller than when pressed lightly. That is to say, in FIG. 3A, when auscultating the signal within the audible range of features, the sound collection section 9a is in contact with the surface of the diagnostic object 1. Compared to the light pressure situation in FIG. 3B, a smaller volume of air layer is formed between the signal input surface of the housing 92a and the buffer film (housing vibration film) 93. The sound pressure in the air layer is determined by the ratio of the volume of the air layer to the amplitude of the surface of the diagnostic object 1 contacted by the buffer film 93. During respiration, the amplitude of the signal within the audible range of features on the surface of the diagnostic object 1 is approximately 0.3 $\mu$m. If the height of the air layer per unit area on the signal input surface of the housing 92a is 3 mm, the sound pressure of the signal within the audible range of features of the diagnostic object is 10 Pa. By limiting the volume of the air layer, the signal within the audible range of features can be acquired with high sensitivity without distortion.

FIG. 3B illustrates the situation where the sound collection component 9a is pressed weakly (gently) against the surface of the diagnostic subject 1, with the buffer film 93 not making contact with the protrusions, thus forming an air layer between the signal input surface located at the lower part of the housing 92a and the buffer film (housing vibration film) 93. Even in the scenario depicted in FIG. 3B, the buffer film 93 imposes a greater restriction on the volume of the air layer compared to the situation without the buffer film 93. According to the electronic stethoscope of the first embodiment, as shown in FIGS. 3A and 3B, the configuration of the sound collection section 9a enables the acquisition of biological sounds with high sensitivity and without distortion. The volume of the air layer affects the frequency characteristics of the electronic stethoscope in the first embodiment.

The material of the buffer film 93 is not particularly restricted, but it is preferably made of an elastic material such as silicone rubber. The buffer film holder 91 and the buffer film 93 can be made of silicone rubber and can be separated from the housing 92a. As shown in FIGS. 3A and 3B, the protruding portion can be integrally formed with the bottom surface of the housing 92a or with the buffer film 93. The protruding portion is not limited to the examples shown in FIGS. 3A and 3B. For instance, the position, number, and shape of the protrusions can be adjusted accordingly to suppress changes in the volume of the air layer caused by flexing of the surface area of the diagnostic object 1 or the buffer film (housing vibration film) 93. In FIG. 2, the planar pattern of the audible range sensors $X_{v1}$-$X_{v4}$ and $X_{w1}$-$X_{w5}$ represents a hexagonal drum-shaped detection area, but the planar pattern of the audible range sensors $X_{v1}$-$X_{v4}$ and $X_{w1}$-$X_{w5}$ is not limited to a hexagonal shape; various drum-shaped detection areas such as rectangular or octagonal shapes can also be adopted.

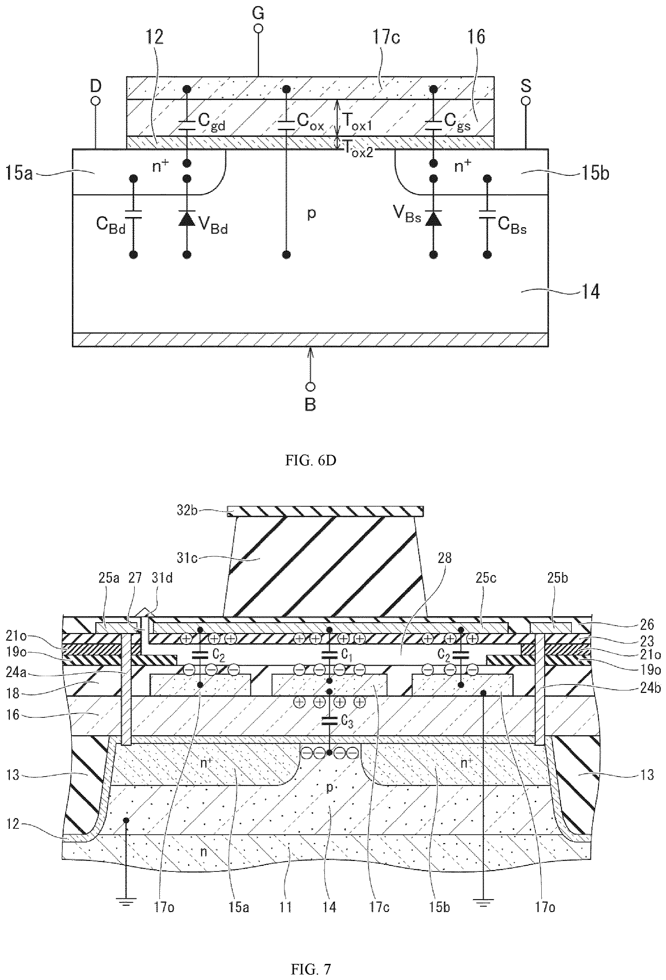

As depicted in FIGS. 5 and 7, the audible range sensors $X_{v1}$-$X_{v4}$ and $X_{w1}$-$X_{w5}$ of the electronic stethoscope, serving as the first embodiment, are each isolated from the upper portion of the common substrate 11 by an element isolation insulating film 13, forming mutually independent electrical channel formation regions 14. These channel formation regions 14 can adopt a two-dimensional arrangement on a single chip. However, it is also feasible to mount each audible range sensor $X_{v1}$-$X_{v4}$ and $X_{w1}$-$X_{w5}$ in a hybrid manner on a printed circuit board or similar circuit board. In any scenario, as shown in FIGS. 5 and 7, above each channel formation region 14, the audible range sensors $X_{v1}$-$X_{v4}$ and $X_{w1}$-$X_{w5}$ are equipped with a vibration cavity 28, which serves as the overall structure of the amplification element. If the channel formation region 14 is a p-type semiconductor region, it functions as an element with a structure and functionality corresponding to the p-type well of a conventional semiconductor integrated circuit. As illustrated in FIG. 5 and subsequent cross-sectional views, the surface of the channel formation region 14 of the first conductivity type is arranged with a first main electrode region 15b of the second conductivity type and a second main electrode region 15a. The semiconductor element, configured as an insulated gate type, serves as a part of the drum-shaped detection component.

In the past, the conventional technical concept of capacitive MEMS (Micro-ElectroMechanical Systems) elements known as cMUT (Capacitive Micro-Mechanical Ultrasonic Transducer) was to detect changes in induced charges within the electrostatic capacitance between the upper and lower electrodes. However, the traditional drum-shaped (capacitive) acoustic wave elements, which detect changes in stored charges within the electrostatic capacitance between the upper and lower electrodes, have limitations in enhancing reception sensitivity. Therefore, the inventor of this disclosure abandoned the traditional drum-shaped acoustic wave element method of detecting changes in induced charges within the electrostatic capacitance and integrated (integrated) an insulated gate semiconductor element as the internal structure of an audible range sensor with built-in amplification components.

In other words, by integrating insulated gate semiconductor components into audible range sensors, according to Gauss's law, the capacitance changes induced by the input of audible range characteristic signals representing the characteristics of the diagnostic object are concentrated on the charges induced on the surface of the channel formation region 14 and the potential variations associated with these charges. That is to say, the charges induced on the surface of the channel formation region 14 control the height of the surface potential of the channel formation region 14. The capacitance changes caused by the input of characteristic audible range signals control the height of the channel barrier through which current flows, thereby utilizing the novel semiconductor component that detects characteristic audible range signal inputs as current variations as the audible range sensor of the electronic stethoscope in the first embodiment.

In the plan view depicted in FIG. 4, the two rectangles indicated by dashed lines represent the first main electrode region 15b and the second main electrode region 15a of the insulated gate type semiconductor element, respectively. These two electrode regions serve as components of the audible range sensors $X_{v1}$-$X_{v4}$ and $X_{w1}$-$X_{w5}$. Two opposing rectangles are arranged within the hexagonal audible range sensors $X_{v1}$-$X_{v4}$ and $X_{w1}$-$X_{w5}$, such that these two opposing rectangles can enter the inner hexagon from the outer ring-shaped hexagon (hexagonal ring). However, FIG. 4 is a schematic diagram, and the distance between the opposing portions of the first main electrode region 15b and the second main electrode region 15a corresponds to the gate length of the insulated gate semiconductor element, which is an exaggerated representation compared to the actual gate length. That is, the actual diagonal length of the central lower electrode 17c is designed to be approximately 300-800 $\mu$m, while the distance (gate length) between the opposing portions of the first main electrode region 15b and the second main electrode region 15a is approximately 1 $\mu$m. In the audible range sensors used in the electronic stethoscope of the first embodiment, the capacitance changes of the audible range sensors $X_{v1}$-$X_{v4}$ and $X_{w1}$-$X_{w5}$ are detected as current changes by the current flowing between the first main electrode region 15b and the second main electrode region 15a.

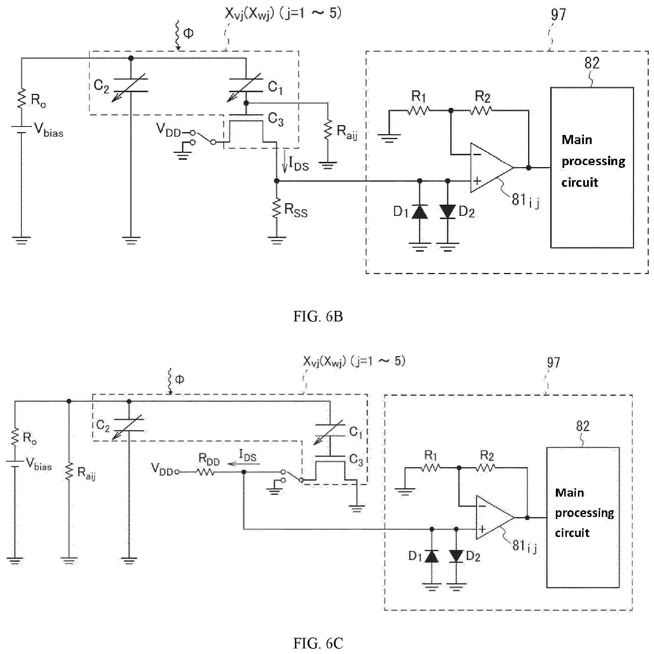

The output signal line $R_i$ is connected to each sounding sensor, facilitating the extraction of current signals from either the first main electrode region 15b or the second main electrode region 15a of each insulated gate semiconductor element arranged in a two-dimensional manner within the groove of the sensor's fixed component. FIG. 4 illustrates an example where the output signal line $R_i$ is connected to the first contact plug on the right side of each sounding sensor with a hexagonal drum-shaped detection area. Although FIG. 4 and FIG. 6A demonstrate the extraction of current signals from the first main electrode region (source region) 15b of the insulated gate semiconductor element, which functions as a source follower using a source resistor $R_{SS}$ as part of the audible range sensor structure, the circuit topology is not limited to the scenarios depicted in FIG. 4 and FIG. 6A. For instance, as shown in FIG. 6C, a structure where the source region is grounded (at the second potential) and the current signal is extracted from the second main electrode region (drain region) 15a side through a drain resistor $R_{DD}$ is also acceptable.

In FIG. 6A, the output signal line $R_i$ is inputted into the individually configured amplifiers $81_{ij}$, which correspond to the audible range sensors $X_{v1}$-$X_{v4}$ and $X_{w1}$-$X_{w5}$, respectively. The outputs from the amplifiers $81_{ij}$ are then fed into a common main processing circuit 82, extracting one audible range sensor. As shown in FIG. 8, the audible range sensors $X_{v1}$-$X_{v4}$ and $X_{w1}$-$X_{w5}$ differ in size (diagonal diameter), resulting in varying characteristics of reception sensitivity with respect to frequency. Each of the amplifiers $81_{ij}$ provided for each audible range sensor $X_{v1}$-$X_{v4}$ and $X_{w1}$-$X_{w5}$ adjusts its amplification factor to ensure that the output levels of the audible range sensors $X_{w1}$-$X_{w5}$ with the first diagonal diameter, the audible range sensors $X_{v1}$-$X_{v4}$ with the second diagonal diameter, and the audible range sensors $X_{w1}$-$X_{w5}$ with the first diagonal diameter are equal.

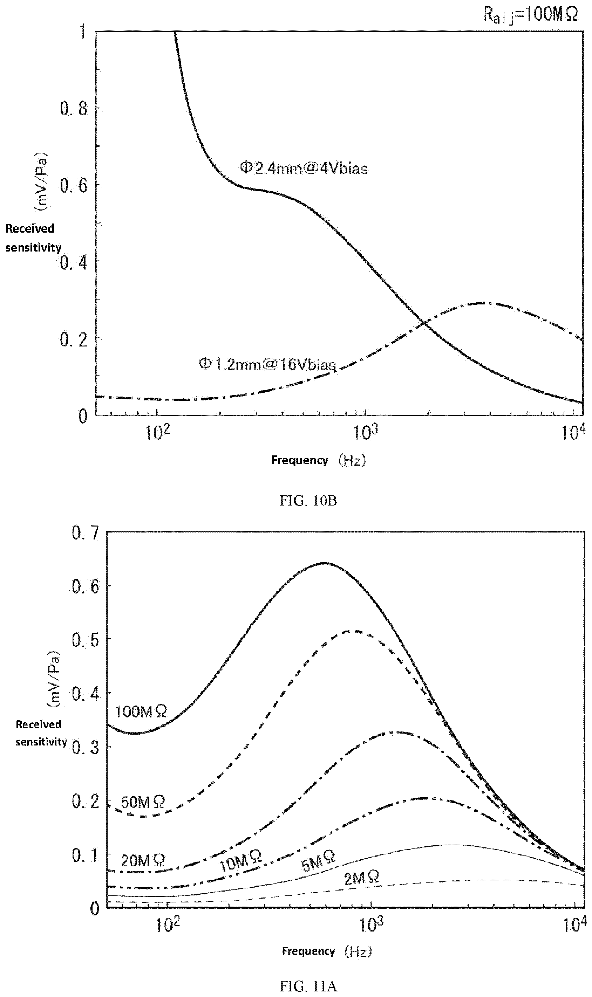

Furthermore, as illustrated in FIG. 11A, the frequency-dependent characteristics of the receiving sensitivity of each audible range sensor $X_{v1}$-$X_{v4}$ and $X_{w1}$-$X_{w5}$ can be altered by modifying the value of the sensitivity adjustment resistor $Ra_{ij}$. Consequently, by adjusting the value of the sensitivity adjustment resistor $Ra_{ij}$ for each audible range sensor $X_{v1}$-$X_{v4}$ with the same diagonal diameter, the frequency-dependent characteristics of the receiving sensitivity of each sensor can be modified. Similarly, by modifying the value of the sensitivity adjustment resistor $Ra_{ij}$ for each audible range sensor $X_{w1}$-$X_{w5}$ with the same diagonal diameter, the frequency-dependent characteristics of the receiving sensitivity of each sensor $X_{w1}$-$X_{w5}$ can be altered.

In other words, the frequency-dependent characteristics of the receiving sensitivity for the nine audible range sensors $X_{v1}$, $X_{v2}$, $X_{v3}$, $X_{v4}$, $X_{w1}$, $X_{w2}$, $X_{w3}$, $X_{wj}$, $X_{w4}$, and $X_{w5}$ depicted in FIG. 2 can be configured to vary from one another, thereby achieving an overall broadband frequency-dependent characteristic of receiving sensitivity. When the frequency-dependent characteristics of the receiving sensitivity for these nine audible range sensors are configured to differ from one another, the amplification factors of each amplifier $81_{ij}$ provided for each audible range sensor $X_{v1}$-$X_{v4}$ and $X_{w1}$-$X_{w5}$ can be individually controlled to adjust the signal levels of the nine inputs to the common main processing circuit 82.

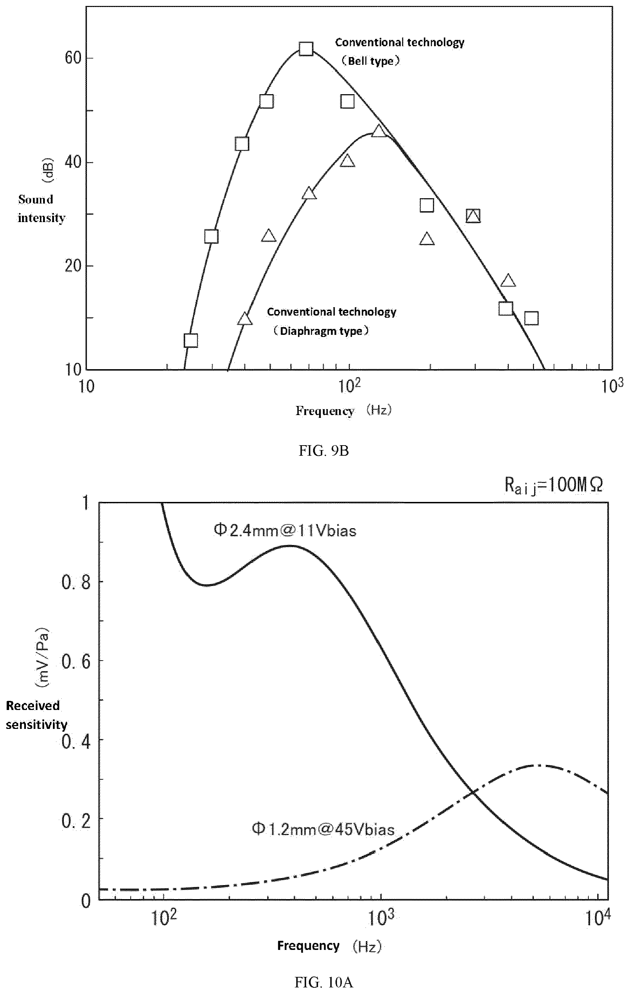

On the other hand, as illustrated in FIGS. 10A and 10B, by varying the value of the direct current voltage $V_{bias}$, which serves as the first potential applied between the upper electrode 25c on the drum-shaped detection unit and the peripheral lower electrode 17o, the resonance frequency and frequency-dependent characteristics of frequency sensitivity of audible range sensors $X_{v1}$-$X_{v4}$ and $X_{w1}$-$X_{w5}$ can be altered respectively. Consequently, by modifying the value of the direct current voltage $V_{bias}$ for each audible range sensor $X_{v1}$-$X_{v4}$ with the same diagonal diameter, the resonance frequency and frequency-dependent characteristics of receiving sensitivity for each sensor can be adjusted. Similarly, by changing the value of the direct current voltage $V_{bias}$ for each audible range sensor $X_{w1}$-$X_{w5}$ with the same diagonal diameter, the resonance frequency and frequency-dependent characteristics of receiving sensitivity for each sensor can be modified.

In other words, by adjusting the value of the direct current voltage $V_{bias}$, it is possible to differentiate the frequency-dependent characteristics of the receiving sensitivity among the nine audible range sensors $X_{v1}$, $X_{v2}$, $X_{v3}$, $X_{v4}$,

$X_{w1}, X_{w2}, X_{w3}, X_{wj}, X_{w4}$, and $X_{w5}$ shown in FIG. 2, thus achieving an overall broadband frequency-dependent characteristic of receiving sensitivity. When the frequency-dependent characteristics of the receiving sensitivity for the nine audible range sensors $X_{v1}, X_{v2}, X_{v3}, X_{v4}, X_{w1}, X_{w2}, X_{w3}, X_{wj}, X_{w4}$, and $X_{w5}$ are differentiated by setting different values of the direct current voltage $V_{bias}$, the amplification factors of the amplifiers $81_{ij}$, which are provided for each audible range sensor $X_{v1}$-$X_{v4}$ and $X_{w1}$-$X_{w5}$, can also be individually controlled. This allows for the adjustment of the nine signal levels input to the shared main processing circuit 82.

Furthermore, by individually adjusting the values of the sensitivity adjustment resistor $Ra_{ij}$ and the direct current voltage $V_{bias}$, all frequency-dependent characteristics of the mechanical resonance frequency and reception sensitivity of the nine audible range sensors $X_{v1}, X_{v2}, X_{v3}, X_{v4}, X_{w1}, X_{w2}, X_{w3}, X_{wj}, X_{w4}$, and $X_{w5}$ shown in FIG. 2 can be set to different values, thereby achieving frequency-dependent characteristics of the overall broadband resonance frequency and reception sensitivity. When the resonance frequency and frequency-dependent characteristics of the reception sensitivity of the nine audible range sensors $X_{v1}, X_{v2}, X_{v3}, X_{v4}, X_{w1}, X_{w2}, X_{w3}, X_{wj}, X_{w4}$, and $X_{w5}$ are all set to be mutually exclusive using the sensitivity adjustment resistor $Ra_{ij}$ and the direct current voltage $V_{bias}$, the amplification factors of the individual amplifiers $81_{ij}$ corresponding to audible range sensors $X_{v1}$-$X_{v4}$ and $X_{w1}$-$X_{w5}$ can also be controlled separately to adjust the levels of the nine signals input to the shared main processing circuit 82.

As illustrated in FIG. 5, each of the channel formation regions 14 constitutes a part of the audible range sensors $X_{v1}$-$X_{v4}$ and $X_{w1}$-$X_{w5}$, and is designated as a corresponding region for the insulated gate semiconductor elements. In other words, as shown in FIG. 5, the insulated gate semiconductor elements are integrated as a part of the respective audible range sensors $X_{v1}$-$X_{v4}$ and $X_{w1}$-$X_{w5}$, such that the central lower electrode 17c of each audible range sensor $X_{v1}$-$X_{v4}$ and $X_{w1}$-$X_{w5}$ shares a common region with the respective gate of the corresponding insulated gate semiconductor element. As depicted in FIG. 4, to drive the insulated gate semiconductor elements integrated as a whole, the power supply wiring $V_{DD}$ is connected to the second main electrode region 15a of the insulated gate semiconductor elements. In FIG. 4, the power supply wiring $V_{DD}$ is exemplified by being connected to the second contact plug on the left side of each audible range sensor $X_{v1}$-$X_{v4}$ and $X_{w1}$-$X_{w5}$ with a hexagonal drum-shaped detection area.

In the following text, each audible range sensor $X_{v1}$-$X_{v4}$ and $X_{w1}$-$X_{w5}$ shall be collectively denoted as "audible range sensor $X_{ij}$" (where i=v, w and j=1-4 or 5). As illustrated in FIG. 4, the audible range sensor $X_{ij}$ integrated into the electronic stethoscope of the first embodiment comprises a perforated hexagonal (hexagonal ring) peripheral lower electrode (fixed potential electrode) 17o and a hexagonal central lower electrode 17o. The central lower electrode 17o is concentrically arranged within the hexagonal opening at the center of the peripheral lower electrode 17o, and is concentrically positioned with the peripheral lower electrode (floating electrode) 17c. The central lower electrode 17c is an electrically floating (floating) flat electrode. The peripheral lower electrode 17o and the central lower electrode 17c constitute the lower electrodes (17c, 17o) of the audible range sensor $X_{ij}$, which serves as an amplifier element integrated sensor. The central lower electrode 17c is electrically floating, while the peripheral lower electrode 17o is connected to a second potential (ground potential). Although FIG. 4 shows a structure in which the peripheral lower electrode 17o and the central lower electrode 17c are divided into concentric hexagons, the topological structure of dividing the lower electrodes (17c, 17o) into peripheral lower electrode 17o and central lower electrode 17c is not limited to the pattern shown in FIG. 4.

For instance, a hexagon can be divided into two parts by a diagonal line passing through the center of the hexagon, with one side facing outward serving as the peripheral lower electrode 17o and the other side serving as the central lower electrode 17c. In this scenario, a topological structure can also be adopted, wherein a groove (notch pattern) is positioned near the center of the peripheral lower electrode 17o on the opposite side of the central lower electrode 17c, and a protrusion located near the center of the peripheral lower electrode 17o is inserted into this groove. Alternatively, the lower electrode can be divided into three or more regions, with each divided lower electrode classified as either a peripheral lower electrode 17o or a central lower electrode 17c. Regions functioning similarly to the peripheral lower electrode 17o are electrically interconnected, and similarly functioning regions to the central lower electrode 17c are also electrically interconnected. These regions can be electrically interconnected. Even if the lower electrode is divided into multiple regions, the regions designated as peripheral lower electrodes 17o are individually connected to a second potential (ground potential), while the regions designated as central lower electrodes 17c are set to electrical floating.

However, in terms of sensitivity characteristics, a topology structure with the central lower electrode 17c and peripheral lower electrodes 17o arranged concentrically is more preferable. When utilizing the split mode, it is advisable to adopt a topology structure where two peripheral lower electrodes 17o are arranged diagonally and hexagonally opposite each other. Subsequently, a groove can be established near the center on both sides opposite the peripheral lower electrodes 17o, forming an opening pattern through the opposing grooves. A concentric topology structure can then be employed, with the central lower electrode 17c inserted into this opening pattern.

In the audible range sensor $X_{ij}$ of the electronic stethoscope in the first embodiment, as illustrated in FIG. 5, the lower electrodes (17c, 17o) featuring a split electrode topology face the upper electrode 25c through the vibration cavity 28, where the upper electrode 25c constitutes a component of the drum-shaped detection component. The planar pattern of the vibration cavity 28 delineates the planar pattern of the drum-shaped detection component. The electric field between the peripheral lower electrode 17o and the upper electrode 25c, which constitute the lower electrodes (17c, 17o), maintains a main voltage during the reception of the audible range sensor $X_{ij}$. Conversely, the first main electrode region 15b and the second main electrode region 15a, depicted as rectangles with dashed lines in FIG. 4, have their short sides oriented towards each other and separated from each other. The central lower electrode 17c, which forms part of the lower electrodes (17c, 17o), remains in a floating state. Consequently, the potential of the channel region formed on the surface of the channel formation region 14 between the first main electrode region 15b and the second main electrode region 15a is governed by electrostatic control, induced by the electric charge sensed by the central lower electrode 17c.

As depicted in FIG. 4, two rectangular grooves (cuts) are established around the periphery of the peripheral lower electrode 17o, serving as planar patterns, thereby forming a hexagonal ring. The first and second contact plugs are respectively positioned within these two grooves. Specifically, the first contact plug 24b is situated in the groove on the right vertical side of the hexagonal planar pattern defined by the periphery of the peripheral lower electrode 17o, while the second contact plug 24a is located in the groove on the left vertical side. Additionally, the third contact plug is positioned diagonally above the right side of the hexagon, encompassing the outer circumference of the peripheral lower electrode 17o.

As illustrated in FIG. 4, the first contact plug 24b is connected to the first main electrode region 15b, facilitating the extraction of current signals from the first main electrode region 15b. This constitutes an insulated gate semiconductor element serving as an audible range sensor $X_{ij}$. Although the example demonstrates the extraction of current signals from the first main electrode region 15b (source region) in the form of a source follower, a configuration in which the source region is grounded and current signals are extracted from the second main electrode region 15a (drain region) is also acceptable. The second contact plug 24a is connected to the second main electrode region 15a, allowing the supply of power voltage $V_{DD}$ from the power supply wiring $V_{DD}$ to the second main electrode region 15a, which contains the insulated gate semiconductor element serving as an audible range sensor $X_{ij}$.

As illustrated in FIG. 5, the audible range sensor utilized in the electronic stethoscope according to the first embodiment comprises a first conductive type (p-type) semiconductor region, with the channel formation region 14 set at a second potential (ground potential GND). The first main electrode region 15b and the second main electrode region 15a of the second conductive type (n-type) are positioned opposite and separated from each other on the surface. Additionally, the audible range sensor is equipped with gate insulating films (12, 16) on the first main electrode region 15b and the second main electrode region 15a, as well as on the channel formation region 14 sandwiched between the first main electrode region 15b and the second main electrode region 15a. The central lower electrode 17c, composed of a conductor layer, is provided in an electrically floating state atop the gate insulating films (12, 16) over the channel formation region 14, functioning similarly to an insulated gate type semiconductor element. The central lower electrode 17c corresponds to the gate electrode of a conventional insulated gate type semiconductor element. The conductor layer of the central lower electrode 17c can be made from a low specific resistance conductor material, such as doped polysilicon (doped polysilicon: DOPOS) thin film. The gate insulating films (12, 16) are not limited to the structure depicted in FIG. 5, although the FIG. shows a dual-layer structure consisting of a lower first gate insulating film 12 and an upper second gate insulating film 16.

However, as illustrated in FIG. 5, a configuration featuring a peripheral lower electrode 17o composed of a conductor layer is further established atop the gate insulating films (12, 16). This conductor layer is situated adjacent to, yet separated from, the central lower electrode 17c and is set at the second potential GND. This arrangement differs from that of conventional insulated-gate semiconductor components. The peripheral lower electrode 17o possesses a central lower electrode 17o. The peripheral lower electrode 17o can employ the same conductor material as the central lower electrode 17c, such as a low-specific-resistance DOPOS film. The central lower electrode 17c and the peripheral lower electrode 17o constitute a split-structure lower electrode (17c, 17o). In other words, a cavity vibration film 23 crafted from an insulating film opposes the lower electrodes (17c, 17o) comprising the central lower electrode 17c and the peripheral lower electrode 17o through a vibration cavity 28, making contact with the upper surface of the cavity vibration film 23. The vibration cavity 28, which is set at the first potential Vbias, features an upper electrode 25c that serves as a constituent element of a drum-shaped detection section, thereby constituting an audible range sensor with an integrated amplification element.

The vibrating cavity 28, situated between the lower electrodes (17c, 17o) and the upper electrode 25c, constitutes a drum-shaped sealed space. In FIG. 5, the insulating film 20, which forms the cavity, is arranged on both the left and right sides of the vibrating cavity 28, encircling it. The insulating film 20, which supports the periphery of the upper electrode 25c, essentially serves as the main body supporting the drumhead (vibrating film) of the drum-shaped detection unit. The support stiffness of the cavity-forming insulating film 20 is a determinant factor in determining the mechanical resonance frequency of the drum-shaped detection unit. In audible range sensors equipped with built-in amplification components, the displacement of the upper electrode 25c, which is a component of the drum-shaped detection unit, can be detected through a characteristic audible range signal $\Phi$ representing the diagnostic object's features. Specifically, this refers to the change in current between the first main electrode region 15b and the second main electrode region 15a. In FIG. 5, two white arrows superimposed on the upper electrode 25c, positioned near the center of its drum-shaped detection area, indicate the displacement of the upper electrode 25c under the influence of the characteristic audible range signal Φ.

As illustrated in FIG. 5, in addition to the upper electrode 25c, a first surface wiring layer 25b and a second surface wiring layer 25a are also provided on the upper surface of the cavity diaphragm 23, located outside the vibration cavity 28. A first contact plug 24b is situated between the first surface wiring layer 25b and the first main electrode region 15b, penetrating through the cavity diaphragm 23, the cavity-forming insulating film 20, the second gate insulating film 16, and the first gate insulating film 12. Similarly, a second contact plug 24a is positioned between the second surface wiring layer 25a and the second main electrode region 15a, capable of penetrating through the cavity diaphragm 23, the cavity-forming insulating film 20, the second gate insulating film 16, and the first gate insulating film 12. In essence, the upper end of the first contact plug 24b is metallically connected to the first surface wiring layer 25b, while its lower end is electrically connected to the first main electrode region 15b, thus establishing electrical continuity between the first surface wiring layer 25b and the first main electrode region 15b via the first contact plug 24b.

Similarly, the upper end of the second contact plug 24a is metallurgically connected to the second surface wiring layer 25a, while the lower end of the second contact plug 24a is electrically connected to the second main electrode region 15a. Consequently, the second surface wiring layer 25a and the second main electrode region 15a are electrically connected through the second contact plug 24a. The first surface wiring layer 25b corresponds to the output signal line $R_i$ shown in FIG. 4. On the other hand, the second surface wiring layer 25a corresponds to the power supply wiring $V_{DD}$ shown in FIG. 4. On the upper part of the cavity diaphragm 23, an upper electrode protective film 26, such as a silicon oxide film, is coated to cover the upper electrode 25c, the first surface wiring layer 25b, and the second surface wiring layer 25a, which are components of the drum-shaped detection section. Additionally, a tip protective film 34, such as a polyimide film, is laminated on the upper electrode protective film 26.

The cavity vibration film 23, the upper electrode 25c, the upper electrode protective film 26, and the tip protective film 34 constitute the "vibration section (23, 25c, 26, 34)" of the drum-shaped detection component. The vibration section (23, 25c, 26, 34) is a multilayer film corresponding to the drumhead (vibration film) of the drum. The dimensions (diagonal diameter) of the vibration section (23, 25c, 26, 34) are determined by factors such as the planar pattern of the vibration cavity 28, the hardness of the corresponding materials, and the thickness of the corresponding materials, which determine the mechanical resonance frequency of the drum-shaped detection component. The tip protective film 34 blocks the liquid introduction hole 27, through which liquid is removed during the formation of the vibration cavity 28, allowing for selective dissolution and removal of the sacrificial layer through wet etching. The liquid introduction hole 27 penetrates the upper electrode protective film 26 and the cavity vibration film 23 to reach the vibration cavity 28, which defines the drum-shaped detection area. By blocking the liquid introduction hole 27, the interior of the vibration cavity 28 is maintained in a decompressed inert gas environment.

In FIG. 5, the capacitance formed between the upper electrode 25c and the central lower electrode 17c near the center of the vibration cavity 28 is denoted as $C_1$. This capacitance defines the planar pattern of the drum-shaped detection area. The capacitance value of $C_1$ varies with the displacement of the upper electrode 25c, thus constituting a variable capacitor, as illustrated in FIGS. 6A to 6C. The capacitance value of $C_1$ influences the mechanical resonance frequency of the drum-shaped detection component, which is determined by the dimensions, hardness, and thickness of the vibrating components (23, 25c, 26, 34) as well as the support stiffness of the cavity-forming insulating film 20. Consequently, the thickness of the gap behind the vibrating components (23, 25c, 26, 34) is a factor that affects the mechanical resonance frequency of the drum-shaped detection component. In FIG. 4, the inter-electrode capacitance of the hexagonal ring, which is formed by the peripheral lower electrode 17o surrounding the capacitor $C_1$ and the upper electrode 25c, is denoted as $C_2$. The inter-electrode capacitance $C_2$ is formed between the peripheral lower electrode 17o and the upper electrode 25c at the ground potential (second potential) GND. As shown in FIGS. 6A to 6C, the capacitance value of $C_2$ also varies with the displacement of the upper electrode 25c, thus constituting a variable capacitor. The capacitance value of $C_2$ also affects the mechanical resonance frequency of the drum-shaped detection component, which is determined by the dimensions, hardness, and thickness of the vibrating components (23, 25c, 26, 34) as well as the support stiffness of the cavity-forming insulating film 20.

To distinguish between these two variable capacitors, they will be referred to hereinafter as "the first variable capacitor $C_1$" and "the second variable capacitor $C_2$". The second variable capacitor $C_2$ is a capacitor that functions similarly to the capacitors configured between the upper and lower electrodes in traditional capacitive devices, such as cMUTs. On the other hand, the first variable capacitor $C_1$ is a capacitor used for charge induction, which is necessary for driving the insulated gate-type semiconductor component that performs the specific function of the hearing range sensor in the electronic stethoscope of the first embodiment. Additionally, in FIG. 5, the gate-source capacitance $C_{gs}$ between the central lower electrode 17c and the first main electrode region 15b, the gate-drain capacitance $C_{gd}$ between the central lower electrode 17c and the second main electrode region 15a, and the insulating film capacitance $C_{OX}$ between the central lower electrode 17c and the channel formation region 14 are depicted.

In the equivalent circuits depicted in FIGS. 6A to 6C, the series circuit of the DC supply line $V_{bias}$ at the first potential and the output resistance (internal resistance of the power supply) $R_O$ is located at the left end of the paper, outside the area $X_{vj}$ ($X_{wj}$) enclosed by the left dashed line. In FIGS. 6A to 6C, the horizontal line extending from the upper part of the area $X_{vj}$ ($X_{wj}$) towards the left edge and protruding from the area $X_{vj}$ ($X_{wj}$) corresponds to the DC supply line $V_{bias}$, which provides the first potential voltage shown in FIG. 4. In FIGS. 6A to 6C, a circuit is shown where the sensitivity adjustment resistor $R_{aij}$ is connected in parallel within the series circuit of the DC supply line $V_{bias}$, at the first potential and the output resistance $R_O$. The DC supply line $V_{bias}$ is grounded through the sensitivity adjustment resistor $Ra_{ij}$.

FIG. 6B illustrates an equivalent circuit in the form of a source follower similar to that shown in FIG. 6A, but with the central lower electrode 17c grounded through a sensitivity adjustment resistor $Ra_{ij}$. If the sensitivity adjustment resistor $Ra_{ij}$ is sufficiently large, the central lower electrode 17c can still function as an analog central lower electrode even when grounded through the sensitivity adjustment resistor $Ra_{ij}$. Subsequently, within the dashed area $X_{vj}$ ($X_{wj}$) in FIGS. 6A and 6C, on the right side, one end of the first variable capacitor $C_1$ is grounded through the sensitivity adjustment resistor $Ra_{ij}$ and a series circuit of an n-channel MOSFET (hereinafter referred to as "nMOSFET"). In FIG. 6B, the equivalent circuit is represented as the connection node between the insulating film capacitor $C_{OX} \fallingdotseq C_3$ of the nMOSFET and the first variable capacitor $C_1$, which is grounded through the sensitivity adjustment resistor $Ra_{ij}$. The sensitivity adjustment resistor $R_{aij}$ shown in FIGS. 6A to 6C can be adjusted by the display and control unit 5 of the sound collection unit 9a based on environmental noise and various physiological parameters (as shown in FIG. 12).

However, the nMOSFET depicted in FIGS. 6A to 6C serves merely as an example, and various insulated gate semiconductor components described in the introductory section of "Implementations of the Disclosure" can also be employed. In addition to the insulated gate semiconductor components mentioned in the initial part of "Implementations of the Disclosure," other insulated gate semiconductor components such as high electron mobility transistors (HEMTs) can also be utilized. In the equivalent circuits illustrated in FIGS. 6A to 6C, the first potential DC supply line $V_{bias}$ is connected in parallel to a series circuit comprising the first variable capacitor $C_1$ and the nMOSFET, as well as to the second variable capacitor $C_2$. FIGS. 6A and 6B depict the equivalent circuit diagrams for the case where the output is in the form of a source follower, where the source of the nMOSFET is connected to the second potential (ground potential) through a source resistor $R_{SS}$, and the current output from the source is directed to an amplifier $81_{ij}$, which is specifically configured for the audible range sensor $X_{ij}$.

In other words, the signal level input to amplifier $81_{ij}$ is exceptionally high, as the signal amplified by the built-in nMOSFET is directed to amplifier $81_{ij}$, which is specifically designed for the audible range sensor $X_{ij}$. Consequently, a relatively high signal-to-noise ratio (SN ratio) can be achieved for the input signal to amplifier $81_{ij}$. Due to the high SN ratio of the input signal to amplifier $81_{ij}$, the SN ratio of the output signal from amplifier $81_{ij}$ is also high, enabling a high level of signal processing to remove noise signals in the main processing circuit 82 subsequent to amplifier $81_{ij}$. Therefore, even when the audible range signal, which is a characteristic of the sound source, is under weak environmental noise conditions, a pure audible range signal with a relatively high SN ratio can be output from the main processing circuit 82 of the signal processing circuit 97.

In the equivalent circuit depicted in FIG. 6C, the DC supply line $V_{bias}$ at the first potential is also connected in series with the first variable capacitor $C_1$ and the nMOSFET series circuit, as well as in parallel with the second variable capacitor $C_2$. FIG. 6C illustrates the equivalent circuit diagram of the current signal originating from the drain region side. Consequently, the drain of the nMOSFET is connected to the power supply $V_{DD}$ through the drain resistor $R_{DD}$, and the current output from the drain is directed to amplifier $81_{ij}$, which is specifically configured for the audible range sensor $X_{ij}$. In FIGS. 6A to 6C, the fundamental configuration comprises the second variable capacitor $C_2$, with one electrode set at the second potential $V_{bias}$, along with the nMOSFET series circuit and the first variable capacitor $C_1$ connected in parallel with this second variable capacitor $C_2$.

As shown in FIG. 6D, within the insulated-gate semiconductor component integrated into the audible range sensor $X_{ij}$ of the electronic stethoscope in the first embodiment, the central lower electrode 17c serves as one electrode of the first variable capacitor $C_1$, corresponding to the control electrode (gate electrode) of a conventional insulated-gate semiconductor component. Typically, in insulated-gate semiconductor components such as nMOSFET, the gate-source capacitance $C_{gs}$, gate-drain capacitance $C_{gd}$, and insulating film capacitance $C_{OX}$ are distributed around the gate. Roughly estimating, the capacitance surrounding the central lower electrode 17c, which corresponds to the gate, is approximately $C_3 = C_{OX}$. The equivalent circuit depicted in FIGS. 6A to 6C can be represented by the capacitance $C_3$. Unlike the first variable capacitor $C_1$ and the second variable capacitor $C_2$, the value of capacitance $C_3$ can be approximated by the insulating film capacitance $C_{OX}$, which is a fixed capacitance because it remains unchanged even if the upper electrode 25c undergoes displacement. Consequently, the capacitance $C_3$ constituted by the central lower electrode 17c will be referred to as "fixed capacitance $C_3$". For simplicity, the fixed capacitance $C_3$ should be considered as the gate-to-substrate capacitance as shown in FIG. 6D.

Firstly, in the series circuit of the first variable capacitor $C_1$ and the fixed capacitor $C_3$, as illustrated in the equivalent circuits of FIGS. 6A to 6C, the investigation focuses on determining the voltage applied across the fixed capacitor $C_3$. Assuming that the amount of charge induced in the first variable capacitor $C_1$ is $q_1$, the voltage across the first variable capacitor $C_1$ is $V_1$, the amount of charge induced in the fixed capacitor $C_3$ is $q_3$, and the voltage across the fixed capacitor C3 is $V_3$, we have $q_1 = q_3 = q$ in the series circuit. Therefore, the voltage $V_{bias}$ across the series circuit of the first variable capacitor $C_1$ and the fixed capacitor $C_3$ can be expressed as follows:

(Number 1)

$$V_{bias} = V_1 + V_3 = \frac{q}{C_1} + \frac{q}{C_3} = \frac{(C_1 + C_3)\,q}{C_1 C_3} \qquad\cdots\cdots\cdots\quad (1)$$

In FIG. 5, the area of the central lower electrode 17c is $S_1$, the electrode spacing between the upper electrode 25c and the central lower electrode 17c that constitute the first variable capacitor $C_1$ is d, and the thickness $t_{OX}$ ($=T_{OX1}+T_{OX2}$) of the gate insulating film (12, 16) that constitutes the fixed capacitor $C_3$. In addition, assuming that the dielectric constant of vacuum is $\varepsilon_0$ and the relative dielectric constant of silicon oxide film is $\varepsilon_r$,

$$C_1 = \varepsilon_0 S_1/d \qquad\qquad \cdots\cdots\cdots (2)$$

$$C_3 = \varepsilon_0 \varepsilon_r S_1/t_{OX} \qquad\qquad \cdots\cdots\cdots (3)$$

Therefore, by performing capacitance division in the series circuit of the first variable capacitor $C_1$ and the fixed capacitor $C_3$, the voltage generated across the fixed capacitor $C_3$ is $V_3$ as shown below,

(Number 2)

$$\frac{V_3}{V_{bias}} = \frac{\dfrac{q}{C_3}}{\dfrac{(C_1 + C_3)\,q}{C_1 C_3}}$$

$$= \frac{C_1}{(C_1 + C_3)}$$

$$= \frac{\varepsilon_0 S_1 / d}{\varepsilon_0 S_1 / d + \varepsilon_0 \varepsilon_r S_1 / t_{OX}}$$

$$= \frac{1}{1 + d\,(\varepsilon_r / t_{OX})} \qquad\qquad \cdots\cdots\cdots (4)$$

For example, assuming $d=2\mu m$, $t_{OX}=100nm$, and the relative dielectric constant of the silicon oxide film is $\varepsilon_r=4.0$, formula (4) shows that 1/81 of the DC voltage $V_{bias}$ is applied to the voltage $V_3$ across the fixed capacitor $C_3$. However, since the area $S_1$ of the central lower electrode 17c for the audible region is larger than the area $S_{AA}$ of the active area of the insulated gate semiconductor element ($S_{AA} \ll S_1$), it is not necessary to completely position the gate insulating film of the insulated gate semiconductor element directly below the central lower electrode 17c. The size of the active area $S_{AA}$ is defined as (gate length) $\times$ (gate width). The value of the fixed capacitance $C_3$ can be arbitrarily adjusted by selecting the thickness $t_{OX}$ of the gate insulating film (12, 16) and the area ratio of the active area $S_{AA}$ to the area $S_1$ of the central lower electrode 17c. Therefore, by selecting the thickness $t_{OX}$ and the size of the active area $S_{AA}$, the value of equation (4) can be adjusted to within a desired range, up to approximately 1/2. Therefore, if the DC voltage $V_{bias}$ is selected as a given value and the structure of the hearing range sensor of the electronic stethoscope is selected to adjust the value of $d(\varepsilon_r/t_{OX})$ in equation (4), the gate voltage of the nMOSFET can be selected to make the nMOSFET operate in the linear region. The question is whether it is possible to obtain a change in the nMOSFET gate voltage $\Delta V_3$ corresponding to the slight displacement $\Delta d$ of the upper electrode 25c when applying a characteristic audible range signal indicating the characteristics of the diagnostic object to cause a slight displacement of the upper electrode 25c. It can be seen from equation (2) that when the electrode spacing d between the upper electrode 25c and the central lower electrode 17c is shifted by $\Delta d$, the capacitance change $\Delta C_1$ of the first variable capacitor $C_1$ can be expressed as,

$$C_1 + \Delta C_1 = \varepsilon_0 S_1/(d-\Delta d) \qquad \cdots\cdots\cdots (5)$$

On the other hand, equation (4) can be rewritten as,
(Number 3)

$$V_3 = \frac{1}{1+(\varepsilon_r / t_{0X})\, d}\; V_{bias} \qquad \cdots\cdots\; (6)$$

If we use $\partial d$ to differentiate the multivariable function in equation (6), it will be as follows: (Number 4)

$$\frac{\partial V_3}{\partial d} = -\left(\frac{\varepsilon_r}{t_{OX}}\right)\frac{1}{(1+(\varepsilon_r / t_{0X})\, d)^2}\; V_{bias} \qquad \cdots\cdots\; (7)$$

Equation (7) is a multivariable function with variables d and $t_{OX}$.

When considering the variable d, it can be seen that when the electrode spacing d between the upper electrode 25c and the central lower electrode 17c is shifted by $\Delta d$, the change in the gate voltage $\Delta V_3$ of the nMOSFET can be represented by the following equation (8).
(Number 5)

$$\Delta V_3 = -\left(\frac{\varepsilon_r}{t_{OX}}\right)\frac{1}{(1+(\varepsilon_r / t_{0X})\, d)^2}\; V_{bias}\; \Delta d \qquad \cdots\cdots\; (8)$$

Equation (8) shows that in order to increase the gate voltage change $\Delta V_3$ when the electrode spacing d is shifted by $\Delta d$, it is best to increase the relative dielectric constant $\varepsilon_r$ of the gate insulating film, reduce the thickness $t_{OX}$ of the gate insulating film, reduce the electrode spacing d, and increase the first potential DC voltage $V_{bias}$. Traditional capacitive components such as cMUT can detect changes in charge as a quantitative variable. However, since the charge value of each element as a quantitative variable decreases as the element becomes thinner, miniaturizing each element to achieve an easily audible electronic stethoscope can pose difficulties in installing the receiver circuit.

On the other hand, according to the audible range sensor in the first embodiment shown in FIG. 5, because the change in voltage generated in the central lower electrode 17c as shown in formula (8) is detected as a strong variable, even if the component is miniaturized, its value will not decrease. Equation (8) is a multivariable function, so if the value of $\varepsilon_r/t_{OX}$ is considered as a variable, the absolute value of equation (8) is a convex function that peaks when $\varepsilon_r/t_{OX}=1/d$. That is, for the film thickness $t_{OX}$ of the gate insulating film, when the electrode spacing d is a value that is a multiple of the relative dielectric constant ($\varepsilon_r$), the function value of the absolute value of expression (8) is maximized, so the film thickness $t_{OX}$ of the gate insulating film can also be thinned as the electrode spacing d changes. However, there are limitations in manufacturing technology and physical properties to make the gate insulating film thickness $t_{OX}$ and electrode spacing d thinner. As shown in (8), if the change in gate voltage $\Delta V_3$ is increased according to the change in $\Delta d$, the DC voltage $V_{bias}$ can be effectively increased. As described in formula (4), by selecting the first potential DC voltage $V_{bias}$, the nMOSFET can select the operating point of the gate voltage, allowing it to operate in the linear region. If the DC voltage $V_{bias}$ is excessively increased according to the requirements of equation (8), the operating point will exceed the linear region. It can be seen that insulated gate SITs (such as MOSSIT and MISSIT) exhibit a triode-type drain voltage-drain current characteristic. Since all operating regions are within the linear region, the audible range sensor covered by the first embodiment is preferred as an insulated gate semiconductor component.

Using the mutual conductance $g_m$ of MOSFET, the change in drain current $I_{ds}$, $\Delta I_{ds}$, is as follows, (Number 6)

$$\Delta I_{DS} = -g_m\left(\frac{\varepsilon_r}{t_{OX}}\right)\frac{1}{(1+(\varepsilon_r / t_{0X})\, d)^2}\; V_{bias}\; \Delta d \qquad \cdots\cdots\; (9)$$

That is, according to the audible range sensor of the first embodiment, as shown in equation (8), by using the voltage change $\Delta V_3$ received by each element as the gate voltage change of the insulated gate semiconductor element built into each element, the amplification function of the insulated gate semiconductor element can detect large current changes. Therefore, by using an audible range sensor with high receiving sensitivity, an electronic stethoscope that is easy to auscultate can be realized.

By using the insulated gate semiconductor element of the nMOSFET as the component assembly element of the audible range sensor covered by the first embodiment, it can be seen that the voltage generated by the capacitive voltage division of the central lower electrode 17c can drive the insulated gate semiconductor element and detect the displacement of the upper electrode 25c as a current change $\Delta I_{ds}$. The gate voltage change value $\Delta V_3$ of the nMOSFET shown in equation (7) is a function that gradually decreases as the electrode spacing d between the upper electrode 25c and the central lower electrode 17c increases, so the smaller the electrode spacing d, the better. On the other hand, as mentioned earlier, if the thickness $t_{OX}$ of the gate insulating film is taken as a variable, the function showing the absolute value in formula (7) exhibits a convex curve shape with a maximum value.

Therefore, considering the shape of the curve of the absolute value of the gate voltage change $\Delta V_3$ as a function of the display thickness $t_{OX}$ as a variable, there is an optimal value for the thickness $t_{OX}$ of the gate insulating film, which depends on the distance d between the electrodes. If the electrode spacing d decreases, the thickness $t_{OX}$ of the gate insulating film, which is the optimal value, also decreases, and the withstand voltage of the gate insulating film decreases. Therefore, the optimal value of the gate dielectric film thickness $t_{OX}$ depends on the distance d between the electrodes, which has been explained. Therefore, if the relative dielectric constant $\varepsilon_r$ of the gate dielectric film and the reciprocal of the thickness $t_{OX}$ of the gate dielectric film $(\varepsilon_r / t_{OX})=x$ are normalized, the multivariate function in formula (7) can be rewritten as: (Number 7)

$$\frac{\partial V_3}{\partial d} = -x\frac{1}{(1+dx)^2}V_{bias} = -V_{bias}\frac{f(x)}{g(x)} \qquad \cdots\cdots\cdots (10)$$

In the formula (10), $f(x)=x$ and $g(x)=(1+dx)^2$. If we use $f_x(x)$ to represent the derivative of the differential of x based on $f(x)$, and use $g_x(x)$ to represent the derivative of the differential of x based on $g(x)$, then it is as follows:

$$f_x(x)=1 \qquad\qquad \cdots\cdots\cdots (11)$$

$$g_x(x)=2d+2d^2x=2d(1+dx) \qquad \cdots\cdots\cdots (12)$$

therefore

$$f_x(x)g(x)-f(x)g_x(x)=(1+dx)^2-2xd(1+dx)$$

$$=1-d^2x^2 \qquad \cdots\cdots\cdots (13)$$

The result of partial differentiation of the multivariate function of equation (7) with respect to variable x using equation (13) is as follows:
(Number 8)

$$\frac{\partial^2 V_3}{\partial x\,\partial d} = -\frac{1-d^2x^2}{(1+dx)^4}V_{bias} \qquad\qquad \cdots\cdots\cdots (14)$$

That is, the multivariable function representing the absolute value of the gate voltage change $\Delta V_3$ shown in equations (7) and (10) has a maximum value when $d^2x^2=1$ for the variable x. $d^2x^2=1$, which makes the value of formula (14) zero, is equivalent to $dx=1$. Therefore, the multivariate function representing the absolute value of the gate voltage change $\Delta V_3$ shown in expressions (7) and (10) represents the maximum value at this time,

$$d/\varepsilon_0=t_{OX}/\varepsilon_0\varepsilon_r \qquad\qquad \cdots\cdots\cdots (15)$$

That is, when the distance between the electrodes of the first variable capacitor $C_1$ and the distance between the electrodes of the fixed capacitor $C_3$ are equalized by standardization with their respective dielectric constants, the absolute value of the gate voltage change $\Delta V_3$ reaches a maximum value. This means that, as shown in equations (2) and (3), when the value of the first variable capacitor $C_1$ is equal to the value of the fixed capacitor $C_3$, as shown in equation (7), the absolute value function representing the change in the nMOSFET gate voltage $\Delta V_3$ is at its maximum.

FIG. 6D shows a large signal equivalent circuit different from a general nMOSFET, with a structure in which an $n^+$ type source region (first main electrode region) 15b and an $n^+$ type drain region (second main electrode region) 15a are opposite to each other on the surface of the p-type channel formation region 14 shown in FIG. 5. Unlike the structure shown in FIG. 5, in FIG. 6D, above the channel regions of the first main electrode region (source region) 15b and the second main electrode region (drain region) 15a, there are two layers of gate insulating films (12, 16), including a first gate insulating film 12 with a thickness of $T_{OX2}$ and a second gate insulating film 16 with a thickness of $T_{OX1}$. The gate structure has a central lower electrode 17c as a gate. The active electrode S is connected to the first main electrode region (source region) 15b, and the drain electrode D is connected to the second main electrode region (drain region) 15a.

As with a general nMOSFET, as shown in FIG. 6D, there is a gate-source capacitance $C_{gs}$ between the central lower electrode 17c and the first main electrode region 15b as the gate, a gate-drain capacitance $C_{gd}$ between the central lower electrode 17c and the second main electrode region 15a, and a gate-substrate capacitance $C_{OX}$ between the central lower electrode 17c and the channel formation region 14. In addition, there is a source-to-substrate capacitance $C_{Bs}$ between the first main electrode region 15b and the channel formation region 14, and a drain-to-substrate capacitance $C_{Bd}$ between the second main electrode region 15a and the channel formation region 14.

In traditional capacitive MEMS devices (such as cMUT), there is a problem that the deflection of the upper electrode and the cavity diaphragm is not the same when ultrasound is input to the MEMS device. That is, the problem is that only near the center of the vibration cavity 28, the upper electrode and the lower electrode are close to each other in a shape of a protrusion below. For example, in the structure shown in FIG. 7, unless some measures are taken, the distance between the upper electrode 25c at the position around the central lower electrode 17c and the peripheral lower electrode 17o tends to be greater than the distance d between the upper electrode 25c near the center of the vibration cavity 28 and the central lower electrode 17c. Therefore, in the structure shown in FIG. 7, a silicon nitride film ($\varepsilon_r$=7.0-7.8) with a higher relative dielectric constant than the silicon oxide film $\varepsilon_r$=3.9-4.5 is inserted into the cavity formed by the insulating film 20 as a field strengthening layer 19, and the vibration cavity 28 is surrounded as a drum-shaped sealed space. This can improve the deflection shape problem, that is, the curvature of the central part of the cavity diaphragm 23 becomes larger. Similarly, as shown in FIG. 7, by selecting a silicon nitride film as an insulating film with high rigidity, using the silicon nitride film as a rigid reinforcement cover 31c on the upper electrode protection film 26, and using a silicon nitride film as the cavity vibration film 23 directly below the upper electrode 25c, a rigid reinforcement structure can also be used to further reduce the curvature of the cavity vibration film 23 protruding downward.

As mentioned above, by using an array composed of multiple audible range sensors $X_{ij}$ (each sensor has its own mechanical resonant frequency within the audible range), the desired characteristic audible range signal can be effectively detected as a voltage signal. In particular, when each of the plurality of audible range sensors $X_{ij}$ integrates an insulated gate semiconductor as an internal structure, the detected characteristic audible range signal can be further amplified by the electrostatic induction effect of the internal structure. Therefore, according to the electronic stethoscope of the first embodiment, even in an environment where the characteristic audible signal as the sound source is weak compared to the noise level, a high SN ratio can be achieved, thereby realizing an electronic stethoscope with high objectivity and auditory accuracy.

**Second embodiment**

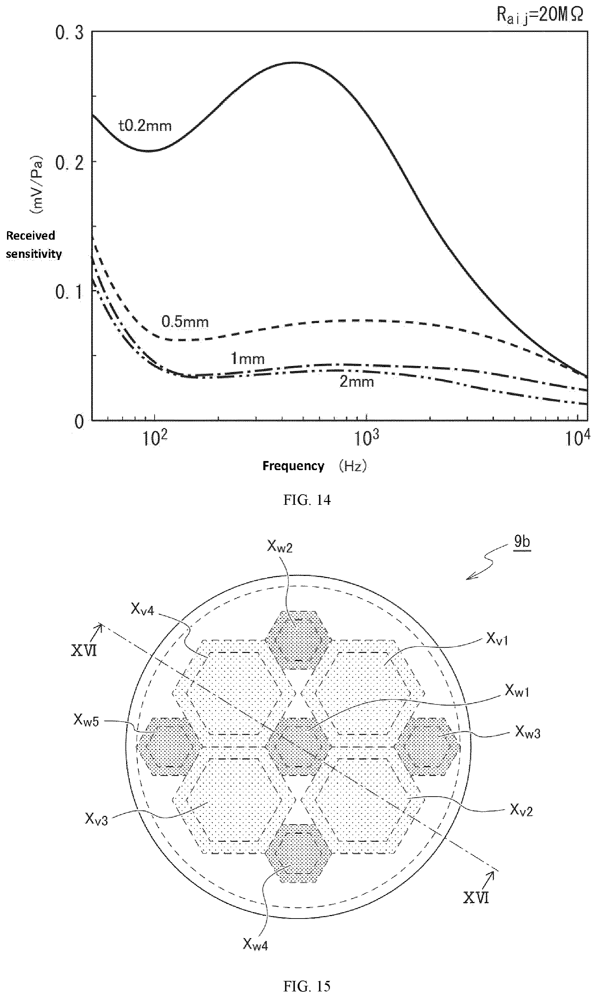

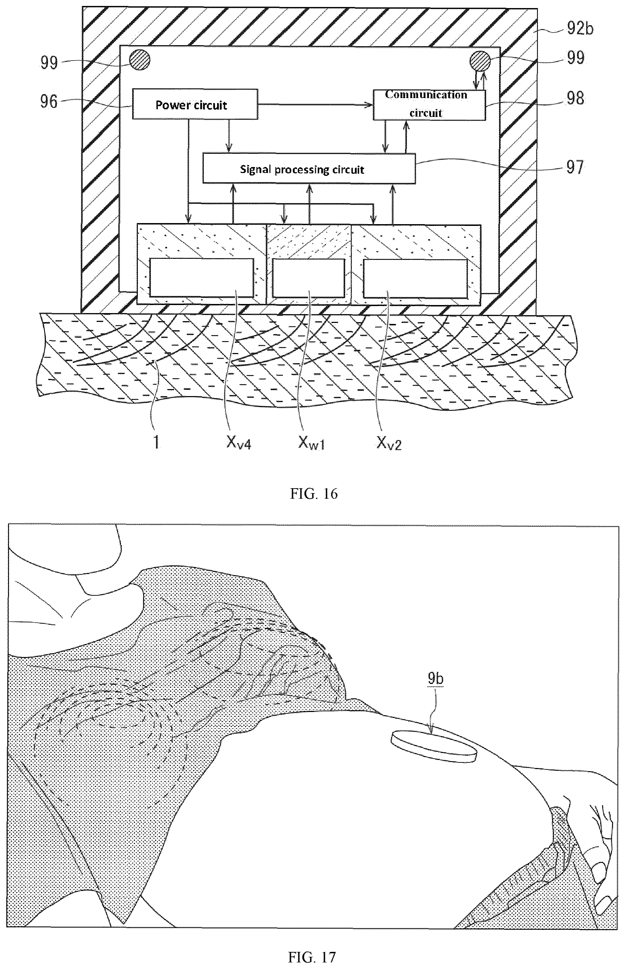

The electronic stethoscope of the first embodiment is provided with a buffer film 93 at the lower part of the housing 92a, as shown in FIG. 3a and other figures. Its structure is similar to that of traditional acoustic stethoscopes, including a buffer film (housing vibration film) 93 and an air layer between the signal input surface. In the electronic stethoscope of the first embodiment, once the characteristic audible range signal generated by the diagnostic object 1 is transmitted to the air layer, it becomes air vibration in the air layer and is captured by the audible range sensor $X_{ij}$. This results in a notable difference in acoustic impedance between the diagnostic object 1 and the air layer, leading to a significant reduction in sensitivity. In addition, if an air layer is arranged between the buffer film 93 and the signal input surface as shown in FIG. 3A and the like, the air in the air layer may become an additional noise source due to thermal vibration. Therefore, the bottom panel-shaped portion of the sound collection part 9b of the electronic stethoscope according to the second embodiment of the present disclosure, that is, a part of the outer skin of the housing 92b, directly contacts the surface of the diagnostic object 1 such as a living body, as shown in FIGS. 15 and 16, which will be explained below.

The flat plate-shaped portion of the bottom side skin of the housing 92b has a storage cavity surrounded by the skin for storing a plurality of audible range sensors $X_{ij}$, each of which has a mechanical resonant frequency within the audible range.

Referring to FIG. 5 described in the first embodiment, the curve of the receiving sensitivity of the drum-shaped audible range sensor (audible range electroacoustic transducer) varies with frequency, reaching a peak resonance frequency, and the six factors that determine the frequency characteristics are ranked in order of importance, as shown in FIG. 6,

(Factor 1) The rigidity of the materials used in the vibration components (23, 25c, 26, 34);
(Factor 2) Thickness of each plate in the vibration section (23, 25c, 26, 34);
(Factor 3) Diameter of the vibrating part (23, 25c, 26, 34);
(Factor 4) The support stiffness of the cavity-forming insulating film 20;
(Factor 5) The value of the sensitivity adjustment resistor $R_{aij}$; as well as
(Factor 6) The height of the vibration cavity 28.

In the following FIGS. 15 and 16, a structure is installed in the housing 92b of the sound collection part 9b including the electronic stethoscope, in which the resonant frequency of the audible range sensors $X_{w1}$-$X_{w5}$ is nearly one order of magnitude different from that of the audible range sensors $X_{v1}$-$X_{v4}$. Before describing the specific structure of the electronic stethoscope according to the second embodiment, the simulation results of the receiving sensitivity (voltage sensitivity) and the like when the audible range sensor $X_{ij}$ is directly in contact with the surface of the diagnostic object 1 such as a living body without using an air layer like the electronic stethoscope according to the first embodiment will be described. The simulation was conducted using the piezoelectric wave analysis software PZFlex, which was originally developed by the American company Weidlinger Associates. During the simulation process, both mechanical vibration generated by the input of characteristic auditory signals in the vibration cavity and electrical vibration caused by changes in the first variable capacitor $C_1$ and the second variable capacitor $C_2$ due to mechanical vibration are considered. Given the vibration of the drum in the drum-shaped sound sensor, its circular shape can be approximated to the topological structure of the hexagonal drum-shaped detector shown in FIG. 4.

In the simulation, the resonant frequencies of the audible range sensors $X_{v1}$-$X_{v4}$ and the audible range sensors $X_{w1}$-$X_{w5}$ installed in the housing 92b including the electronic stethoscope sound collection portion 9b are different. In this case, if the single chips of the audible range sensors $X_{v1}$-$X_{v4}$ and the audible range sensors $X_{w1}$-$X_{w5}$ are manufactured using the same manufacturing process, the resonant frequencies of the audible range sensors $X_{v1}$-$X_{v4}$ and $X_{w1}$-$X_{w5}$ can be easily changed by changing the planar pattern size of the third factor, while maintaining the first, second, fourth, and sixth factors in common. As described in the first embodiment, in the following description of the second embodiment, the audible range sensors $X_{v1}$-$X_{v4}$ and $X_{w1}$-$X_{w5}$ are described at any time using the inclusive expression "audible range sensor $X_{ij}$".

The curves shown by the solid line and the dotted line in FIG. 8 are different sizes of the planar pattern, which is the third factor determining the frequency characteristics. Each solid curve and single-point curve in FIG. 8 corresponds to the simulated receiving sensitivity (voltage sensitivity) of an auditory range sensor with a concentric, separated circular central lower electrode pattern and a circular ring-shaped peripheral lower electrode. The left vertical axis of FIG. 8 shows the voltage change of the central lower electrode in the audible range sensor $X_{ij}$ with a concentric grid structure, which is the receiving sensitivity, while the horizontal axis shows the frequency change.

However, the solid line and the dashed line in FIG. 8 show the simulation results when the amplification function of the integrated insulated gate semiconductor component in the audible range sensor is ignored, as shown in formula (9). It should be noted that, as shown in formula (8), the change in gate voltage $\Delta V_3$ of the insulated gate semiconductor element represents the receiving sensitivity during output. In addition, it is assumed that the structure has an upper electrode protection film 26 made of a $Si_3N_4$ film with a thickness of $10.0\mu m$ on the entire surface above the upper electrode 25c and around the cavity forming the insulating film 20 above the vibration cavity 28. Above the upper electrode protection film 26 is a front end protection film 34 made of room temperature curing silicone rubber with a thickness of 0.2mm, which constitutes the vibration part (25c, 26, 34). However, unlike the structure shown in FIG. 5, the vibration part (25c, 26, 34) omits the cavity vibration film 23 directly below the upper electrode 25c.

In the simulation, similar to the drum-shaped structure shown in FIG. 5, an insulating film 20 made of $SiO_2$ thin film that forms a cavity surrounds the vibration cavity 28 that determines the drum-shaped detection portion and supports the edges (peripheries) of the vibration portions (25c, 26, 34). Unlike the structure shown in FIG. 7, this simulation did not use an electric field enhancement layer 19o composed of $Si_3N_4$ thin film around the vibration cavity 28. The curve shown by the solid line in FIG. 8 indicates that the height of the vibration cavity 28 (the sixth factor determining frequency characteristics) is set to $4.4\mu m$. On the other hand, the curve indicated by the dotted line in FIG. 8 sets the height of the vibration cavity 28 to $3.8\mu m$. The height of the vibration cavity 28 corresponds to the "body height" of the drum.

In addition, in the electronic stethoscope of the second embodiment, unlike the first embodiment, as shown in FIG. 16 below, by bringing the sound collection component 9a into contact with the surface of the diagnostic object 1, it is possible to listen to the sound (characteristic audible range signal) generated in the diagnostic object 1 such as a living body without an air layer. The frequency characteristics of the electronic stethoscope according to the second embodiment depend on the structure of the diagnostic object 1 such as a living body as an elastic body and the sound collecting part 9a. The change in resonance frequency depends on the mass of the sound-collecting component 9a and the elastic constant of the diagnostic object 1. It should also be noted that the simulation results shown by the solid line and the dotted line in FIG. 8 are used to approximate the elastic constants of the diagnostic object 1 using the physical properties of water, which accounts for the majority of the organism's weight (approximately 60%).

The topology of the concentric hexagon shown in FIG. 4 is roughly the same, with the concentric separation pattern of the central lower electrode 17c, which is separated from the peripheral lower electrode 17o, arranged inside the circular opening set in the center of the circular pattern of the outer peripheral lower electrode 17o as the object, simulating the frequency dependence of sensitivity characteristics. The solid line in FIG. 8 shows that the diameter of the circular central lower electrode 17c is 0.94mm, and the diameter of the peripheral lower electrode 17o is 3mm. The inner diameter of the peripheral lower electrode 17o is $94\mu m$ away from the outer diameter of the central lower electrode 17c in the radial direction. Therefore, the solid line in FIG. 8 represents the case where the outer diameter of the upper electrode 25c, which is opposite to the peripheral lower electrode 17o and the central lower electrode 17c, is also 3mm, and the outer diameter of the vibration part (25c, 26, 34) as the third element, which is composed of the upper electrode 25c, the upper electrode protective film 26, and the front end protective film 34, is 3mm.

In the dashed line in FIG. 8, the diameter of the circular central lower electrode 17c is 0.625mm, and the diameter of the peripheral lower electrode 17o is 2mm. The inner diameter of the peripheral lower electrode 17o is 62.5$\mu$m away from the outer diameter of the central lower electrode 17c in the diameter direction. In the case of the dotted line, the outer diameter of the upper electrode 25c, which is opposite to the peripheral lower electrode 17o and the central lower electrode 17c, is also 2mm. The outer diameter of the vibrating part (25c, 26, 34), which is the third element, is 2mm. This vibrating part consists of the upper electrode 25c, the upper electrode protective film 26, and the tip protective film 34. Under the peripheral lower electrode 17o and the central lower electrode 17c, there is a layer of 20.2$\mu$m thick field insulating film made of $SiO_2$ thin film. An active region defined by $S_{AA}$=(gate length)$\times$(gate width) is defined inside a field insulating film with a thickness of 20.2$\mu$m, and a gate insulating film for an insulated gate semiconductor element with a thickness of 100nm can be formed inside the active region. In the simulation shown in FIG. 8, the upper electrode 25c, which forms part of the drum-shaped detection area, is grounded, and its sensitivity adjustment resistor $R_{aij}$=100M$\Omega$, which is the fifth factor determining the frequency characteristics.

In the simulation shown in FIG. 8, as described above, the height of the solid-line vibration cavity 28 is 4.4$\mu$m, and the height of the dot-dash-line vibration cavity 28 is 3.8$\mu$m. Both the solid line and the dot-dash line indicate the results of a simulation in which a DC voltage $V_{bias}$ of 50V was applied as the first potential between the upper electrode 25c and the surrounding lower electrode 17o. When each individual chip of the audible range sensors $X_{v1}$-$X_{v4}$ and $X_{w1}$- $X_{w5}$ is manufactured using the same manufacturing process, the height of the vibration cavity 28, which determines the sixth factor of frequency characteristics, is common. If the height of the vibration cavity 28 of the sixth factor is the same, it is desirable to greatly change the DC voltage $V_{bias}$ applied as the seventh factor in addition to the first to sixth factors. On the other hand, in the simulation shown in FIG. 8, multiple audible range sensors use a common DC voltage $V_{bias}$. Therefore, if the DC voltage $V_{bias}$ remains unchanged, the sixth element should be changed.

The sensitivity curve of the vibration components (25c, 26, 34) with an outer diameter of 3mm, as shown by the solid line in FIG. 8, has a receiving sensitivity of 3.0mV/Pa at approximately 65Hz. Below about 65Hz, the receiving sensitivity is higher than 3.0mV/Pa. The sensitivity curve shown by the solid line starts at a value of 3.0mV/Pa at around 65Hz and decreases with increasing frequency, reaching a minimum value of 2.2mV/Pa at around 110Hz. After reaching the minimum value, the value begins to increase, reaching a maximum of 2.7mV/Pa at approximately 320Hz, and then decreases again as the frequency increases, forming an S-shaped curve. The peak of the sensitivity curve in FIG. 8 reaches its maximum value at around 320Hz, indicating the mechanical resonance frequency, which is located near the center of the audible range.

For the main diagnostic frequency band of around 20Hz-710Hz shown in FIG. 12, it can be seen that a receiving sensitivity of 2.1mV/Pa or higher can be obtained in the frequency band of 65-700Hz included in the main diagnostic frequency band. As previously mentioned, the sensitivity characteristics shown in FIG. 8 are simulation results when the amplification function of the insulated gate semiconductor element included in the audible range sensor is ignored. Taking into account the amplification factor of the insulated gate semiconductor element, such as the mutual conductance $g_m$ of the MOSFET as shown in formula (9), the receiving sensitivity is expected to be higher than 2.1mV/Pa by more than one digit, so the hearing range sensor of the electronic stethoscope of the second embodiment can achieve extremely high sensitivity.

The dotted line in FIG. 8 shows the sensitivity characteristics of the audible range sensor $X_{ij}$ using a split gate structure, with the outer diameter of its vibrating components (25c, 26, 34) being 2mm. The receiving sensitivity gradually increases with the increase of frequency from about 50Hz, reaching a maximum value of 1.05mV/Pa at about 1kHz, and then decreases with the increase of frequency, forming an upward protruding bowl shaped curve shape. The peak of the sensitivity curve in FIG. 8 reaches its maximum at approximately 1kHz, indicating the mechanical resonance frequency, which indicates that the resonance frequency is within the audible range. When the outer diameter of the vibrating components (25c, 26, 34) is 2mm, a receiving sensitivity of 0.25-1.0mV/Pa can be obtained within the frequency band of 50-700Hz, which is the main diagnostic frequency band. Taking into account the amplification factor of the insulated gate semiconductor element such as the transconductance $g_m$ of the MOSFET as shown in equation (9), the receiving sensitivity is expected to be higher than 0.25-1.0mV/Pa by more than one digit. Therefore, even with a diameter of 2mm, the audible range sensor of the electronic stethoscope of the second embodiment can achieve extremely high sensitivity. As shown in FIG. 9A, the noise that may cause problems in electronic stethoscopes includes 1/f noise that is inversely proportional to frequency f and shot noise (white noise) that is independent of frequency f. As shown in FIG. 9A, the higher the frequency, the smaller the 1/f noise. Therefore, at an angular frequency fcnr≈200Hz, the noise level is equal to white noise. When the frequency is higher than the angular frequency fcnr, white noise becomes the main component, while when the frequency is lower, 1/f noise becomes the main component. In the electronic stethoscope of the second embodiment, the receiving sensitivity can be improved by adjusting the values of the sensitivity adjustment resistor $Ra_{ij}$ and the DC voltage $V_{bias}$, so that the curve showing the frequency-dependent characteristics of the receiving sensitivity is located in the upper right region of the noise characteristics shown in FIG. 9A, including the resonance frequency peak, thereby achieving a high SN ratio.

As shown in the upper part of FIG. 9A, the characteristic audible range signal I sound, as an example, is said to appear immediately after the onset of the systolic phase of the heart, mainly caused by the closure of the mitral valve, and is mainly in the frequency band of 30-120Hz. The I sound also contains components of tricuspid valve closure, usually split harmonics. It is said that the II sound is generated when the aortic and pulmonary valves close at the beginning of the diastolic phase of the heart, with its main frequency band between 70-150Hz. The II sound is said to have been discovered by the aforementioned Laennec. On the other hand, the I sound was discovered 200 years ago in 1616.

In the upper section of FIG. 9A, as an example of characteristic audible range signals, the expansion phase noise emitted from the aortic valve and the noise caused by mitral valve regurgitation in the frequency band of 180Hz-71 0Hz are shown, and the vibration of respiratory organs in the frequency band of 210Hz-630Hz is the target of electronic stethoscope diagnosis. Respiratory oscillation can be detected by measuring the loudness, left-right difference, and abnormalities in auscultatory sites of respiratory sounds. Abnormal auscultation site refers to the presence of bronchial sounds in the alveolar respiratory auscultation site. When the electronic stethoscope of the second embodiment is used to detect characteristic audible signals caused by mechanical equipment anomalies, structural cracks, etc., it is not limited to the characteristic audible signal frequency band shown in the upper part of FIG. 9A.

Non-patent Document 1 reports the results of measuring the sound pressure spectrum of heart sounds perceived by the ear using a traditional acoustic stethoscope (non-electronic stethoscope), which uses human heart sounds recorded by an electrocardiograph as a sound source, and obtains the results shown in FIG. 9B. According to FIG. 9B, the traditional bell-shaped stethoscope represented by the white rectangle shows a bell-shaped frequency spectrum distribution that bulges upward between 24Hz and 500Hz, with a maximum value of approximately 68Hz. On the other hand, the traditional diaphragm (diaphragm) stethoscope shown in the white triangle shows an upward convex bell-shaped frequency spectrum distribution from 40Hz-400Hz, with a maximum value of about 130Hz. Non-patent Document 1 states that "below 100Hz, the sound pressure of a diaphragm stethoscope is about 20-25dB lower than that of a bell stethoscope, and the diaphragm stethoscope acts as a high-pass filter.". According to the annotation in Non-Patent Document 1, it can be inferred that the heart sound used as the sound source is close to the spectral shape of a bell stethoscope, with a maximum value of approximately 68Hz in FIG. 9B.

FIG. 9B shows that in traditional acoustic stethoscopes, the shape, size, and internal structure of the stethoscope can affect sensitivity characteristics. In medical practice, when using a bell stethoscope to listen to heart sounds and heart murmurs, as shown in FIG. 3A, if the stethoscope is pressed firmly against the chest wall surface that forms part of the diagnostic object 1, the pressure level is higher, allowing for better hearing of high-frequency sounds. Conversely, as shown in FIG. 3B, if the pressure level is lower, it allows for better hearing of low-frequency sounds, making it suitable for screening. On the other hand, people often mistakenly believe that using a diaphragm stethoscope cannot hear low-frequency sounds such as rapid sounds, but in fact, some people have pointed out that diaphragm stethoscopes are better at hearing various sounds because they have better transmission from solid objects to solid objects.

As described above, the frequency characteristics of the stethoscope are generally determined by the structure of the biological body as an elastic body and the sound collecting part 9a. The mass of the sound collection unit 9a and the elastic constant of the living body or the like will generate a resonance frequency, and the sound above this frequency will decrease as the frequency increases. Even if the vibration produced by the organism has a constant amplitude (acceleration) relative to the frequency, the loudness of the sound heard by the stethoscope depends on the mass of the organism if it is lower than the resonant frequency; If it is higher than the resonant frequency, it depends on the amplitude of the vibration, regardless of the mass of the organism. The amplitude of vibration is inversely proportional to the square of the frequency. Therefore, even if the amplitude of vibration generated in the body is constant in relation to the frequency, it will decay inversely proportional to the square of the frequency above the resonant frequency.

As shown in FIG. 1A, when there is a sound tube 7 between the sound collection part 9a and the outer ear connection part 6, high-frequency sounds will be attenuated due to the propagation characteristics of the sound tube 7. The resonant frequency of a traditional ordinary stethoscope is at most a few hundred Hz, and when the frequency exceeds a few hundred Hz, the sensitivity will drop sharply. Therefore, for the I and II sound and other frequencies below approximately 300Hz shown in the upper part of FIG. 9A, traditional acoustic stethoscopes increase the sensitivity below the resonant frequency by making the stethoscope heavier, thereby improving the accuracy of auscultation. Similarly, in an acoustic stethoscope, the sensitivity to sounds from the respiratory tract, aortic valve diastolic murmurs, and murmurs caused by mitral valve regurgitation, as shown in the upper section of FIG. 9A, rapidly decreases after exceeding approximately 300Hz.

Therefore, in the prior art of using an acoustic stethoscope for auscultation, there is a problem that high-frequency sounds around 300Hz are masked by low-frequency sounds and cannot be heard. This problem also exists in previous electronic stethoscopes. This is because the receiving sensitivity of the traditional electronic stethoscope is more than one order of magnitude lower than that of the electronic stethoscope of the second embodiment, making it impossible to achieve a high signal-to-noise ratio (SN ratio). According to the electronic stethoscope of the second embodiment, as shown by the solid and dashed lines in FIG. 8, high receiving sensitivity can be achieved even above approximately 300Hz.

Therefore, even if the characteristic audible signal as the sound source is weak, the stethoscope can achieve a high SN ratio, thereby improving the accuracy of the stethoscope.

The solid line in FIG. 10A indicates that the diameter of the circular central lower electrode 17c is 0.75mm, the diameter of the peripheral lower electrode 17o is 2.4mm, and the inner diameter of the peripheral lower electrode 17o is 75μm away from the outer diameter of the central lower electrode 17c in the diameter direction. The outer diameter of the upper electrode 25c, which is opposite to the peripheral lower electrode 17o and the central lower electrode 17c, is also 2.4mm, and the outer diameter of the vibrating portion (25c, 26, 34) composed of the upper electrode 25c and other components, which is the third element, is 2.4mm. The dotted line in FIG. 10A shows that the diameter of the circular central lower electrode 17c is 0.375mm, and the diameter of the peripheral lower electrode 17o is 1.2mm. The inner diameter of the peripheral lower electrode 17o is 37.5μm away from the outer diameter of the central lower electrode 17c in the radial direction.

In the case of the one-dot chain line in FIG. 10A, the outer diameter of the upper electrode 25c facing the peripheral lower electrode 17o and the like is also 1.2mm, and the outer diameter of the vibration part (25c, 26, 34) composed of the upper electrode 25c and the like as the third element is 1.2mm. A field insulating film made of a $SiO_2$ thin film with a thickness of 9.2μm is provided below each of the peripheral lower electrode 17o and the central lower electrode 17c. An active region defined by $S_{AA}$ = (gate length) × (gate width) is delineated within a 9.2μm thick field insulating film, and a gate insulating film for an insulating gate semiconductor element with a thickness of 100nm can be formed inside the active region. The upper electrode 25c, which forms part of the drum-shaped detection area, is grounded, and the sensitivity adjustment resistor $R_{aij}$=100MΩ. The solid line in FIG. 10A shows the simulation result when a DC voltage $V_{bias}$ of 11V is applied between the upper electrode 25c and the peripheral lower electrode 17o as the first potential.

In the simulation shown in FIG. 10A, the cavity-forming insulating film 20 made of a $SiO_2$ thin film surrounds the vibration cavity 28. In both the solid line and the dashed-dotted line simulations, the height of the sixth factor vibration cavity 28 is uniformly set to 2.0μm. The white squares shown on the cross-sections of the audible range sensors $X_{v2}$ and $X_{v4}$ in FIG. 16 are schematic diagrams exaggerating the height, while each actual vibration cavity is a flat rectangle with a width of approximately 2.4mm and a height of approximately 2μm. Similarly, the white rectangle on the cross-section of the audible range sensor $X_{w1}$ shown in FIG. 16 is also a flat rectangle with a width of approximately 1.2mm and a height of approximately 2 microns, which is almost invisible. Moreover, an upper electrode protection film 26 made of a $Si_3N_4$ film with a thickness of 10.0μm is provided on the entire surface above the upper electrode 25c and above the insulating film 20 surrounding the cavity of the vibration cavity 28. Above the upper electrode protection film 26 is a tip protection film 34 made of room temperature curing silicone rubber with a thickness of 0.2mm. As shown in FIG. 8, the upper surface of the front protective film 34 is simulated under conditions of contact with water, which is the most common contact for most organisms.

As shown in FIG. 15, when arranging audible range sensors $X_{v1}$-$X_{v4}$ and $X_{w1}$-$X_{w5}$ with different resonant frequencies within the cabinet, if the individual chips of each audible range sensor $X_{v1}$-$X_{v4}$ and $X_{w1}$-$X_{w5}$ are manufactured in the same manufacturing process, the manufacturing cost can be reduced. When the audible range sensors $X_{v1}$-$X_{v4}$ and $X_{w1}$-$X_{w5}$ having different resonant frequencies are manufactured in the same manufacturing process, it is convenient to make the height of the sixth factor vibration cavity 28 the same. If the height of the vibration cavity 28 is the same, in addition to the first to sixth factors that determine the frequency characteristics, the DC voltage $V_{bias}$ of the seventh factor also needs to be significantly changed. The solid line in FIG. 10A represents the case where the outer diameter (diameter) of the vibrating components (25c, 26, 34) is 2.4mm and the DC voltage $V_{bias}$=11V. The dotted line represents the case where the outer diameter of the vibrating components (25c, 26, 34) is 1.2mm and the DC voltage $V_{bias}$ is 45V.

The sensitivity characteristic shown by the solid line in FIG. 10A reaches a receiving sensitivity of 1.0mV/Pa at approximately 95Hz; Below about 95Hz, the receiving sensitivity is higher than 1.0mV/Pa. The sensitivity curve shown by the solid line starts at a receiving sensitivity value of 1.0mV/Pa at about 95Hz and decreases with increasing frequency, reaching a minimum value of 0.78mV/Pa at about 140Hz. After reaching the minimum value, it began to increase again, reaching a maximum value of 0.88mV/Pa at around 350Hz, and then forming an S-shaped curve that decreased with increasing frequency. The peak of the sensitivity curve in FIG. 10A reaches its maximum value at around 350Hz, indicating the mechanical resonance frequency, which is located near the center of the audible range. Compared to the main diagnostic frequency band of around 20Hz-710Hz shown in FIG. 12, when the outer diameter (diameter) of the vibrating components (25c, 26, 34) is 2.4mm and the DC voltage $V_{bias}$=11V, a receiving sensitivity of 0.78mV/Pa or higher can be obtained within the main diagnostic frequency band of 95-700Hz. However, considering the amplification factor of the built-in insulated gate semiconductor component, the receiving sensitivity is expected to be more than one order of magnitude higher than 0.78mV/Pa.

The sensitivity characteristic of the audible range sensor $X_{ij}$ shown by the dotted line in FIG. 10A, exhibits a bowl-shaped upward convex curve shape. The receiving sensitivity gradually increases with increasing frequency from approximately 50Hz, reaching a maximum value of 0.35mV/Pa at approximately 5.3kHz, and then decreases again with increasing frequency. The peak of the sensitivity curve in FIG. 10A at approximately 5.3kHz shows the mechanical resonance frequency, indicating that the resonance frequency exists in the audible range, but is close to the high-frequency side of the audible region. When the outer diameter (diameter) of the vibrating components (25c, 26, 34) is 1.2 mm and the DC voltage $V_{bias}$ is 45V, a receiving sensitivity of 0.025-0.1mV/Pa can be obtained in the frequency band of 50-700Hz, which is included in the main examination frequency band. However, considering the amplification factor of the built-in insulated gate semiconductor component, the receiving sensitivity is expected to be more than one order of magnitude higher than 0.025-0.1mV/Pa. As shown by the solid line and the dashed line in FIG. 10A, according to the electronic stethoscope of the second embodiment, high receiving sensitivity can be achieved even above approximately 300Hz. Therefore, even if the characteristic audible range signal of the sound source is weak, the stethoscope can achieve a high SN ratio, thereby improving the accuracy of the stethoscope. The solid line in FIG. 10B is the same as the solid line in FIG. 10A, with a diameter of 0.75mm for the circular central lower electrode 17c and an outer diameter (diameter) of 2.4mm for the vibrating parts (25c, 26, 34). The dotted line in FIG. 10B is similar to FIG. 10A, with a diameter of 0.375mm for the circular central lower electrode 17c and an outer diameter (diameter) of 1.2mm for the vibrating parts (25c, 26, 34). The upper electrode 25c is grounded, and the sensitivity adjustment resistor $R_{aij}$=100MΩ, which is also the same as in FIG. 10A.

In addition, in the simulation shown in FIG. 10B, as in FIG. 10A, a 9.2$\mu$m thick field insulating film composed of a SiO$_2$ thin film is provided below the peripheral lower electrode 17o and the central lower electrode 17c, respectively. An active region is defined within a 9.2$\mu$m thick field insulating film, and a gate insulating film for an insulating gate semiconductor element with a thickness of 100nm or similar thickness can be formed within the active region. An insulating film 20 formed of a SiO$_2$ thin film is also arranged around the vibration cavity 28. In the solid and dashed lines of FIG. 10B, the height of the vibration cavity 28 (the sixth factor determining frequency characteristics) is uniformly set to 2.0$\mu$m. The upper electrode protection film 26 is made of a Si$_3$N$_4$ thin film with a thickness of 10.0$\mu$m, covering the upper electrode 25c and the insulating film 20 forming the cavity, surrounding the entire surface of the vibration cavity 28. Above the upper electrode protection film 26 is a tip protection film 34 made of room temperature curing silicone rubber with a thickness of 0.2mm. As in FIG. 10A, the simulation assumes that the upper surface of the front protective film 34 is in contact with water.

However, the solid line in FIG. 10B is the simulation result when a DC voltage $V_{bias}$ of 4V is applied between the upper electrode 25c and the peripheral lower electrode 17o as the first potential. The sensitivity curve shown by the solid line in FIG. 10B is different from the wave number dependence characteristic of the reception sensitivity shown by the solid line in FIG. 10A, and the peak of the sensitivity curve representing the maximum value is not significant. That is, the sensitivity characteristics of the vibration parts (25c, 26, 34) shown by the solid line in FIG. 10B have an outer diameter (diameter) of 2.4mm and a DC voltage $V_{bias}$ of 4V, reaching a receiving sensitivity of 1.0mV/Pa at around 130Hz, and a receiving sensitivity of 1.0mV/Pa or higher below around 130Hz. The sensitivity curve shown by the solid line decreases from a receiving sensitivity of 1.0mV/Pa around 130Hz, and reaches an inflection point value of approximately 0.6mV/Pa around 210Hz. After reaching the inflection point value, the shoulder portion smoothly decreases to around 430Hz, and then forms an S-shaped curve that decreases as the wave number increases. The shoulder that also appears on the high-frequency side of the graph in FIG. 10B, which is higher than 210Hz, corresponds to the mechanical resonance frequency, indicating that the mechanical resonance frequency exists near the approximate center of the audible range. Compared to the main inspection frequency band of about 20Hz-710Hz shown in FIG. 12, when the outer diameter (diameter) of the vibration part (25c, 26, 34) is 2.4mm and the DC voltage $V_{bias}$ is 4V, it is known that a receiving sensitivity of about 0.48mV/Pa or more can be obtained in the frequency band of 130-700Hz included in the main inspection frequency band. However, considering the amplification factor of the insulated gate semiconductor element, it is expected to achieve a reception sensitivity that is one digit or more higher than 0.48mV/Pa.

The dashed line in FIG. 10B shows the sensitivity characteristic of the audible range sensor $X_{ij}$ in the divided gate structure, where the outer diameter of the vibrating section (25c, 26, 34) is 1.2mm and the DC voltage $V_{bias}$ is 16V. Starting from around 50Hz, the receiving sensitivity gradually increases as the frequency increases, reaching a maximum of 0.29mV/Pa at around 3.6kHz, and then decreases again as the frequency increases, forming a bowl-shaped curve that curves upward. The peak of the sensitivity curve in FIG. 10B, which reaches a maximum around 3.6kHz, indicates the mechanical resonance frequency when the outer diameter is 1.2mm. It can be seen that the resonance frequency exists in the audible region, but when the outer diameter is 1.2mm, it is close to the end of the high-wave side of the audible region. In the case where the outer diameter of the vibrating components (25c, 26, 34) is 1.2mm and the DC voltage $V_{bias}$ is 16V, it is known that a receiving sensitivity of 0.04-0.11mV/Pa can be obtained in the frequency band of 50-700Hz included in the main inspection frequency band. However, if the amplification factor of the insulated gate semiconductor element is taken into account, it is expected to achieve a reception sensitivity that is one digit or more higher than 0.04 -0.11mV/Pa at an outer diameter of 1.2mm and a DC voltage $V_{bias}$ of 16V. According to the electronic stethoscope of the second embodiment, as shown by the solid line and the one-dot chain line in FIG.10B, high receiving sensitivity can be achieved even above 300Hz, so even in the case where the characteristic audible range signal as the sound source is weak, by achieving a high SN ratio, the accuracy of auscultation can be improved.

The resonant frequency of the audible range sensor $X_{ij}$ also varies with the time constant $C_1 \cdot R_{aij}$, which is determined by the product of the capacitance $C_1$ formed by the drum-shaped structure and the sensitivity adjustment

resistor $R_{aij}$ (fifth factor) that grounds one electrode of the capacitance $C_1$. If the time constant $C_1 \cdot R_{aij}$ is set to be the same as the mechanical resonance frequency determined by the first, second, third, and fourth factors, a broadband characteristic as an auditory sensor can be obtained. When the sixth coefficient height of the vibration cavity 28 changes, the capacitance $C_1$ also changes, so the time constant $C_1 \cdot R_{aij}$ also changes, resulting in a change in the resonant frequency. FIG. 11A shows the change of the sound-emitting sensor $X_{ij}$ with the split structure shown in FIG. 4 and other FIGS. when the value of the sensitivity adjustment resistor $R_{aij}$ changes. The change is based on the voltage change of the central lower electrode 17c as the receiving sensitivity. The diameter of the inner central lower electrode 17c is 0.625mm, and the diameter of the peripheral lower electrode 17o is 2.0mm. During the simulation, the distance d between the upper electrode 25c and the central lower electrode 17c is 1.5μm. The outer diameter of the upper electrode 25c, which faces the lower electrode 17o and other components, is also 2.0mm. The outer diameter of the vibrating section (25c, 26, 34) formed by the upper electrode 25c and other components is 2.0mm, serving as the third factor. That is, the cavity formed by the $SiO_2$ thin film makes the insulating film 20 surround the vibration cavity 28, and the height of the vibration cavity 28 (the sixth factor determining the frequency characteristics) is set to 1.5μm. On the other hand, there is a 6.9μm thick field insulating film made of $SiO_2$ thin film under the peripheral lower electrode 17o and the central lower electrode 17c. An active region is defined within a 6.9μm thick field insulating film, within which a gate insulating film for an insulating gate semiconductor element with a thickness of 100nm or similar thickness can be formed. The upper electrode protection film 26 is composed of a $Si_3N_4$ film with a thickness of 10.0μm, covering the entire surface above the upper electrode 25c and above the insulating film 20 surrounding the cavity of the vibration cavity 28. Above the upper electrode protection film 26 is a tip protection film 34 made of room temperature curing silicone rubber with a thickness of 0.2mm. As shown in FIGS.8, 10A, and 10B, the upper surface of the tip protective film 34 is simulated under conditions of contact with water, whose elastic constant is close to that of the living body of the diagnostic object 1. FIG. 11A shows that, as the value of the sensitivity adjustment resistor $R_{aij}$, which is the fifth coefficient, is sequentially decreased from $R_{aij} = 100M\Omega \rightarrow 50M\Omega \rightarrow 20M\Omega \rightarrow 10M\Omega \rightarrow 5M\Omega \rightarrow 2M\Omega$, the value of the peak on the extreme side (mechanical resonance frequency) of the curve representing the frequency dependence of the receiving sensitivity is gradually decreased. That is, the peak value on the extreme side of the curve representing frequency dependence decreases from approximately 0.65mV/Pa when the sensitivity adjustment resistance $R_{aij} = 100M\Omega$ to approximately 0.05mV/Pa when the sensitivity adjustment resistance $R_{aij} = 2M\Omega$. It can also be seen that when the sensitivity adjustment resistor $R_{aij}$ for the upper electrode 25c is gradually reduced from $R_{aij} = 100M\Omega \rightarrow 50M\Omega \rightarrow 20M\Omega \rightarrow 10M\Omega \rightarrow 5M\Omega \rightarrow 2M\Omega$, and the value of the sensitivity adjustment resistor $R_{aij}$ decreases sequentially, the center frequency of the resonance peak appearing on the extreme side of the frequency-dependent curve moves to the high-frequency side. That is, the center frequency value of the resonance peak appearing on the extreme side of the frequency-dependent curve decreases from about 600Hz when the sensitivity adjustment resistance $R_{aij} = 100M\Omega$ to about 4kHz when the sensitivity adjustment resistance $R_{aj} = 2M\Omega$. FIG. 11B shows the variation in voltage reception sensitivity with different values of sensitivity adjustment resistor $R_{aij}$ in the case of a drum-shaped structure of upper and lower electrodes similar to cMUT, where the voltage variation of a uniform lower electrode without a segmented gate structure is used as the voltage reception sensitivity. The diameter of the unified lower electrode is 2.0mm. The outer diameter of the upper electrode 25c, which is opposite to the lower electrode, is also 2.0mm. The vibrating part (25c, 26, 34) composed of the upper electrode 25c and other electrodes has an outer diameter of 2.0mm as a third factor, as shown in FIG. 11A. In addition, as in FIG. 11A, there is a field insulating film with a thickness of 6.9μm under the lower electrode, consisting of a SiO2 thin film. An active region is defined within a 6.9μm thick field insulating film, within which a gate insulating film or the like for an insulating gate type semiconductor element with a thickness of 100nm can be formed. In addition, a cavity formed by a $SiO_2$ thin film is arranged around the vibration cavity, and the height of the vibration cavity is set to 1.5μm. The upper electrode protection film consists of a $Si_3N_4$ thin film with a thickness of 10.0μm, covering the entire surface above the upper electrode and the upper part of the insulating film formed by the cavity around the vibration cavity. The upper electrode protective film is followed by a 0.2mm thick top protective film made of room temperature curing silicone rubber. As shown in FIG. 11A, the upper surface of the tip protective film 34 is simulated under conditions of contact with water, and the elastic constant of water is close to that of the diagnostic object 1 (living body).

In the case of the structure shown in FIG. 11B, where the lower electrode is not divided, the sensitivity adjustment resistor $R_{aij} = 20M\Omega \rightarrow 10M\Omega \rightarrow 5M\Omega \rightarrow 2M\Omega \rightarrow 1M\Omega \rightarrow 500k\Omega$, and as the same lower electrode is grounded, the value of the sensitivity adjustment resistor $R_{aij}$ decreases in stages, indicating that the peak value (mechanical resonance frequency) on the maximum side of the curve representing the voltage detection sensitivity dependence decreases compared to the peak value of the sensitivity curve when the sensitivity adjustment resistor $R_{aij} = 20M\Omega$. In the case of the audible range sensor $X_{ij}$ with a split gate structure in FIG. 11A, with a sensitivity adjustment resistor $R_{aij} = 20M\Omega$, the peak voltage reception sensitivity is approximately 0.33mV/Pa. In the case without the split-gate structure in FIG. 11B, with a sensitivity adjustment resistor $R_{aij} = 20M\Omega$, the peak voltage reception sensitivity is approximately 0.25mV/Pa, reduced to approximately 2/3. However, the data shown in FIGS. 11A and 11B do not take into account the amplification factor of the insulated gate semiconductor element shown in equation (9). Taking into account the amplification factor of the insulated gate semiconductor element integrated into the audible range sensor $X_{ij}$, the voltage reception sensitivity of the audible range sensor for the electronic stethoscope of the second embodiment is actually higher by at least one digit than that of the case in FIG. 11B. Therefore, when comparing the values of the resistors $R_{aij}$ adjusted with the same sensitivity, the audible range sensor $X_{ij}$ with an insulated gate semiconductor element in FIG. 11A has significantly higher voltage reception sensitivity than the case of the structure without an insulated gate semiconductor element shown in FIG. 11B.

In the structure where the lower electrode is integrated as shown in FIG. 11B, as the sensitivity adjustment resistor $R_{aij}=20M\Omega \rightarrow 10M\Omega \rightarrow 5M\Omega \rightarrow 2M\Omega \rightarrow 1M\Omega \rightarrow 500k\Omega$, the value of the sensitivity adjustment resistor $R_{aij}$ decreases in stages. It can also be seen that the center frequency of the resonance peak appearing on the maximum side of the frequency dependence curve moves from a value of about 440Hz when the sensitivity adjustment resistor $R_{aij}=20M\Omega$ to a high frequency side of about 3.0kHz. In the case of the split gate structure of FIG. 11A, when the sensitivity adjustment resistor $R_{aij}=20M\Omega$, the center frequency of the resonance peak appearing on the maximum side of the frequency dependence curve is 1.03kHz. In the case of the drum-shaped element without the split gate structure of FIG. 11B, when the sensitivity adjustment resistor $R_{aij}=20M\Omega$, the center frequency of the resonance peak decreases to 440Hz. In the case of the split gate structure in FIG. 11A, when the upper electrode 25c is grounded using a sensitivity adjustment resistor $R_{aij}=2M\Omega$, the center frequency of the resonance peak appearing on the extreme side of the frequency-dependent curve is approximately 4.0kHz. In contrast, in the drum-shaped element without a split gate structure shown in FIG. 11B, when the sensitivity adjustment resistor $R_{aij}=2M\Omega$, the center frequency of the resonance peak drops to 1.05kHz. Therefore, it can be seen that, in the case of the same value of the sensitivity adjustment resistor $R_{aij}$, the resonant peak center frequency of the audible range sensor $X_{ij}$ with a split structure applied to the electronic stethoscope of the second embodiment shown in FIG. 11A tends to be higher than that of the drum-shaped element without a split gate structure shown in FIG. 11B. Compared to the center frequency of the resonance peak of the audible range sensor $X_{ij}$ shown in FIG. 11A, the center frequency of all the resonance peaks of the curves without the split gate structure shown in FIG. 11B tends to move towards the low frequency side.

FIG. 12 shows the frequency bands of characteristic sound signals generated by the electronic stethoscope of the second embodiment for various physiological parameters. In the description of FIG. 9A, "I and II sound", "breathing", and "aortic and mitral valve regurgitation" have been mentioned. The second characteristic audible range signal S3 in the lower part of the frequency range on the left side of FIG. 12 is the sound of blood flowing rapidly from the atrium to the ventricle during early diastole, while the characteristic audible range signal S4 is the sound of atrial contraction during late diastole. As shown in FIG. 12, S3 and S4 are heart sounds that can be heard in the frequency range of approximately 20Hz-60Hz. Due to the low frequency, it is difficult to hear with an acoustic stethoscope.

Mitral stenosis is a condition in which the narrowing of the mitral valve obstructs blood flow from the left atrium to the left ventricle. The "mitral stenosis" shown in the third frequency band of 23-75Hz on the lower left side of FIG. 12 refers to the characteristic audible range signals of large I sounds, early diastolic valve opening sounds, and low pitched gradually decreasing diastolic vibrato. The 'expansion period vibrato' is a bass sound that follows the opening sound. The "digestive tract" shown in the frequency band of 56-420Hz in the upper part of FIG. 12 refers to the characteristic audible range signal of intestinal peristalsis sound during auscultation. The "driving noise" shown in the frequency band of 120-420Hz on the right side of FIG. 12, from top to bottom, is a characteristic audible range signal generated by forward turbulence through the valves or outflow pathways of the heart that cause narrowing or arrhythmia. The opposite of 'driving out' is' reverse flow '.

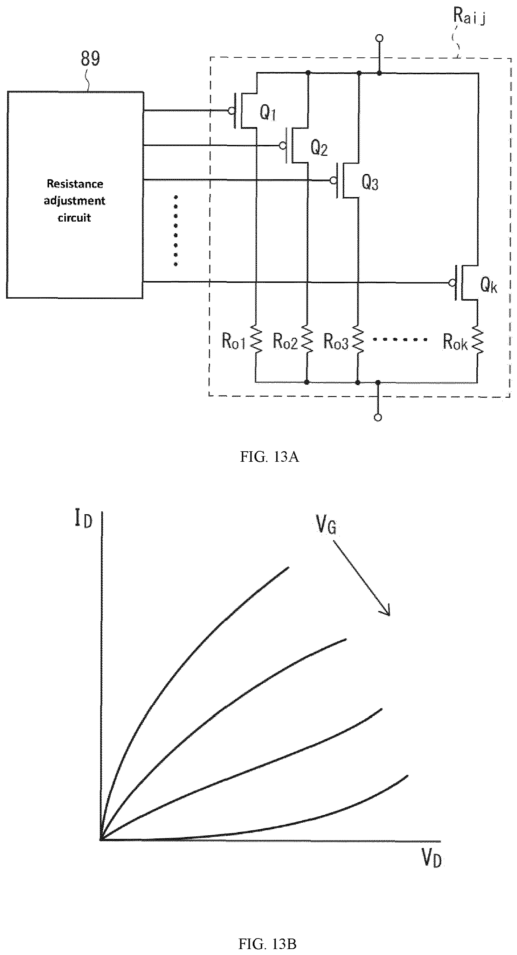

In FIG. 13A, the first adjusting resistor $R_{o1}=100M\Omega$ is connected in series with the first switching element $Q_1$ to form a first series circuit, and the current flowing through the first adjusting resistor $R_{o1}$ is controlled by the first switching element $Q_1$. The second adjusting resistor $R_{o2}=50M\Omega$ is connected in series with the second switching element $Q_2$ to form a second series circuit connected in parallel with the first series circuit. The current flowing through the second adjusting resistor $R_{o2}$ is controlled by the second switching element $Q_2$. Furthermore, the third adjusting resistor $R_{o3}=20M\Omega$ is connected in series with the third switching element $Q_3$, forming a third series circuit connected in parallel with the first and second series circuits. The current flowing through the third adjusting resistor $R_{o3}$ is controlled by the third switching element $Q_3$. Moreover, the k-th adjusting resistor $R_{ok}=2M\Omega$ is connected in series with the k-th switching element $Q_k$, forming a k-th series circuit connected in parallel with the 1st to (k-1) th series circuits. The current flowing through the k-th adjusting resistor $R_{ok}$ is controlled by the k-th switching element $Q_k$.

From the curve of the receiving sensitivity as a function of frequency for a set of receiving sensitivities with the sensitivity adjustment resistor $R_{aij}$ shown in FIG. 11A as a parameter, it can be seen that by changing the sensitivity adjustment resistor $R_{aij}$, when selecting a specific frequency band among the various consultation frequency bands shown in FIG. 12, it can be seen that the sensitivity characteristics of the electronic stethoscope of the second embodiment can be adjusted to obtain the optimal signal-to-noise ratio for that specific frequency band. For example, as shown in FIG. 13A, the first adjustable resistor $R_{o1}$, the second adjustable resistor $R_{o2}$, the third adjustable resistor $R_{o3}$, ... the kth adjustable resistor $R_{ok}$, through the gate signal from the resistance adjustment circuit 89, correspond to the first switching element $Q_1$, the second switching element $Q_2$, the third switching element $Q_3$, ..., the kth switching element $Q_k$, If the switch drive is selectively performed and the desired adjustment resistance $R_{oj}$ is selected, the value of the sensitivity adjustment resistance $R_{aij}$ can be selected to obtain the best SN ratio for the frequency bands of the various physiological parameters shown in FIG. 12. The resistance adjustment circuit 89 can be controlled by the operation of the display/operation unit 5 set in the sound collecting unit 9a shown in FIG. 1A.

In addition, as shown in FIG. 13B, the gate voltage $V_G$ of a 3-terminal semiconductor element with variable resistance characteristics of drain current $I_D$-drain voltage $V_D$ can be changed by a gate signal from the resistance adjustment circuit 89. In this way, for various frequency bands of physiological parameters shown in FIG. 12, the optimal signal-to-noise ratio can be selected by adjusting the value of the sensitivity adjustment resistor $R_{aij}$, so that the $I_D$-$V_D$ characteristics shown in FIG. 13B are between the $I_D$-$V_D$ characteristics of a triode-type SIT and a pentode-type FET. This can be achieved by selecting the gate spacing and channel impurity density of a junction-type SIT. Therefore, according to the electronic stethoscope of the second embodiment, by operating the display and operation unit 5 of FIG. 1A, it is possible to obtain the optimal SN ratio for various inspection bands shown in FIG. 12, and even in an environment where the characteristic audible frequency signal as a sound source is weak, it is possible to obtain a high SN ratio and perform diagnosis with high objectivity and auditory accuracy.

As shown in FIG. 16 below, the shape of the housing 92b of the electronic stethoscope of the second embodiment is cylindrical, so the curved surface as the cylindrical side surface, the flat surface as the cylindrical upper surface (top), and the flat surface as the cylindrical lower surface (bottom) are the main parts of the outer skin. In the housing 92b shown in FIG. 16, the flat plate-shaped portions are the upper and lower surfaces of the cylinder. However, in the sound collection section 9b of the electronic stethoscope of the second embodiment, the "signal input surface" is defined on the lower surface portion where the characteristic audible range signal is input. That is, in FIG. 16, the area where the bottom surface of the plate-shaped portion that constitutes a part of the outer skin of the housing 92b is in direct contact with the surface of the diagnostic object 1 such as a living body is defined as a "signal input surface". In the sound collection section 9b of the electronic stethoscope of the second embodiment, by bringing the signal input surface defined on the bottom surface of the housing 92b into contact with the surface of the diagnostic object 1, characteristic sound signals such as biological sound signals can be detected.

As can be seen from FIG. 16 below, the housing 92b of the electronic stethoscope of the second embodiment does not have a buffer film (housing vibration film) 93 as shown in FIG. 3A and other figures, and therefore does not have an air layer. That is, in the electronic stethoscope of the second embodiment, since the lower part of the housing 92b is directly in contact with the surface of the diagnostic object 1 such as a living body, soft plastic such as PVC can be used as the material of the housing 92b. However, considering the influence of acoustic impedance on the audible range characteristic signal propagating through the bottom surface of the housing 92b, it is preferable to use an elastic material such as room temperature curing silicone rubber, which is used as the material for the tip protection film 34 in simulations such as those shown in FIGS. 8, 10A, 10B, 11A, and 11B.

FIG. 14 shows the frequency dependence of the receiving sensitivity detected as the voltage change of the central lower electrode 17c in the audible range sensor $X_{ij}$ with a shunt gate structure, using the thickness of the tip protection film 34 on the upper portion of the upper electrode protection film 26 shown in FIG. 5 and elsewhere as a parameter. As shown in FIGS. 8, 10A, 10B, 11A, and 11B, the upper surface of the tip protective film 34 is simulated under conditions of contact with water, whose elastic constant is close to that of the diagnostic object 1 (living body). Assuming that the needle tip protective film 34 is made of room temperature curing silicone rubber, when the thickness t of the room temperature curing silicone rubber is gradually increased from t=0.2mm→0.5mm→1.0mm→2.0mm→5mm→2mm, the frequency dependence of the receiving sensitivity for each thickness is shown. The sensitivity adjustment resistor has a constant value of $R_{aij}$=20MΩ, which can approximate the pattern of the upper electrode 25c to a drum-shaped detection area.

In the simulation shown in FIG. 14, the diameter of the inner central lower electrode 17c is 0.625mm, the diameter of the outer peripheral lower electrode 17o is 2.0mm, and the electrode spacing d between the upper electrode 25c and the central lower electrode 17c is 1.5μm. The outer diameter of the upper electrode 25c, which is opposite to the peripheral lower electrode 17o and other components, is also 2.0mm. The outer diameter of the vibrating part (25c, 26, 34) composed of the upper electrode 25c and other components as the third element is 2.0mm. That is, the insulating film 20 formed of a $SiO_2$ thin film that forms a cavity surrounds the vibration cavity 28 that determines the drum-shaped detection area, and the height of the vibration cavity 28 (as a sixth element) is 1.5μm. On the other hand, a 6.9μm thick field insulating film, composed of $SiO_2$ thin film, is situated beneath each of the peripheral lower electrode 17o and the central lower electrode 17c. The active region is defined within a 6.9μm thick field insulating film, within which a gate insulating film for an insulating gate semiconductor element with a thickness of 100nm or similar thickness can be formed. The upper electrode protection film 26 is composed of a $Si_3N_4$ film with a thickness of 10.0μm, covering the upper electrode 25c and the insulating film 20 forming the cavity, which surrounds the vibration cavity 28 on the entire surface.

According to the sensitivity curve in FIG. 14, as the thickness t of the tip protective film 34 of the audible range sensor $X_{ij}$ with a built-in amplification element increases sequentially (stepwise), from t=0.2mm→0.5mm→1.0mm→2.0mm, the position of the curve showing the frequency dependence of the receiving sensitivity is compared with the position of the sensitivity curve with a thickness t=0.2mm. It can be seen that the sensitivity decreases as it moves towards the lower side of the paper. The sensitivity curve shown by the solid line in FIG. 14 with a thickness t=0.2mm has a receiving sensitivity of 0.23mV/Pa at approximately 50Hz. The solid line represents the case of a thickness t=2mm, and its sensitivity curve decreases with increasing frequency starting from a receiving sensitivity value of 0.23mV/Pa at approximately 50Hz, reaching a minimum value of 0.21mV/Pa at approximately 93Hz. After reaching the minimum value, the sensitivity curve turns upward, reaching a maximum of 0.275mV/Pa at about 440Hz, and then decreases again as the frequency increases, forming an S-shaped sensitivity curve. The peak of the maximum value of the entity sensitivity curve at about 440Hz in FIG. 14 indicates the mechanical resonance frequency. Therefore, the sensitivity curve at t=0.2mm indicates that the mechanical resonance frequency is located near the center of the audible range.

On the other hand, the sensitivity curve of the audible range sensor $X_{ij}$ with a built-in amplification element of thickness t=0.5mm shown in the dashed line in FIG. 14 has a receiving sensitivity of 0.14mV/Pa at approximately 50Hz, and then begins to decrease with increasing frequency, reaching a minimum value of 0.065mV/Pa at approximately 130Hz. After reaching the minimum value, the sensitivity curve gradually rises, reaching a maximum of 0.075mV/Pa at approximately 1kHz, and then decreases again as the frequency increases, resembling an S-shaped curve with both ends extending left and right. The peak of the dashed curve in FIG. 14 shows the maximum value at approximately 1kHz, indicating the mechanical resonance frequency. The maximum value of the sensitivity curve at a thickness t=0.5mm, as shown by the dashed line in FIG. 14, is approximately 1/4 of the maximum value of the sensitivity curve at a thickness t=0.2mm, as shown by the solid line, and the maximum value rapidly decreases between 0.2mm and 0.5mm. The shape of the sensitivity curve near 1kHz when the thickness t=0.5mm, shown by the dashed line in FIG. 14, is more like a gentle hill near the shoulder than a maximum value.

The sensitivity curve of the audible range sensor $X_{ij}$ with a thickness of t=1.0mm, shown by a single dashed line in FIG. 14, is located below the sensitivity curve of t=0.5mm. After reaching a receiving sensitivity of 0.125mV/Pa at approximately 50Hz, the sensitivity begins to decrease as the frequency increases, reaching a minimum value of 0.03mV/Pa at approximately 160Hz. After reaching the minimum value, it slowly rises, almost in parallel, reaching a maximum of 0.045mV/Pa at approximately 1kHz, and then decreases again as the frequency increases, forming a curve similar to an S-shape. The single-point peak in FIG. 14 reaches its maximum value at approximately 1kHz, corresponding to the mechanical resonance frequency. The maximum value of the sensitivity curve at a thickness of t=1.0mm (as shown by the single-dot dashed line) is approximately 3/5 of the maximum value of the sensitivity curve at a thickness of t=0.5mm (as shown by the dashed line), although it is not as large as the change between 0.2mm and 0.5mm, but there is still a significant decrease. In the case of a thickness t=1.0mm, the shape of the sensitivity curve near 1kHz is shown by a dashed line in FIG. 14, which is more like a gentle hill near the shoulder than a maximum value.

Moreover, the sensitivity curve of the audible range sensor $X_{ij}$ with a thickness of t=2.0mm, indicated by a double-dot dashed line in FIG. 14, is located below the sensitivity curve with a thickness of t=1.0mm. After reaching a receiving sensitivity of 0.11mV/Pa around 50Hz, it begins to decrease along with the wave number, reaching a minimum value of 0.03mV/Pa around 160Hz. The minimum value around 160Hz is approximately the same as the minimum value of thickness t=1.0mm shown by the single-dot dashed line. After reaching a minimum value, the sensitivity curve for a thickness of t=2.0mm begins to increase gently, roughly approaching a parallel line. At around 800Hz, it reaches a maximum value of 0.035mV/Pa, and then again becomes a shape close to an S-shaped curve that decreases with the wave number. The peak of the sensitivity curve in the case of a thickness t=1.0mm shown by the one-dot chain line that becomes a maximum around 800Hz in FIG. 14 corresponds to the mechanical resonance frequency. The maximum value of the sensitivity curve at a thickness t=2.0mm, indicated by the double-dot-dash line, is slightly smaller than the sensitivity curve at a thickness t=1.0mm, indicated by the single-dot-dash line, and is a negligible reduction compared to the change between 0.2mm and 0.5mm. The shape of the sensitivity curve near 800Hz when the thickness t=2.0mm is indicated by the double-dot dashed line in FIG. 14 is more like a gentle hill near the shoulder than a maximum value.

FIG. 14 shows that when the thickness t increases from t=0.2mm to 0.5mm, the center frequency of the resonance peak appearing in the frequency dependence of the audible range sensor $X_{ij}$ with built-in amplification components tends to shift towards the high-frequency side. That is to say, when the thickness t=0.2mm, the center frequency value of the resonance peak appearing in the frequency correlation is about 440Hz, while when the thickness t=0.5mm, the center frequency value of the resonance peak is about 1kHz. However, when the thickness t increases step by step from 0.5mm→1.0mm→2.0mm, the shift of the center frequency of the resonance peak in the frequency dependence relationship is not significant, but tends to move towards lower frequencies. That is, the center frequency of the resonance peak appearing in the frequency dependence decreases from about 1kHz at a thickness t=0.5mm to about 800Hz at a thickness t=2mm.

On the other hand, when the thickness t increases from t=0.2mm to 0.5mm, the position of the minimum value that appears in the frequency dependence relationship shifts to the high-frequency side. In other words, in FIG. 14, the minimum value position appearing in the frequency correlation changes from about 93Hz when the thickness t=0.2mm to about 130Hz when the thickness t=0.5mm. However, when the thickness t is increased step by step from 0.5mm→ 1.0mm→2.0mm, the shift of the minimum frequency position in the frequency correlation is less obvious. That is, the frequency position of the minimum value appearing in the frequency dependence changes from about 130Hz when the thickness t=0.5mm to about 160Hz, which is common for thicknesses t=1mm and 2mm, so the frequency position of the minimum value seems to have stopped moving.

As shown in FIGS. 15 and 16, the sound collecting unit 9b of the electronic stethoscope according to the second embodiment includes audible range sensors $X_{v1}$, $X_{v2}$, $X_{v3}$, $X_{v4}$, $X_{w1}$, $X_{w2}$, $X_{w3}$, $X_{wj}$, $X_{w4}$, and $X_{w5}$ as individual components that can be housed inside the housing 92b. As described in the first embodiment, the flat pattern of the vibration cavity 28 indicated by the dashed line in FIG. 15 defines the flat pattern of the drum-shaped detection unit. The respective outer shapes of the audible range sensors $X_{ij}$ are formed into a shape that is approximately similar to the flat pattern of the vibration cavity 28. The dashed lines on the outside and inside of FIG. 15 represent concentric hexagons, and the nine taiko-shaped detection sections form a flat pattern of hexagons. As shown in FIGS. 8, 10A, 10B, and 11A, the audible range sensors $X_{ij}$ each have a mechanical resonance frequency in the audible range, and therefore have high receiving sensitivity.

Unlike the structure shown in FIG. 3A and the like, the outer diameter of the housing 92b is smaller than that of the sound collection component 9a of the electronic stethoscope of the first embodiment shown in FIG. 2, because there is no need for a buffer film fixing component to fix the peripheral portion of the buffer film on the lower outer side of the housing 92b. The audible range sensor $X_{ij}$ may be configured as a semiconductor element having an amplification function, and amplifies the characteristic audible range signal by being directly input to the gate, similar to the sound collector 9a of the electronic stethoscope in the first embodiment. The sound collector 9b can be placed in a position in contact or close proximity to the biological sample to be examined, such as the patient's chest, abdomen, limbs (such as arms or legs), or any other body part of the patient.

The receiving sensitivity of the audible range sensors $X_{v1}$-$X_{v4}$ and $X_{w1}$-$X_{w5}$ in the sound collection part 9b of the electronic stethoscope according to the second embodiment depends on the inner diameter, diagonal diameter, and other dimensions occupied by the audible range sensors, as explained in the electronic stethoscope according to the first embodiment. As shown in FIG. 15, in the sound collector 9b of the electronic stethoscope according to the second embodiment, the first audible range sensor $X_{w1}$ includes a hexagonal drum-shaped detection portion having a first diagonal diameter, which is placed at the center of the element array portion of the sound collector 9b. The second audible range sensor $X_{v1}$, the third audible range sensor $X_{v2}$, the fourth audible range sensor $X_{v3}$, and the fifth audible range sensor $X_{v4}$ each have a second diagonal diameter longer than the first diagonal diameter, and are arranged around the first audible range sensor $X_{w1}$. The planar patterns of the second audible range sensor $X_{v1}$, the third audible range sensor $X_{v2}$, the fourth audible range sensor $X_{v3}$, and the fifth audible range sensor $X_{v4}$ are also hexagonal drum-shaped detection area patterns.

Furthermore, a sixth audible range sensor $X_{w2}$ having a first diagonal path shorter than the second diagonal path is disposed between the fifth audible range sensor $X_{v4}$ and the second audible range sensor $X_{v1}$. Similarly, the seventh audible range sensor $X_{w3}$ having the first diagonal diameter is provided between the second audible range sensor $X_{v1}$ and the third audible range sensor $X_{v2}$, and the eighth audible range sensor $X_{w4}$ having the first diagonal diameter is arranged between the third audible range sensor $X_{v2}$ and the fourth audible range sensor $X_{v3}$. Moreover, a ninth audible range sensor $X_{w4}$ having a first diagonal diameter is also disposed between the fourth audible range sensor $X_{v3}$ and the fifth audible range sensor $X_{v4}$. The flat patterns of the sixth audible range sensor $X_{w2}$, the seventh audible range sensor $X_{w3}$, the eighth audible range sensor $X_{w4}$, and the ninth audible range sensor $X_{w5}$ are also hexagonal drum-shaped detection unit patterns.

As shown in FIG. 15, the housing 92b of the electronic stethoscope of the second embodiment includes a detection array consisting of multiple drum-shaped detection sections with different diagonal diameters. The first audible range sensor $X_{w1}$ is a drum-shaped detection component located in the middle of the detection component array, and its diagonal diameter is smaller than that of the second to fifth audible range sensors $X_{v1}$-$X_{v4}$. These sensors are arranged around the first audible range sensor $X_{w1}$ and each have a drum-shaped detection component. According to the planar layout of the detection array, including the audible range sensors $X_{v1}$-$X_{v4}$ of the drum-shaped detection portion with a large diagonal diameter and the audible range sensors $X_{w1}$-$X_{w5}$ of the drum-shaped detection portion with a small diagonal diameter, as shown by the dashed line in FIG. 15, the mechanical resonance of the audible range sensors $X_{v1}$-$X_{v4}$ and the audible range sensors $X_{w1}$-$X_{w5}$ is shown in FIG. 8 and other figures. The frequencies are different from each other. That is, by intentionally shifting the mechanical resonance frequencies of each of the audible range sensors $X_{v1}$-$X_{v4}$ and the audible range sensors $X_{w1}$-$X_{w5}$ in the array composed of multiple drum-shaped detection sections, the frequency band of the electronic stethoscope according to the second embodiment can be expanded, thereby improving overall reception sensitivity.

FIG. 16 shows a cross-sectional view from the XVI-XVI direction of FIG. 15, showing the structure of the first audible range sensor $X_{w1}$, with the fifth audible range sensor $X_{v4}$ located on the left side and the third audible range sensor $X_{v2}$ located on the right side. FIG. 5 and elsewhere show detailed cross-sectional structure examples of the fifth audible range sensor $X_{v4}$ and the third audible range sensor $X_{v2}$. As shown in FIG. 16, the sound collection unit 9b of the electronic stethoscope of the second embodiment has a disc-shaped housing 92b with a storage cavity inside. A groove is provided on the bottom surface inside the housing 92b as a sensor fixing part. The area where the bottom surface of the plate-shaped portion forming part of the outer skin of the housing 92b opposes the sensor fixing portion is the "signal input surface" of the electronic stethoscope of the second embodiment. The first audible range sensor $X_{w1}$, the third audible

range sensor $X_{v2}$, and the fifth audible range sensor $X_{v4}$ are fixed on the sensor fixing member, and their respective lower parts are installed in the sensor fixing member. In addition, a power circuit 96, a signal processing circuit 97, and a communication circuit 98 are also installed in the storage chamber formed by the housing 92b. The power circuit 96, the signal processing circuit 97, and the communication circuit 98 may be configured as a hybrid integrated circuit using a printed circuit board or the like, or at least a portion of the power circuit 96, the signal processing circuit 97, and the communication circuit 98 may be three-dimensionally mounted using a bump connection or the like. As shown in FIG. 16, an antenna 99 is connected to the input/output terminal of the communication circuit 98. The antenna 99 can be a dipole antenna, a helical antenna, or a planar antenna (patch antenna). The power circuit 96 provides the required power for the nine audible range sensors $X_{v1}$-$X_{v4}$ and $X_{w1}$-$X_{w5}$, the signal processing circuit 97, and the communication circuit 98. The signal processing circuit 97 receives signals from the audible range sensors $X_{v1}$- $X_{v4}$ and $X_{w1}$-$X_{w5}$ of different sizes, converts the received signals into A/D, and performs signal processing such as smoothing frequency characteristics and noise reduction using digital technology.

The communication circuit 98 transmits the information processed by the signal processing circuit 97 to each information system 3 in real time, in the same manner as shown in FIG. 1A. The communication between the sound collector 9b and the information system 3 can use electromagnetic waves in the 2.4GHz frequency band, with an antenna power not exceeding 10 mW. Through wireless or other communication methods, the information processed by the signal processing circuit 97 is transmitted in real time to the information system 3 for visualization, and further transmitted to institutional databases or cloud-based medical systems, such as databases related to clinics, hospitals, or hospital networks. It can be stored. The information system 3 of the institution interested in various biological information such as heartbeat, breathing, blood flow, digestion, etc. of the patient can access the information signal processed by the signal processing circuit 97 through the communication circuit 98 built into the sound collection unit 9b by wireless communication or other means. When the information system 3 is equipped with AI, the information obtained by the electronic stethoscope of the second embodiment can be machine-learned by AI, thereby achieving highly objective auscultation.

By installing the nine audible range sensors $X_{v1}$-$X_{v4}$ and $X_{w1}$-$X_{w5}$ shown in FIG. 15 on the sensor fixing part to detect and amplify characteristic audible range signals (such as biological audible range signals), even if the diameter of the housing 92b is relatively short, the characteristic audible range signals can be detected with sufficient signal strength. By reducing the number of audible range sensors $X_{v1}$-$X_{v4}$ and $X_{w1}$-$X_{w5}$ from the nine audible range sensors shown in FIG. 15, the outer diameter of the sound collection portion 9b can be reduced to an unconventional size to achieve good audibility. The material of the housing 92b is not limited, and may be hard resin or metal, such as Al or Ti. In order to improve the adhesion with the surface of the diagnostic object 1 and match the elastic impedance, it is recommended to use an elastic material such as silicone rubber for the housing 92b.

According to the sound collector 9b of the electronic stethoscope of the second embodiment, as shown in FIG. 16, the signal input surface defined at the lower part of the housing 92b contacts the surface of the diagnostic object 1, so that the audible range signals (bio-audible range signals) of biological sounds and other features can be directly transmitted to the audible range sensors $X_{v1}$-$X_{v4}$ and $X_{w1}$-$X_{w5}$ inside the sound collector 9b. In the sound collection section 9b of the electronic stethoscope according to the second embodiment, an insulated gate semiconductor element is integrated (incorporated) as an internal structure of an audible range sensor of a built-in amplification element. The capacitance change caused by the characteristic audible range signal directly input from the signal input surface of the housing 92b is caused by the charge on the surface of the channel formation region 14 of the insulated gate semiconductor element and the accompanying change in electromotive force of the charge. In other words, the charges induced on the surface of the channel formation region 14 control the height of the surface potential of the channel formation region 14 and achieve the amplification function shown in formula (9), thereby achieving extremely high receiving sensitivity.

The electronic stethoscope of the second embodiment does not require the acoustic tube 7 or the external ear connection part 6 as shown in FIG. 1A. For example, as shown in FIG. 17, a disc-shaped sound collection component 9b is separately in contact with the surface of the diagnostic object 1, and receives instructions from the information system 3 of the clinic or hospital through wireless means. The information acquired by the sound collection component 9b, such as fetal heart rate, maternal heart rate, uterine activity, and intrauterine pressure (IUP), is transmitted to the information system 3 of the clinic or hospital. This information can be transmitted wirelessly in real time.

In addition, in the sound collection part 9b of the electronic stethoscope of the second embodiment, the display/operation part 5 shown in FIG. 1A can also be omitted, and the display/operation part 5 can be reduced to a simple function and integrated inside the sound collection part 9b. That is to say, since the external information system 3 can issue commands through the communication circuit 98 to switch the power circuit, adjust the sensitivity adjustment resistor $R_{aij}$, adjust the gain of the amplifier, control the communication circuit, adjust the volume, and switch modes, the display/operation section 5 is not essential. The sound collection section 9b of the electronic stethoscope of the second embodiment can also display the signal-processed information on the external information system 3 through the communication circuit 98. If the display/operation section 5 is simplified to a simple function and integrated within the sound collection section 9b, for example, only the power circuit can be turned on/off and only the LED indicator light indicating that the device is in operation can be executed in the display/operation section 5.

According to the electronic stethoscope of the second embodiment, the receiving sensitivity is not reduced because it does not involve the process of converting acoustic waves (once radiated from the diagnostic object 1 into the characteristic audible range signal in the air) into electrical signals, as in the electronic stethoscope according to the first embodiment. That is, the characteristic audible signal generated in the diagnostic object 1 is received as an acoustic wave in a viscoelastic body with an acoustic impedance close to that of the diagnostic object 1 without sacrificing the receiving sensitivity, and the received acoustic wave is converted into an electrical signal, thereby improving the receiving sensitivity. In addition, in the electronic stethoscope of the second embodiment, multiple audible range sensors $X_{ij}$ are used, each of which has a mechanical resonance frequency within the audible range. By allowing multiple audible range sensors $X_{ij}$ to function without an air layer, characteristic audible range signals can be effectively detected as voltage signals.

In particular, when each of the multiple audible range sensors $X_{ij}$ integrates an insulated gate semiconductor as an internal structure, the detected characteristic stethoscope signal can be further amplified by the electrostatic induction effect of the internal structure. Therefore, according to the electronic stethoscope of the second embodiment, even in an environment where the characteristic audible signal as a sound source is weak compared to the noise level, a high SN ratio can be achieved, thereby realizing an electronic stethoscope with high objectivity and auditory accuracy.

**Third embodiment**

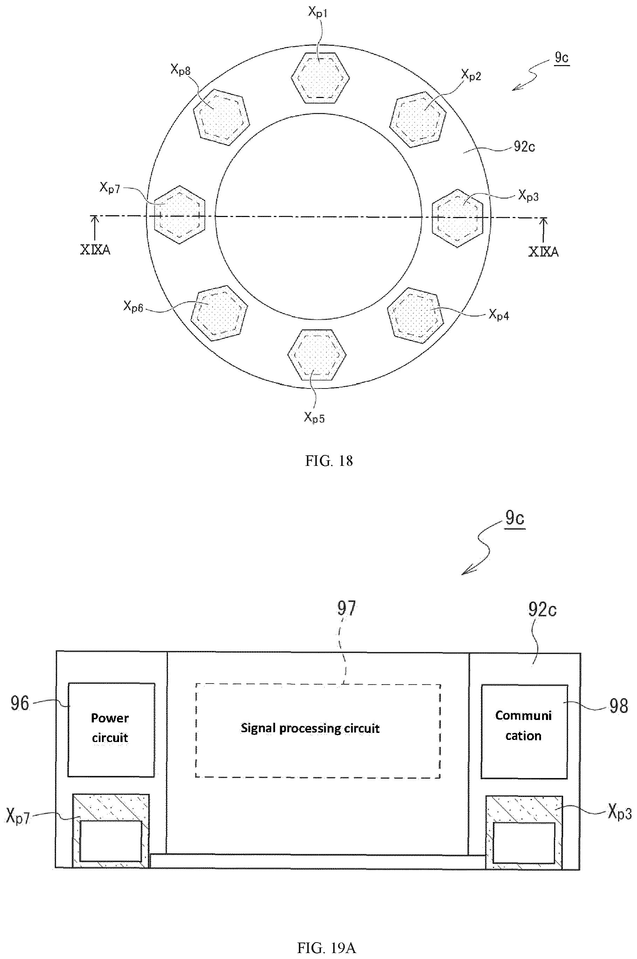

Like the electronic stethoscopes of the first and second embodiments, the electronic stethoscope of the third embodiment of the present disclosure also uses an audible range sensor $X_{ij}$ with a mechanical resonance frequency within the audible range. Like the electronic stethoscope of the second embodiment, the electronic stethoscope of the third embodiment also does not have a buffer film (housing vibration film) 93. FIG. 14 shows that the thickness of the tip protective film 34 of a single component, namely the chip-shaped audible range sensor $X_{ij}$, should be thinner. The sound collection part 9b of the electronic stethoscope of the second embodiment shows an example in which the audible range sensor $X_{ij}$ is installed in a groove provided as a sensor fixing part on the lower side inside the housing 92b. However, in order to achieve the effect of FIG. 14, a layer on the lower side of the housing 92b is not desirably present on the lower side of the tip protection film 34 above the audible range sensor $X_{ij}$. The casing 92c of the sound collection section 9c in the electronic stethoscope of the third embodiment is an annular body (circular ring), with two rectangles appearing on both sides in the cross-sectional view of FIG. 19A (FIG. 19A is a cross-sectional view taken along the XVI-XVI direction in FIG. 18). The housing 92c is surrounded by a skin, which has at least a partially flat plate-like portion, forming a storage cavity within the skin.

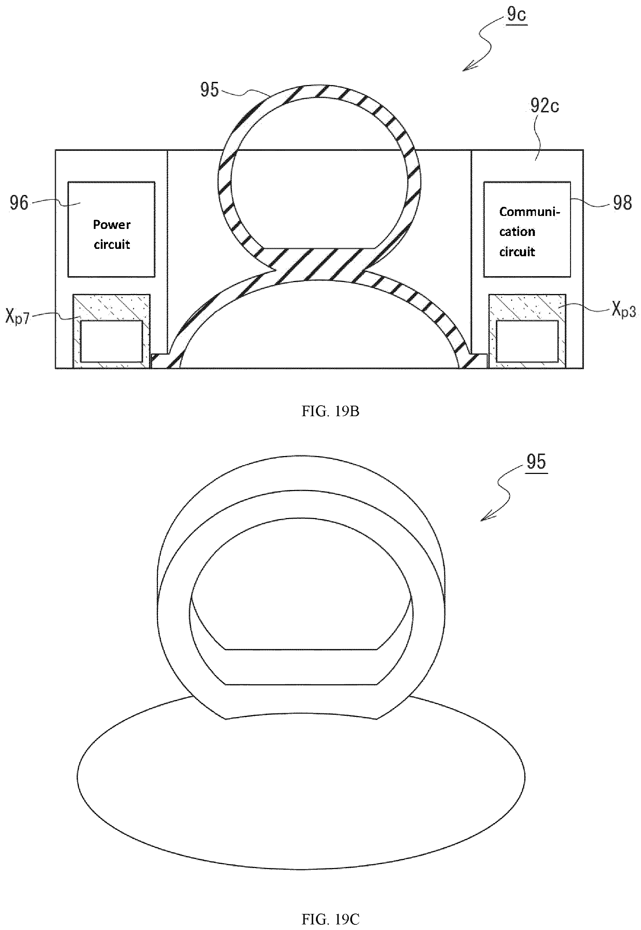

The shape of the housing 92c shown in FIG. 19A and other FIGS. is a ring with a rectangular cross-section, so the first arc surface as the outer cylindrical surface, the second arc surface as the inner cylindrical surface, the plane as the upper surface (top) of the ring portion, and the plane as the lower surface (bottom) of the ring portion are the main parts of the outer skin. In the case 92c shown in FIG. 19A and the like, the flat plate-shaped portion is the upper surface and the lower surface of the annular portion. However, in the electronic stethoscope of the third embodiment, the "signal input surface" is defined on a portion of the lower surface where the characteristic audible range signal is input. As shown in FIG. 19B, the sound collection part 9c of the electronic stethoscope of the third embodiment is provided with a adsorption assist device 95 at the center of the ring-shaped body. The component is made of an elastic material such as silicone rubber and has a vacuum suction function as a suction pad. FIG. 19C is a bird's-eye view of the adsorption assist device 95. By pressing down on the central ring, the adsorption assist device 95, which acts as a suction pad, can be adsorbed onto the surface of the diagnostic object 1 (such as a living body), allowing the sound collection component 9c to be easily and stably adsorbed onto the surface of the diagnostic object 1. By pulling the central ring upwards with a finger, the sound collection component 9c can be easily released from the surface of the diagnostic object 1.

As shown in FIG. 19A, $X_{p1}$, $X_{p2}$, $X_{p3}$, and the tip protective film 34 (see FIG. 5) of the $X_{p8}$ audible range sensor is exposed from the lower portion of the housing 92c. and is exposed when in contact with the diagnostic object 1. In the electronic stethoscope designed according to the third embodiment, the entire tip protective film of each audible range sensor $X_{p1}$, $X_{p2}$, $X_{p3}$, ... and $X_{p8}$ exposed from the bottom surface of the housing 92c and in contact with the diagnostic object 1 constitutes a "signal input surface". The sound collection section 9c of the electronic stethoscope of the third embodiment detects the sound of the patient's heart by detecting the audible range sensors $X_{p1}$, $X_{p2}$, $X_{p3}$, The tip protective film of the $X_{p8}$ is in contact with the diagnostic object 1 to detect signals, thereby improving the receiving sensitivity. Audible range sensors $X_{p1}$, $X_{p2}$, $X_{p3}$, ... for each built-in amplification component and $X_{p8}$ are chip-like single elements having a hexagonal planar pattern with the same diagonal diameter (in the description of the third embodiment below, the inclusive expression "audible range sensor $X_{ij}$" is used from time to time).

As described in the paragraph of the first embodiment, the planar pattern of the vibration cavity (shown as a dashed inner hexagon within the pattern area of the audible range sensor unit $X_{ij}$ shown in FIG. 18) corresponds to the planar pattern of the drum-shaped detection portion. That is to say, the eight inner hexagons shown in FIG. 18 correspond to the eight planar patterns of the drum-shaped detection part. As shown in FIG. 18, the sound collection part 9c of the electronic stethoscope of the third embodiment can be installed (built-in) inside the housing 92c, with audible range

sensors $X_{ij}$ with built-in amplification components arranged at equal intervals on the circumference of eight equally divided rings. The audible range sensor $X_{ij}$ may be configured as a semiconductor element having an amplification function, which amplifies a signal by directly inputting a characteristic audible range signal to a gate, similar to the sound collector 9a of the electronic stethoscope of the first and second embodiments. The sound collector 9c can be adsorbed onto the biological sample for examination using the adsorption assist device 95, such as the patient's chest, abdomen, arm, or leg, or any other body part of the patient, and can be easily released after examination.

As shown in FIG. 19A, the sound collecting portion 9c of the electronic stethoscope of the third embodiment has an annular casing 92c surrounding the adsorption assist device 95. The periphery of the adsorption assist device 95 is fixed at the lower part of the annular inner circumference. As shown in FIG. 19B, the annular housing 92c is a storage cavity. Eight hexagonal through holes are arranged at equal intervals along the lower side of the storage cavity of the ring-shaped body as sensor fixing parts. Therefore, the lower parts of the eight audible range sensors $X_{ij}$ are sequentially stored in the through holes of the eight sensor fixing parts, and are fixed to the inner diameters of the through holes and other parts using adhesive or other methods. As shown in FIG. 19A, the annular lower portion constituting the lower portion of the housing 92c is a flat and uniform plane. The bottom surface of the circular ring forming the housing 92c is aligned with the planar "outer surface" of each tip protective film of the multiple audible range sensors $X_{ij}$, and the bottom surface of the housing 92c is a uniform circular surface. In the electronic stethoscope of the third embodiment, as shown in FIG. 19B, the structure in which the tip protective film of the audible range sensor $X_{ij}$ is exposed and in contact with the diagnostic object 1 is described as "the tip protective film of the audible range sensor $X_{ij}$ is exposed on the signal input surface".

In addition, as shown in FIG. 19B, the annular storage cavity formed by the housing 92c is equipped with a power circuit 96, a signal processing circuit 97, and a communication circuit 98. The power circuit 96 provides the necessary power for the eight audible range sensors $X_{ij}$, as well as the signal processing circuit 97 and communication circuit 98. The signal processing circuit 97 receives signals from a plurality of audible range sensors $X_{ij}$ and performs signal processing on the received signals. The power circuit 96, the signal processing circuit 97, and the communication circuit 98 may be configured as a hybrid integrated circuit using a printed circuit board or the like, or at least a portion of the power circuit 96, the signal processing circuit 97, and the communication circuit 98 may be three-dimensionally mounted using a bump connection or the like.

The communication circuit 98 transmits the information processed by the signal processing circuit 97 to each information system 3 in real time, in the same manner as shown in FIG. 1A. The communication between the sound collector 9c and the information system 3 can use electromagnetic waves in the 2.4GHz frequency band, with an antenna power not exceeding 10mW. The information processed by the signal processing circuit 97 can be transmitted to the information system 3 in real time through wireless or other communication methods for visualization. The information system 3 of the relevant institution can obtain various biological information such as heartbeat, breathing, blood flow, digestion, etc. of the patient through wireless communication and other means.

By storing the eight audible range sensors $X_{ij}$ shown in FIG. 18 near the upper side in the through hole as a sensor fixing component, and detecting and amplifying characteristic hearing range signals such as biological hearing range signals, even if the annular diameter of the housing 92c is small, the sound collection component 9c can detect characteristic hearing range signals with sufficient signal strength. By reducing the number of audible range sensors from the eight audible range sensors $X_{ij}$ shown in FIG. 18, the outer diameter of the sound collector 9c can be further reduced, achieving good auscultatory effects even with a smaller size. The material of the housing 92c is not particularly limited, and may be, for example, a soft plastic such as PVC or a phthalate rubber using di-2-ethylhexyl phthalate (DEHP) as a plasticizer. In addition, it can also be made of hard plastic or metal, such as aluminum or titanium. In order to improve the adhesion with the surface of the diagnostic object 1 and match the elastic impedance, it is best to use an elastic material such as silicone rubber as a needle tip protective film.

According to the sound collection unit 9c of the electronic stethoscope of the third embodiment, as shown in FIG. 19A, by directly contacting the tip protective film of each of the multiple audible range sensors $X_{ij}$ with the surface of the diagnostic object 1, the audible range sensors $X_{ij}$ within the sound collection unit 9c can effectively detect and amplify characteristic audible range signals (bio-audible range signals) such as biological sounds. Therefore, even in an environment where the characteristic audible signal as a sound source is weak relative to the noise level, a high SN ratio can be obtained, thereby achieving high objectivity and auditory accuracy in diagnosis.

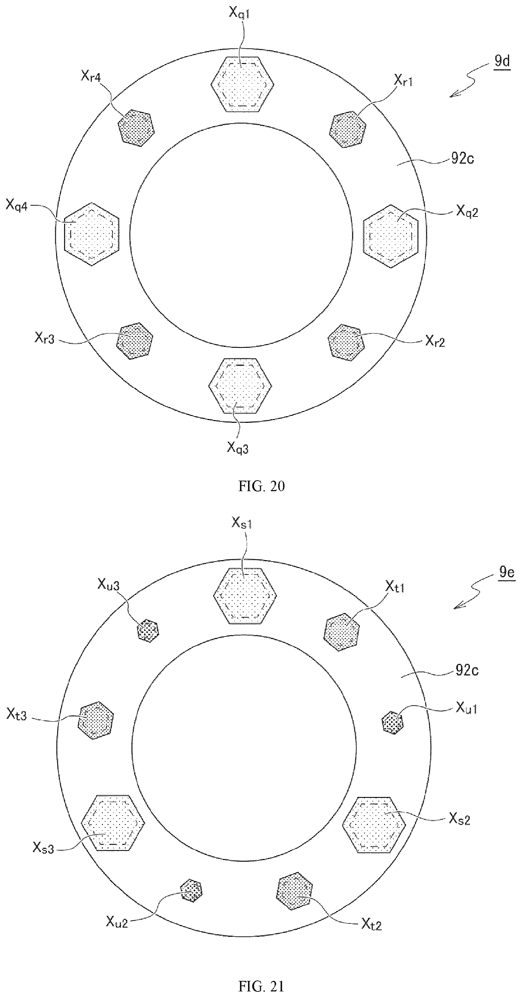

The reception sensitivity of the audible range sensors $X_{q1}$-$X_{q4}$ and $X_{r1}$-$X_{r4}$ built into the sound collection portion 9d of the electronic stethoscope according to the third embodiment depends on the inner diameter, diagonal diameter, and other dimensions occupied by the audible range sensors, as explained in the electronic stethoscope according to the first embodiment. In the electronic stethoscope of the third embodiment, as shown in FIG. 20, the first audible range sensor $X_{q1}$, the second audible range sensor $X_{q2}$, the third audible range sensor $X_{q3}$, and the fourth audible range sensor $X_{q4}$ are arranged sequentially at positions that divide the lower circumference of the sound collection section 9d into four equal parts. Each sensor includes a hexagonal drum-shaped detection section with a first diagonal diameter. The fifth audible range sensor $X_{r1}$ has a second diagonal diameter that is shorter than the first diagonal diameter, and is placed between the first and second audible range sensors $X_{q1}$ and $X_{q2}$. In addition, a sixth audible range sensor $X_{r2}$ is placed between the second audible range sensor $X_{q2}$ and the third audible range sensor $X_{q3}$, and its second diagonal diameter is shorter than the first diagonal diameter. In addition, a seventh audible range sensor $X_{r3}$ with a second diagonal diameter shorter than the first diagonal diameter is placed between the third audible range sensor $X_{q3}$ and the fourth audible range sensor $X_{q4}$, and an eighth audible range sensor $X_{r4}$ with a second diagonal diameter shorter than the first diagonal diameter is placed between the fourth audible range sensor $X_{q4}$ and the first audible range sensor $X_{q1}$.

The receiving sensitivity of the audible range sensors $X_{s1}$-$X_{s3}$, $X_{t1}$-$X_{t3}$, and $X_{u1}$-$X_{u3}$ built into the sound collection part 9e of the electronic stethoscope according to the third embodiment depends on the size of the inner diameter and diagonal diameter occupied by the audible range sensors, which has been described in the electronic stethoscope according to the first embodiment. In the electronic stethoscope according to the third embodiment, as shown in FIG. 21, the first audible range sensor $X_{s1}$, the second audible range sensor $X_{s2}$, and the third audible range sensor $X_{s3}$ are sequentially arranged at positions equally divided by the circle 3 at the lower portion of the torus of the sound collecting portion 9e. As shown in FIG. 21, a fourth audible range sensor $X_{t1}$ having a second diagonal diameter shorter than the first diagonal diameter is disposed at a position rotated clockwise by 40 degrees from the first audible range sensor $X_{s1}$. In addition, a fifth audible range sensor $X_{t2}$ having a second diagonal diameter shorter than the first diagonal diameter is disposed at a position rotated clockwise by 40 degrees from the second audible range sensor $X_{s2}$. A sixth audible range sensor $X_{t3}$ having a second diagonal diameter shorter than the first diagonal diameter is disposed at a position rotated clockwise by 40 degrees from the third audible range sensor $X_{s3}$.

As shown by the dashed line in FIG. 20, the audible range sensors $X_{q1}$-$X_{q4}$ in the detection array have a drum-shaped detection portion with a large diagonal diameter, while the audible range sensors $X_{r1}$-$X_{r4}$ have a drum-shaped detection portion with a small diagonal diameter. The mechanical resonance frequencies of the audible range sensors $X_{q1}$-$X_{q4}$ and $X_{r1}$-$X_{r4}$ are different. Therefore, by intentionally shifting the respective mechanical resonance frequencies of the audible range sensors $X_{q1}$-$X_{q4}$ and the audible range sensors $X_{r1}$-$X_{r4}$ in the array composed of multiple drum-shaped detection sections, the frequency band of the electronic stethoscope according to the third embodiment can be expanded, thereby improving overall reception sensitivity.

In addition, as shown in FIG. 21, a seventh audible range sensor $X_{u1}$ with a third diagonal diameter shorter than the second diagonal diameter is located at a position 40° clockwise around the circumferential direction from the fourth audible range sensor $X_{t1}$. The third diagonal diameter of the eighth audible range sensor $X_{u2}$ is shorter than the second diagonal diameter, and is located at a position 40° clockwise around the circumference from the fifth audible range sensor $X_{t2}$. The ninth audible range sensor $X_{u3}$, which has a shorter diameter than the second diagonal, is located at a position 40° clockwise from the sixth audible range sensor $X_{t3}$ in the circumferential direction. FIG. 14 shows that the thickness of the tip protection film on the upper electrode protection film 26 of the audible range sensor $X_{ij}$ should be thinner.

As shown by the dotted line in FIG. 21, the audible range sensors $X_{s1}$-$X_{s3}$ of the drum-shaped detection unit with a large diagonal diameter, the audible range sensors $X_{t1}$-$X_{t3}$ of the drum-shaped detection unit with a medium diagonal diameter, and the audible range sensors $X_{u1}$-$X_{u3}$ of the drum-shaped detection unit with a small diagonal diameter can be used in the detection unit array including the audible range sensors $X_{s1}$-$X_{s3}$, $X_{t1}$-$X_{t3}$, and $X_{u1}$-$X_{u3}$ of the drum-shaped detection unit with a small diagonal diameter. The mechanical resonance frequencies of the audible range sensors $X_{s1}$-$X_{s3}$, $X_{t1}$-$X_{t3}$, and $X_{u1}$-$X_{u3}$ are different from each other. Therefore, by intentionally shifting the mechanical resonance frequencies of the audible range sensors $X_{s1}$-$X_{s3}$, $X_{t1}$-$X_{t3}$, and $X_{u1}$-$X_{u3}$ in the array composed of multiple drum-shaped detection units shown in FIG. 21, it is possible to expand the frequency band of the electronic stethoscope according to the third embodiment and improve the overall receiving sensitivity.

The configurations shown in FIG. 20 and FIG. 21 also allow the effective detection of characteristic audible signals as voltage signals by directly contacting the surface of the diagnostic object 1 with the tip protective film of each of the multiple audible range sensors $X_{ij}$. Each sensor has a mechanical resonance frequency within the audible range. In particular, if each of the plurality of audible range sensors $X_{ij}$ integrates an insulated gate semiconductor as an internal structure for amplifying the characteristic audible range signal, the detected characteristic audible range signal can be further amplified by relying on the electrostatic induction effect of the internal structure. Therefore, even in an environment where the characteristic audible signal as a sound source is weak compared to the noise level, a high signal-to-noise ratio can be obtained, resulting in a diagnosis with high objectivity and auditory accuracy. As described above, according to the electronic stethoscope of the third embodiment, even in an environment where the characteristic audible signal as the sound source is weak compared to the noise level, a high signal-to-noise ratio can be achieved, thereby realizing an electronic stethoscope with high objectivity and auscultatory accuracy.

**Fourth embodiment**

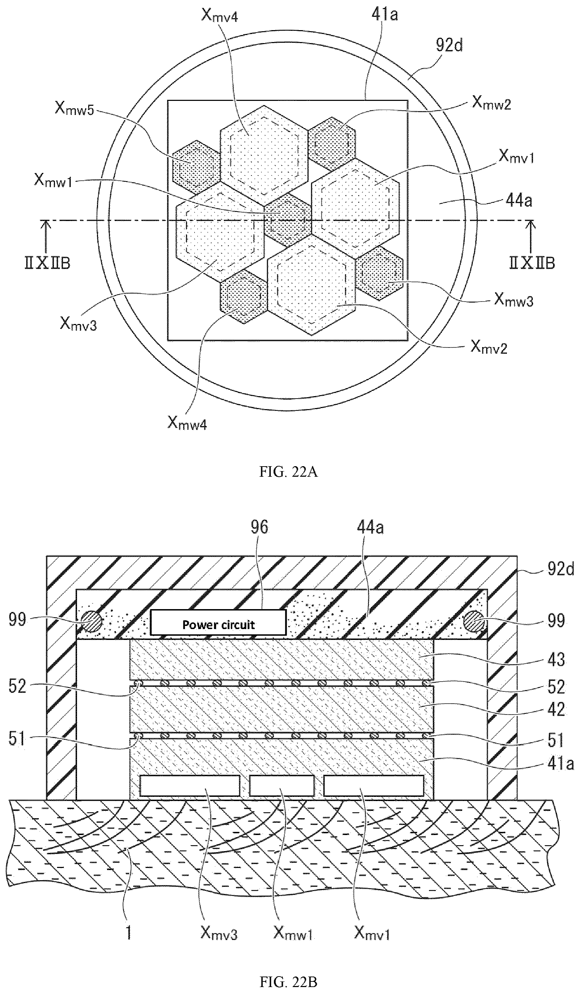

In the electronic stethoscopes of the first to third embodiments described above, a hybrid mounting structure is described in which chip-shaped audible range sensors $X_{ij}$, which are separate elements, are arranged on the signal input side of the lower surfaces of housings 92a, 92b, 92c, and the like. In the electronic stethoscope according to the fourth embodiment of the present disclosure, as shown in FIGS. 22A and 22B, a sensor array including audible range sensor

units $X_{mv1}$-$X_{mv4}$ and $X_{mw1}$-$X_{mw5}$ is monolithically integrated on the surface of a semiconductor chip (first semiconductor chip) 41a, which is an independent element in the electronic stethoscopes of the first to third embodiments. It is different from the array structure of the audible range sensor $X_{ij}$, which is as described in the first embodiment, with a planar pattern of the vibration cavity as shown in FIG. 22A, which is respectively an inner hexagon with dashed lines within the pattern area of the audible range sensor units $X_{mv1}$-$X_{mv4}$ and $X_{mw1}$-$X_{mw5}$, corresponding to the planar pattern of the drum-shaped detection component.

That is to say, the nine inner hexagons shown in FIG. 22A correspond to the planar patterns of the nine drum-shaped detection areas, while the blank areas shown by the three white rectangles in FIG. 22B represent the vibration cavities that serve as the drum-shaped detection areas. As in the first to third embodiments, the three white rectangles shown in FIG. 22B are highly exaggerated schematic diagrams, with aspect ratios different from the actual structures. Each actual vibration cavity is a flat rectangle with a width of about 1.2-2.4mm and a height of about 2$\mu$m, so the height is almost invisible. Each of the audible range sensor units $X_{mv1}$-$X_{mv4}$ and $X_{mw1}$-$X_{mw5}$ should include an insulated gate semiconductor as an internal structure to amplify the characteristic audible range signal, but it is not necessary to include an insulated gate semiconductor as an internal structure.

As shown in FIG. 22B, the semiconductor chip 41a employs a flip-chip layout, so that the sensor array including the audible range sensor units $X_{mv1}$-$X_{mv4}$ and $X_{mw1}$-$X_{mw5}$ is located on the signal input side of the lower surface of the sound collection component housing 92d. Then, a characteristic sound signal is input to the upper electrode of the drum-shaped detection portion of each monolithically integrated audible range sensor unit $X_{mv1}$-$X_{mv4}$ and $X_{mw1}$-$X_{mw5}$, and the upper electrode vibrates, operating as a drum-shaped (capacitive) audible range sensor. Each of the audible range sensor units $X_{mv1}$-$X_{mv4}$ and $X_{mw1}$-$X_{mw5}$ has a mechanical resonance frequency in the audible range, similar to the electronic stethoscopes in the first to third embodiments, and therefore has high receiving sensitivity (see, for example, FIG. 8). Like the electronic stethoscopes of the second and third embodiments, the electronic stethoscope of the fourth embodiment does not have the buffer film 93 described in the electronic stethoscope of the first embodiment. The specific structure of each unit of the audible range sensor units $X_{mv1}$-$X_{mv4}$ and $X_{mw1}$-$X_{mw5}$ is not shown, but when the insulated gate semiconductor is integrated as an internal structure, it is similar to the drum-shaped structure already described in FIG. 5. As shown in FIG. 14, the tip protective film 34 (see FIG. 5) of the audible range sensor unit $X_{mij}$ should be thinner.

As shown in FIG. 22A, the first audible range sensor unit $X_{mw1}$ is located at the center of the rectangular semiconductor chip 41a, and includes a hexagonal drum-shaped detection portion having a first diagonal diameter. The detailed structural example of the planar pattern of the first audible range sensor unit $X_{mw1}$ is similar to the structure already described in FIG. 4, where insulated gate semiconductors are integrated together as internal structures. However, the split structure of the electrodes into a central lower electrode and a peripheral lower electrode shown in FIG. 4 is not necessarily essential. As shown in FIG. 22A, the first audible range sensor unit $X_{mw1}$, the second audible range sensor unit $X_{mv1}$, the third audible range sensor unit $X_{mv2}$, the fourth audible range sensor unit $X_{mv3}$, and the fifth audible range sensor unit $X_{mv4}$ are monolithically integrated on the surface of the semiconductor chip 41a, and the second diagonal diameter of each unit is longer than the first diagonal diameter. The following elements are monolithically integrated on the surface of the semiconductor chip 41a. As shown in FIG. 4, the planar patterns of the second audible range sensor unit $X_{mv1}$, the third audible range sensor unit $X_{mv2}$, the fourth audible range sensor unit $X_{mv3}$, and the fifth audible range sensor unit $X_{mv4}$ may also adopt a hexagonal shape.

In addition, between the fifth audible range sensor unit $X_{mv4}$ and the second audible range sensor unit $X_{mv1}$ on the semiconductor chip 41a, a sixth audible range sensor unit $X_{mw2}$ is monolithically integrated, with a first diagonal diameter shorter than a second diagonal diameter. Similarly, a seventh audible range sensor unit $X_{mw3}$ having a first diagonal diameter is monolithically integrated between the second audible range sensor unit $X_{mv1}$ and the third audible range sensor unit $X_{mv2}$, and an eighth audible range sensor unit $X_{mw4}$ having a first diagonal diameter is monolithically integrated between the third audible range sensor unit $X_{mv2}$ and the fourth audible range sensor unit $X_{mv3}$. In addition, a ninth audible range sensor unit $X_{mw5}$ having a first diagonal diameter is monolithically integrated between the fourth audible range sensor unit $X_{mv3}$ and the fifth audible range sensor unit $X_{mv4}$. As shown in FIG. 4, the planar patterns of the sixth audible range sensor unit $X_{mw2}$, the seventh audible range sensor unit $X_{mw3}$, the eighth audible range sensor unit $X_{mw4}$, and the ninth audible range sensor unit $X_{mw5}$ can also adopt a hexagonal shape.

As shown by the dashed lines in FIG. 22A, the monolithically integrated arrays of the audible range sensor units $X_{mv1}$-$X_{mv4}$ having large diagonal drum-shaped detection areas and the audible range sensor units $X_{mw1}$-$X_{mw5}$ having small diagonal drum-shaped detection areas have different mechanical resonance frequencies from each other, as can be seen from FIG. 8 and other figures. When the audible range sensor units $X_{mv1}$-$X_{mv4}$ and the audible range sensor units $X_{mw1}$-$X_{mw5}$ having different mechanical resonance frequencies are monolithically integrated on the same semiconductor chip 41a to form an array, the first, second, fourth, and sixth factors that determine frequency characteristics, which have been previously introduced, can be conveniently used together without increasing the number of processes. Therefore, in the semiconductor chip 41a shown in FIGS. 22A and 22B, the resonant frequency is also changed by changing the planar size, which is the third factor determining the frequency characteristics. By deliberately changing the mechanical resonance frequency of each audible range sensor unit in a monolithic integrated array consisting of multiple drum-shaped detection parts, the frequency band of the electronic stethoscope of the fourth embodiment can be expanded, thereby improving overall receiving sensitivity.

In the following description, for convenience, each of the audible range sensor units $X_{mv1}$-$X_{mv4}$ and $X_{mw1}$-$X_{mw5}$ is not individually represented, but is collectively represented as "audible range sensor unit $X_{mij}$" when necessary. As shown in FIG. 22A and FIG. 22B, the housing 92d of the sound collection part of the electronic stethoscope of the fourth embodiment is cylindrical, with a U-shaped cross-section cutting the center line of the circle (FIG. 22B is a cross-section of FIG. 22A viewed from the direction of IIXIIB-IIXIIB). The outer skin of the housing 92d corresponds to the portion of the housing 92d that exhibits a U-shaped cross section, with the upper surface being a flat plate-like portion (plane portion) and the lower surface being an open end. That is to say, FIG. 22B is a model diagram illustrating the formation of a storage cavity within a U-shaped outer skin in cross section. However, it should be noted that the lower surface of the storage cavity is an open end face, and the schematic diagram showing the storage cavity appears to be an open space, without showing the actual installation structure. As shown in FIGS. 22A and 22B, the housing 92d of the sound collection part of the electronic stethoscope according to the fourth embodiment is cylindrical, with the cross section of the center line of the circle being U-shaped (FIG. 22B is a cross section viewed from the direction IIXIIBIIXIIB of FIG. 22A). The outer skin of the housing 92d corresponds to the U-shaped section, and is schematically represented as a flat plate-like portion (flat portion) on the top and an open end on the bottom. That is, FIG. 22B is a model diagram for explaining the formation of a storage cavity inside a skin with a U-shaped cross section. However, it should be noted that the lower part of the accommodation chamber is an open end and the schematic representation of the accommodation chamber as if it were an open space does not represent the actual installation structure.

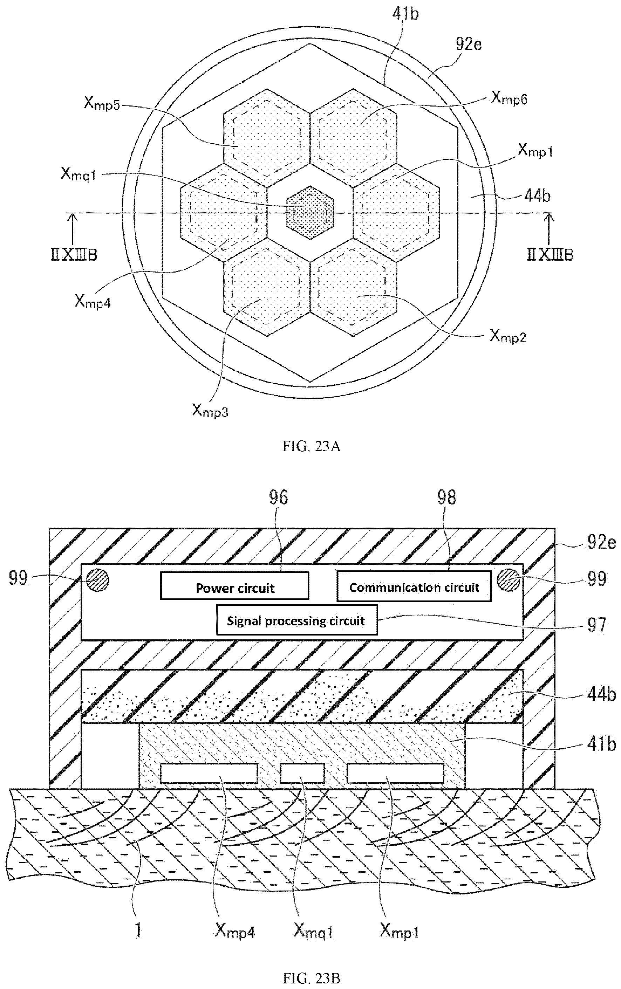

At the position below the open end, the semiconductor chip 41a is flip-chip mounted so that the audible range sensor units $X_{mv1}$-$X_{mv4}$ and $X_{mw1}$-$X_{mw5}$ face downward, thereby enclosing the main area of the open end. FIG. 22B is a schematic diagram illustrating how to make the storage cavity a nearly closed space by placing the semiconductor chip 41a. Similar to a standard stethoscope, the electronic stethoscope in the fourth embodiment should be periodically disinfected. After each examination of a patient, it should be wiped down with a cloth (made of alcohol cotton) dipped in 70% isopropyl alcohol or with soapy water to remove organic matter. Therefore, in the actual installation structure, the bottom surface of the housing 92d is not an open end, but a plate-shaped sealing layer that fills the bottom opening area, forming a sealing surface that is impervious to isopropyl alcohol or soapy water. This plate-shaped sealing layer is omitted in FIG. 23B. The plate-shaped sealing layer, which is omitted in the figure, is aligned with the outer lower surfaces of the audible range sensor units $X_{mv1}$-$X_{mv4}$ and $X_{mw1}$-$X_{mw5}$. As shown in FIG. 16, if regular disinfection, cleaning, and other maintenance in actual use are taken into account, the entire lower part of the housing 92d can be covered with silicone rubber or similar materials.

In the electronic stethoscope according to the fourth embodiment, a "signal input surface" for inputting a characteristic audible range signal is defined in a portion of the area corresponding to the lower portion of the almost enclosed space composed of the housing 92d and the semiconductor chip 41a shown in FIG. 22B. Then, as shown in FIG. 22B, by exposing each upper end protective film of the upper portion of the plurality of monolithically integrated audible range sensor units $X_{mij}$ from the lower surface of the housing 92d, the top protective film of the audible range sensor unit $X_{mij}$ can be brought into contact with the surface of the diagnostic object 1, respectively. In other words, in the electronic stethoscope according to the fourth embodiment, the respective top protective films of the plurality of audible range sensor units $X_{mij}$ exposed from the lower surface of the housing 92d contact the surface of the diagnostic object 1 to constitute a signal input surface. The housing 92d of the sound collector of the electronic stethoscope of the fourth embodiment detects a characteristic audible range signal by using a top protective film of each of a plurality of audible range sensor units $X_{mij}$ that are in contact with the diagnostic object 1, thereby improving the reception sensitivity.

Each of the audible range sensor units $X_{mw1}$-$X_{mw5}$ is an element of a monolithic integrated circuit forming a hexagonal planar pattern having the same first diagonal diameter, and each of the audible range sensor units $X_{mv1}$-$X_{mv4}$ is an element of a monolithic integrated circuit forming a hexagonal planar pattern having the same second diagonal diameter. As shown in FIG. 22A, the housing 92d of the electronic stethoscope of the fourth embodiment has a semiconductor chip 41a that integrates multiple audible range sensor units $X_{mij}$ in a single piece, corresponding to multiple drum-shaped detection sections with different diagonal diameters, located at the center of the housing 92d. Moreover, the diagonal diameter of the first audible range sensor unit $X_{mw1}$, which is arranged in the central portion of the semiconductor chip 41a and has a drum-shaped detection portion, is arranged near the first audible range sensor unit $X_{mw1}$ and is smaller than the diagonal diameters of the second to fifth audible range sensor units $X_{mv1}$-$X_{mv4}$, each of which has a drum-shaped detection portion.

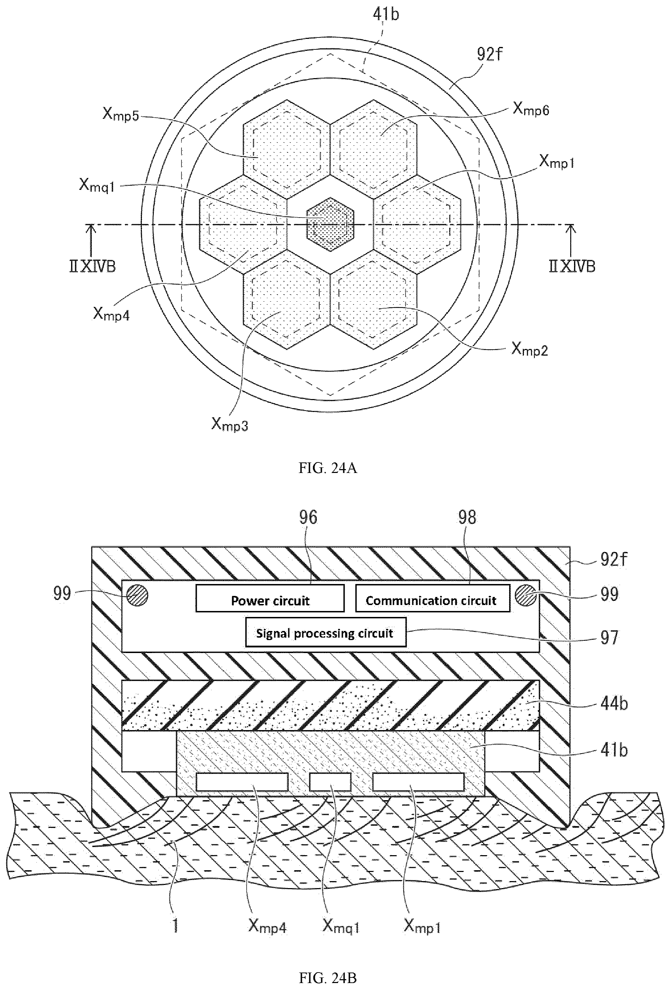

As shown in FIGS. 24A and 24B, the housing 92d can be configured to include a closed cylindrical shape, and nine hexagonal through holes can be provided along the lower surface of the storage cavity formed by the closed cylindrical shape. When a closed cylindrical skin is configured, each of the nine audible range sensor units $X_{mij}$ can be inserted into nine through-holes, allowing the protective film on the upper tip of each audible range sensor unit $X_{mij}$ to protrude from the skin formed by the housing 92d or align with the exterior of the diagnostic object 1.

Further, as shown in FIG. 22B, a second semiconductor chip 42 is stacked on the upper portion of a semiconductor chip (first semiconductor chip) 41a through a plurality of gold (Au) bumps 51, and a third semiconductor chip 43 is stacked on the upper portion of the second semiconductor chip 42 through a plurality of gold bumps 52, forming a three-dimensional integrated circuit. The three-layer stacked structure of the first semiconductor chip 41a, the second semiconductor chip 42, and the third semiconductor chip 43 is one example, and the third semiconductor chip 43 may be omitted. The two-layer stacked structure of the first semiconductor chip 41a and the second semiconductor chip 42 may constitute a three-dimensional integrated circuit.

In addition, above the three-dimensional integrated circuit composed of the first semiconductor chip 41a, the second semiconductor chip 42, and the third semiconductor chip 43, as shown in FIG. 22B, a pressure absorption layer 44a composed of an elastic material is stacked so as to contact the upper surface of the storage cavity formed by the housing 92d. The pressure absorbing layer 44a functions to absorb the pressure applied to each of the audible range sensor units $X_{mv1}$-$X_{mv4}$ and $X_{mw1}$-$X_{mw5}$ when the housing 92d is pressed against the diagnostic object 1, thereby protecting the audible range sensor units $X_{mv1}$-$X_{mv4}$ and $X_{mw1}$-$X_{mw5}$ from damage. Furthermore, the power circuit 96 is housed inside the pressure absorption layer 44a, and constitutes a three-dimensional integrated circuit with the first semiconductor chip 41a, the second semiconductor chip 42, and the third semiconductor chip 43 to form a three-dimensional hybrid integrated circuit.

The power circuit 96 provides the required power for the nine audible range sensor units $X_{mij}$ integrated in the first semiconductor chip 41a, the signal processing circuit integrated in the second semiconductor chip 42, and the communication circuit integrated in the third semiconductor chip 43. When the three-dimensional integrated circuit is configured as a two-layer stacked structure, the power circuit 96 provides the required power for the communication circuit integrated in the second semiconductor chip 42. The signal processing circuit integrated in the second semiconductor chip 42 receives signals from a plurality of audible range sensor units $X_{mij}$ of different sizes, converts the received signals into A/D signals, and then performs signal processing, such as smoothing frequency characteristics using digital technology and noise processing.

As shown in FIG. 22B, the loop antenna 99 is located near the inner wall of the housing 92d within the pressure absorption layer 44a. The antenna 99 may employ a dipole antenna, a helical antenna, and a planar antenna (patch antenna). Although the wiring and the like are omitted in the figure, the communication circuit integrated in the third semiconductor chip 43 is of course electrically connected to the antenna 99 through bumps, surface wiring, and the like. In the three-dimensional integrated circuit with a two-layer stacked structure, the communication circuit integrated in the second semiconductor chip 42 is electrically connected to the antenna 99.

Through the antenna 99, the communication circuit integrated in the third semiconductor chip 43 can transmit the information signal processed by the signal processing circuit of the second semiconductor chip 42 to each information system 3 in real time, in the same way as shown in FIG. 1A when configuring a three-dimensional integrated circuit with a two-layer stacked structure. The information signal processed by the signal processing circuit integrated in the chip 42 is transmitted to each information system 3 in real time through the communication circuit integrated in the second semiconductor chip 42. In the communication between the sound collector's housing 92d and the information system, wireless communication can be performed using electromagnetic waves in the 2.4GHz frequency band with an antenna power of 10mW or less. The information processed by the signal processing circuit is transmitted to the information system in real time through antenna 99 or other radio communication methods for visualization. Various biological information generated by the patient's heartbeat, breathing, blood flow, digestion, etc. can be obtained by the information system of the relevant institution through wireless communication and other methods.

By detecting characteristic audible range signals, such as biological audible range signals, using the nine audible range sensor units $X_{mij}$ shown in FIG. 22A, even if the housing 92d is small and the outer diameter is short, it is possible to detect characteristic audible range signals with sufficient signal strength. By reducing the number of nine audible range sensor units $X_{mij}$ shown in FIG. 22A, the outer diameter of the sound collector housing 92d can be further reduced, achieving good auscultatory effects even at a smaller size. the material of the housing 92d is not particularly limited. For example, it can be soft plastic such as PVC, phthalate rubber using DEHP as a plasticizer, hard resin, or metal such as Al or Ti. In order to improve the elastic impedance matching with the diagnostic object 1, it is best to use elastic materials such as silicone rubber as a needle tip protective layer.

According to the electronic stethoscope of the fourth embodiment, as shown in FIG. 22B, each of the plurality of audible range sensor units $X_{mij}$ has a mechanical resonance frequency within the audible range, and by directly contacting the tip protective film of each unit with the surface of the diagnostic object 1, it is possible to effectively detect characteristic audible frequency signals as voltage signals. In addition, if each of the multiple audible range sensor units $X_{mij}$ integrates an insulated gate semiconductor as an internal structure, the detected characteristic acoustic frequency signal can be self-amplified by the electrostatic induction effect of the internal structure. Therefore, even in an environment where the characteristic auditory signal as a noise source is weak relative to the noise level, a high SN ratio can be achieved, resulting in a diagnosis with high objectivity and auditory accuracy.

**First variation of the fourth embodiment**

As shown in FIG. 23A and FIG. 23B, the housing 92e of the sound collection part of the electronic stethoscope of the first variation of the fourth embodiment of the present disclosure is cylindrical, which looks like the structure of a high-rise building, where the upper part of the cross section of the center line of the circle is rectangular (FIG. 23B is a cross section viewed from the direction IIXIIIB-IIXIIIB of FIG. 23A.). In the electronic stethoscope of the fourth embodiment shown in FIGS. 22A and 22B, a rectangular semiconductor chip 41a in which audible range sensor units $X_{mv1}$-$X_{mv4}$ and $X_{mw1}$-$X_{mw5}$ are monolithically integrated is used inside the housing 92d. Therefore, dead corners are generated at the four corners of the chip along the diagonal direction of the rectangle.

In contrast, as shown in FIGS. 23A and 23B, in the electronic stethoscope designed according to the first variation of the fourth embodiment of the present disclosure, a hexagonal semiconductor chip 41b with monolithically integrated audible range sensor units $X_{mp1}$- $X_{mp6}$ and $X_{mq1}$ is stored in a lower storage cavity located under the floor of a high-rise building, and a housing 92e is included therein. This can reduce dead space and achieve more miniaturization. As mentioned earlier, the planar pattern of the vibration cavity (as shown in FIG. 23A, the inner hexagons with dashed lines within the pattern areas of the audible range sensor units $X_{mp1}$-$X_{mp6}$ and $X_{mq1}$, respectively) corresponds to the planar pattern of the drum detection unit. That is to say, the seven inner hexagons shown in FIG. 23A correspond to the seven planar patterns of the drum-shaped detection component, while the blank areas of the three white rectangles shown in FIG. 23B represent the vibration cavity of the drum-shaped detection component. However, the three white rectangles shown in FIG. 23B are only schematic diagrams, and each actual vibration cavity is a flat rectangle with a width of approximately 1.2-2.4mm and a height of approximately $2\mu m$.

The outer skin of the housing 92e corresponds to the outer skin portion of the high-rise building. FIG. 23B provides a structural diagram, in which the upper surface of the rectangular portion of the upper part of the enclosure structure 92e is a flat plate portion (plane portion), while the lower surface of the lower part of the floor is an open end. That is to say, the living space represented by the lower part of the floor and the upper rectangular part of the high-rise structure are the storage chambers of the "lower storage chamber" and the "upper storage chamber", respectively. In the first variation of the fourth embodiment, the upper storage cavity of the living space composed of high-rise buildings and the lower storage cavity of the underfloor portion are collectively referred to as "storage cavities". This is a convenient expression that illustrates that the semiconductor chip 41b is located in the bottom portion of the high-rise building, to approximately close the open end of the lower storage chamber of the bottom portion, thereby forming the lower storage chamber into an approximately enclosed space.

In practical applications, the electronic stethoscope of the first variant of the fourth embodiment needs to be disinfected regularly, such as removing organic matter after each examination of the patient. For this reason, the lower surface of the housing 92e is not an open end, and a plate-shaped sealing layer is filled in the opening portion of the lower surface to form a sealing surface that is impervious to isopropyl alcohol and soapy water, but this is omitted in FIG. 23B. The plate-shaped sealing layer, which is omitted in the figure, is aligned with the outer lower surfaces of the audible range sensor units $X_{mv1}$-$X_{mv4}$ and $X_{mw1}$-$X_{mw5}$. As shown in FIG. 16, the entire lower portion of the housing 92e may be covered with silicone rubber or the like if periodic disinfection, cleaning, and other maintenance in actual use are taken into consideration. In FIG. 23B, the structure in which the upper and lower storage chambers are divided by a uniform flat plate is illustrated as an example, but it is only an example. In the electronic stethoscope of the first variation of the fourth embodiment, the upper and lower storage chambers are spaces collectively referred to as "storage chambers", so there may be gaps or openings on the plates that divide the upper and lower storage chambers. In addition, the storage cavity in the cross-sectional view shown in FIG. 22B can also be divided by a flat plate portion that protrudes horizontally from the middle of the side wall with a downward U-shaped structural feature, as shown in FIG. 22A, to form a sandwich floor. FIG. 23B reveals a convenient model representation to illustrate that the semiconductor chip 41b is located in the lower storage chamber of the ground floor of a high-rise building, with its position approximately enclosing the open end of the ground floor, thereby forming the lower storage chamber into an approximately enclosed space.

In the electronic stethoscope of the first variation of the fourth embodiment, a portion of the bottom surface of the space defined in the lower reservoir of the housing 92e's bottom plate, as shown in FIG. 23B, serves as the "signal input surface", where characteristic sound path signals are input. Then, as shown in FIG. 23B, on the signal input surface of the housing 92e, the tip protective film 34 (see FIG. 5) located at the upper end of the multiple monolithically integrated audible range sensor units $X_{mij}$ is exposed to the contact surface of the diagnostic object 1 for each unit. In this way, the top protective films of multiple audible range sensor units $X_{mij}$ can be respectively in contact with the surface of the diagnostic object 1. In other words, in the electronic stethoscope of the first variation of the fourth embodiment, the entire top protective film of each unit of the multiple audible range sensor units $X_{mij}$ is exposed from the lower surface of the space defined in the lower storage chamber (i.e., the lower portion of the floor of the housing 92e) to come into contact with the diagnostic object 1, forming a signal input surface. The sound collector housing 92e of the electronic stethoscope of the first variation of the fourth embodiment improves the receiving sensitivity by detecting characteristic audible range signals from the tip protective films of a plurality of audible range sensor units $X_{mij}$ that are in contact with the diagnostic object 1.

As shown in FIG. 23B, the semiconductor chip 41b adopts a flip-chip arrangement, with the sensor array containing audible range sensor units $X_{mp1}$-$X_{mp6}$ and $X_{mq1}$ located on the signal input side of the housing 92e. Then, a characteristic sound signal is input to the upper electrode of the drum-shaped detection portion of each monolithically integrated audible range sensor unit $X_{mp1}$-$X_{mp6}$ and $X_{mq1}$, and the upper electrode vibrates, operating as a drum-shaped (capacitive) audible range sensor. Each of the audible range sensor units $X_{mp1}$-$X_{mp6}$ and $X_{mq1}$ has a mechanical resonance frequency in the audible range, similar to the electronic stethoscopes in the first to fourth embodiments, and therefore has high receiving sensitivity (see, for example, FIG. 8). The specific structure of each audible range sensor unit $X_{mp1}$-$X_{mp6}$ and $X_{mq1}$ is not shown, but when the insulated gate semiconductor is integrated as an internal structure, it is similar to the drum-shaped structure shown in FIG. 5, which has been described previously. In the structure shown in FIG. 5, the thickness of the protective film on the tip of the audible range sensor unit $X_{mij}$ should be thinner, as shown in FIG. 14.

As shown in FIG. 23A, the first audible range sensor unit $X_{mq1}$ is located at the center of the rectangular semiconductor chip 41b, and includes a hexagonal drum-shaped detection portion having a first diagonal diameter. A detailed structural example of the planar pattern of the first audible range sensor unit $X_{mq1}$ is similar to that described in FIG. 4, where the internal structure includes an integrated insulated gate semiconductor. However, the division structure shown in FIG. 4, which divides the unit into a central lower electrode and a peripheral lower electrode, is not necessarily essential.

Moreover, as shown in FIG. 23A, on the surface of the semiconductor chip 41b, in addition to the first audible range sensor unit $X_{mq1}$, second audible range sensor units $X_{mp1}$, third audible range sensor units $X_{mp2}$, fourth audible range sensor units $X_{mp3}$, fifth audible range sensor units $X_{mp4}$, sixth audible range sensor units $X_{mp5}$, and seventh audible range sensor units $X_{mp6}$ each having a second diagonal diameter longer than the first diagonal diameter are arranged at positions equally dividing the circle 6 and are monolithically integrated. The planar patterns of the second audible range sensor unit $X_{mp1}$, the third audible range sensor unit $X_{mp2}$, the fourth audible range sensor unit $X_{mp3}$, the fifth audible range sensor unit $X_{mp4}$, the sixth audible range sensor unit $X_{mp5}$, and the seventh audible range sensor unit $X_{mp6}$ can also adopt the same hexagonal shape as illustrated in FIG. 4.

The audible range sensor unit $X_{mq1}$ located at the center has a hexagonal planar pattern with a first diagonal diameter, and each of the peripheral hearing sensor units $X_{mp1}$-$X_{mp6}$ is an element of a monolithic integrated circuit having a hexagonal planar pattern with the same second diagonal diameter. As shown in FIG. 23A, the electronic stethoscope of the first variation of the fourth embodiment has a semiconductor chip 41b in its housing 92e, which integrates multiple audible range sensor units $X_{mij}$ on a single chip, corresponding to multiple drum-shaped detection sections with different diagonal diameters, arranged in the center of the housing 92e. The diagonal diameter of the first audible range sensor unit $X_{mq1}$, which includes a drum-shaped detection portion disposed at the center of the semiconductor chip 41b, is smaller than the diagonal diameters of the second to seventh audible range sensor units $X_{mp1}$-$X_{mp6}$, which are arranged around the first audible range sensor unit $X_{mq1}$, each having a drum-shaped detection portion. In the following description, for convenience, each of the audible range sensor units $X_{mp1}$-$X_{mp6}$ and $X_{mq1}$ should not be individually mentioned, but should be collectively referred to as "audible range sensor units $X_{mij}$".

As shown by the dashed line in FIG. 23A, the auditory sensor units $X_{mp1}$-$X_{mp6}$ in the monolithic integrated array are drum-shaped detection sections with a larger diagonal diameter, while the audible range sensor unit $X_{mq1}$ is a drum-shaped detection section with a smaller diagonal diameter. The mechanical resonance frequencies between them are different. Therefore, in a monolithic integrated array composed of multiple drum-shaped detection sections, the mechanical resonance frequencies of each audible range sensor unit $X_{mij}$ can be intentionally shifted from each other to increase the frequency bandwidth, thereby improving the overall receiving sensitivity of the semiconductor chip 41b.

As shown in FIG. 24A and FIG. 24B, the cross-sectional structure of the lower storage chamber, that is, the lower part of the floor of the housing 92e, can be made into a closed rectangle that is stacked in two levels, similar to two floors. In FIG. 24B, the structural schematic diagram shows a two-story building with two rectangles stacked together. The lower rectangle includes the lower storage chamber, and the upper rectangle includes the upper storage chamber. In this two-story-like structure, the lower surface of the lower storage chamber can be provided with seven hexagonal through holes corresponding to the seven hexagons in FIG. 24A. When the closed lower storage chamber configuration is completed, the seven audible range sensor units $X_{mij}$ can be inserted into the seven through holes, and the top protective films of the multiple audible range sensor units $X_{mij}$ can protrude from the lower storage chamber or align externally to contact the diagnostic object 1.

In addition, as shown in FIG. 23B, a pressure absorption layer 44b composed of an elastic material is stacked on the upper portion of the semiconductor chip 41b so as to contact the upper surface of the lower storage cavity. The pressure absorbing layer 44b functions to absorb the pressure applied to each of the audible range sensor units $X_{mp1}$-$X_{mp6}$ and $X_{mq1}$-$X_{mq5}$ when the housing 92e is pressed against the diagnostic object 1, thereby protecting the audible range sensor units $X_{mp1}$-$X_{mp6}$ and $X_{mq1}$-$X_{mq5}$ from damage. The upper storage chamber of the high-rise building shown in FIG. 23B contains a power circuit 96, a signal processing circuit 97, and a communication circuit 98, which together with the first semiconductor chip 41b in the lower storage chamber form a three-dimensional hybrid integrated circuit.

The power circuit 96 provides necessary power to the seven audible range sensor units $X_{mij}$, the signal

processing circuit 97, and the communication circuit 98 integrated in the first semiconductor chip 41b. The signal processing circuit 97 receives signals from multiple audible range sensor units $X_{mij}$ and processes the received signals. As shown in FIG. 23B, the loop antenna 99 is located in the upper storage cavity of the high-rise building. The antenna 99 may employ a dipole antenna, a helical antenna, or a planar antenna. The input and output terminals of the communication circuit 98 are of course connected to the antenna 99, but the connection lines and other details are omitted in the figure.

As shown in FIG. 1A, the communication circuit 98 can transmit the information processed by the signal processing circuit 97 to various information systems 3 in real time through the antenna 99. In the communication between the sound collector's housing 92e and the information system, wireless communication can be performed using electromagnetic waves in the 2.4GHz frequency band, with antenna power not exceeding 10mW. The information processed by the signal processing circuit 97 is transmitted to the information system in real time through the antenna 99 or other radio communication methods for visualization. Various biological information generated by the patient's heartbeat, breathing, blood flow, digestion, etc. can be obtained by the information system of relevant institutions through wireless communication and other methods.

The characteristic audible range signal (such as a biological audible range signal) is detected by the seven audible range sensor units $X_{mij}$ shown in FIG. 23A, and can be detected with sufficient signal strength even in a housing 92e with a small outer diameter. By reducing the number of seven audible range sensor units $X_{mij}$ shown in FIG. 23A, the outer diameter of the sound collector housing 92e can be further reduced, and good audibility can be obtained even with a smaller outer diameter. the material of the housing 92e is not particularly limited. For example, it can be PVC, soft plastic such as phthalate rubber, hard resin, or metal such as Al or Ti. To enhance the elastic impedance matching with the diagnostic object 1, it is best to use elastic materials such as silicone rubber for the needle tip protective film.

According to the electronic stethoscope of the first variation of the fourth embodiment, as shown in FIG. 23B, by directly contacting the tip protective film of a plurality of audible range sensor units $X_{mij}$ with the surface of the diagnostic object 1, the mechanical resonance frequency of each audible range sensor unit $X_{mij}$ is within the audible range, and the characteristic audible range signal can be effectively detected by the audible range sensor unit $X_{mij}$ within the housing 92e of the sound collection part. $X_{mij}$ can be effectively detected as a voltage signal by the audible range sensor unit $X_{mij}$ within the housing 92e of the sound collection part. In addition, if each of the multiple audible range sensor units $X_{mij}$ has an internal structure that includes an integrated insulated gate semiconductor, the detected characteristic audible range signal can be self-amplified by the electrostatic induction effect of the internal structure. Therefore, even in an environment where the characteristic auditory signal as a noise source is weak relative to the noise level, a high SN ratio can be obtained, thereby achieving a diagnosis with high objectivity and auditory accuracy.

## Second variation of the fourth embodiment

The housing 92f of the sound collector of the electronic stethoscope of the second variation of the fourth embodiment of the present disclosure has a quasi-cylindrical cross-sectional structure, as shown in FIGS. 24A and 24B, where the cross-sectional structure of the center line of the cutting circle is similar to that of a two-story structure, with two rectangles stacked together (FIG. 24B is a cross-sectional view of FIG. 24A in the direction of IIXIVB-IIXIVB). As shown in FIG. 24B, the term "pseudo-cylindrical" refers to the external shape of the space in the lower (first) floor of a two-story building, which is not a perfect rectangle but rather a concave polygon with a concave lower (bottom) surface. The space surrounded by the concave polygon on the lower side constitutes the lower storage room, while the rectangular space on the upper side (second floor) constitutes the upper storage room.

In the second variation of the fourth embodiment, the upper and lower storage rooms that constitute the two-story building are collectively referred to as "storage rooms". In fact, there may be gaps, openings, etc. in the plate-shaped parts that separate the upper and lower storage compartments shown in FIG. 24B. The structure shown in FIG. 24B is only an example. For example, a flat plate protruding in the horizontal direction perpendicular to the side wall can be placed in the middle of the side wall of the downward U-shaped structure shown in FIG. 22A to divide part of the storage cavity in the cross-sectional view shown in FIG. 24B, forming a sandwich floor. The outer skin of the coaming 92f corresponds to the outer skin of the two-story building, but unlike the structure shown in FIG. 22A to FIG. 23B, the polygonal concave surface of the first floor is inverted in the shape of a crater, making the lower storage chamber a closed space. Similarly to the structures shown in FIGS. 22A to 23B, the upper surface of the upper rectangular portion is a flat plate portion (plane portion).

In the electronic stethoscope of the second variation of the fourth embodiment, the partial crater-like concave surface of the lower surface of the concave polygon portion of the first bottom plate of the housing 92f shown in FIG. 24B becomes a "signal input surface", and the characteristic audible range signal is input to this "signal input surface". FIG. 24B schematically shows a case where, as with a conventional acoustic stethoscope, when the housing 92f is pressed against the human body as the diagnostic object 1, a portion of the surface of the human body is concave and comes into contact with the signal input surface, which is part of a crater-shaped concave surface. In FIG. 24B, the downward protruding parts on both sides of the housing 92f, such as the volcanic crater outer ring surrounding the signal input surface, correspond to the edge parts (film fixing ring) of the diaphragm stethoscope.

Then, as shown in FIG. 24B, on the signal input surface of the housing 92f, the tip protective film 34 (see FIG. 5) on the upper electrode side of the monolithically integrated multiple audible range sensor units $X_{mij}$ is exposed, and the tip protective film 34 (see FIG. 5) that can directly contact the surface of the diagnostic object 1 is exposed. The tip protective film of the audible range sensor unit $X_{mij}$ can directly contact the surface of the diagnostic object 1. In other words, the entire top protective film of each audible range sensor unit $X_{mij}$ exposed from the concave lower part of the concave polygon of the first layer of the housing 92f in contact with the diagnostic object 1 constitutes the signal input surface. The housing 92f of the sound collection part of the electronic stethoscope of the second variation of the fourth embodiment improves the reception sensitivity by directly detecting the characteristic sound signal of the tip protective film of the audible range sensor unit $X_{mij}$ as a signal input surface.

As shown in FIG. 24A, like FIG. 23A, a sensor array including audible range sensor units $X_{mp1}$-$X_{mp6}$ and $X_{mq1}$ is arranged on the semiconductor chip 41b. The area between the second circle and the third circle in FIG. 24A, as viewed from the outside, is the slope area of the outer crater ring. As shown in FIG. 24A, from the outside, there is a signal input surface inside the third circle. The signal input plane within the third circle has seven hexagonal through-holes, as shown in the seven hexagons in FIG. 24A. As shown in FIG. 24B, the semiconductor chip 41b is a flip-chip, with the top of each audible range sensor unit $X_{mp1}$-$X_{mp6}$ and $X_{mq1}$ slidably inserted into seven hexagonal through-holes. However, the space between the top of the audible range sensor units $X_{mp1}$-$X_{mp6}$ and $X_{mq1}$ and the corresponding seven through-holes is set as a confidential space, which will not be penetrated by isopropyl alcohol and soapy water during cleaning and maintenance. As shown in FIG. 16, if regular disinfection, cleaning, and other maintenance are considered in practical use, the entire lower part of the housing 92f can be covered with silicone rubber or other materials.

As in FIG. 23A, the planar patterns of the vibration cavities shown in the pattern areas of the audible range sensor units $X_{mp1}$-$X_{mp6}$ and $X_{mq1}$ shown in FIG. 24A, which are displayed with dashed inner hexagons, correspond to the planar patterns of the drum-shaped detection portions, respectively. That is to say, the seven inner hexagons shown in FIG. 24A correspond to the seven planar patterns of the drum-shaped detection portion, while the blank areas shown as three white rectangles in FIG. 24B represent the vibration cavity of the drum-shaped detection portion. However, the three white rectangles shown in FIG. 24B are only schematic diagrams, and each actual vibration cavity is a flat rectangle with a width of about 1.2-2.4mm and a height of about $2\mu m$.

As shown in FIG. 24A, the housing 92f of the electronic stethoscope according to the second variant of the fourth embodiment has a semiconductor chip 41b that integrates multiple audible range sensor units $X_{mij}$ in a single piece, corresponding to multiple drum-shaped detection portions with different diagonal diameters, arranged in the center of the housing 92f. Moreover, as shown in FIG. 23A, the diagonal diameter of the first audible range sensor unit $X_{mq1}$ including the drum-shaped detection portion is arranged at the center of the semiconductor chip 41b, which is smaller than the diagonal diameters of the second to seventh audible range sensor units $X_{mp1}$-$X_{mp6}$, which are arranged around the first audible range sensor unit $X_{mq1}$ and each have a drum-shaped detection portion.

As shown in FIG. 23A, the auditory sensor units $X_{mp1}$-$X_{mp6}$ in the monolithically integrated array are drum-shaped detection portions having a large diagonal diameter, and the auditory sensor unit $X_{mq1}$ is a drum-shaped detection portion having a small diagonal diameter, and they have different mechanical resonance frequencies from each other, as indicated by the dotted line in FIG. 24A. Therefore, by intentionally shifting the mechanical resonance frequencies of each auditory sensor unit in a monolithic integrated array composed of multiple drum-shaped detection sections, the frequency band can be expanded, thereby improving the overall receiving sensitivity of the semiconductor chip 41b.

Under the premise of confidentiality, slide the seven corresponding audible range sensor units $X_{mij}$ into the seven through-holes, and the protective coating on the tip of each audible range sensor unit $X_{mij}$ will come into contact with the diagnostic object 1. As shown in FIG. 24A, the hexagonal outline of the semiconductor chip 41b, which integrates the audible range sensor units $X_{mp1}$-$X_{mp6}$ and $X_{mq1}$, is displayed in the form of a dashed line in the shadow of the slope area presented outside the crater's outer ring, showing the area between the second and third circles when viewed from the outside.

As shown in FIGS. 24A and 24B, the hexagonal semiconductor chip 41b integrated with the audible range sensor units $X_{mp1}$-$X_{mp6}$ and $X_{mq1}$ can be compactly stored in the concave polygonal storage cavity in the lower part of the housing 92f. Therefore, according to the electronic stethoscope of the second variation of the fourth embodiment, compared to the rectangular semiconductor chip 41a shown in FIGS. 22A and 22B, the dead angle is smaller and the structure is more compact. The specific structure of each audible range sensor unit $X_{mp1}$-$X_{mp6}$ and $X_{mq1}$ is not shown, but is similar to the drum-shaped structure already described in FIG. 5, with an internal structure integrating an insulated gate semiconductor.

Then, the characteristic sound signal is input to the top electrode of the drum-shaped detection portion of each of the plurality of monolithically integrated audible range sensor units $X_{mp1}$-$X_{mp6}$ and $X_{mq1}$ through the tip protective film 34 and the top electrode protective film 26, and the upper electrode 25c vibrates to operate as a drum-shaped (capacitive) MEMS sensor (see FIG. 5). Each of the audible range sensor units $X_{mp1}$-$X_{mp6}$ and $X_{mq1}$ has a mechanical resonance frequency within the audible range, similar to the electronic stethoscopes in the first to fourth embodiments, and therefore has high receiving sensitivity (see, for example, FIG. 8). In the drum-shaped structure shown in FIG. 5, the thickness of the tip protective film 34 of the audible range sensor unit $X_{mij}$ should be thinner, which can be explained with reference to FIG. 14. The detailed structural example of the planar pattern of the first audible range sensor unit $X_{mij}$ is similar to that already described in FIG. 4, with an integrated insulated gate semiconductor in the internal structure. However, the segmentation structure shown in FIG. 4, which divides the electrode into a central lower electrode and a peripheral lower electrode, is not necessarily essential.

As shown in FIG. 24B, a pressure absorption layer 44b composed of an elastic material is stacked on top of the semiconductor chip 41b so as to contact the top surface of the lower storage cavity. The pressure absorbing layer 44b functions to absorb the pressure applied to each of the audible range sensor units $X_{mp1}$-$X_{mp6}$ and $X_{mq1}$-$X_{mq5}$ when the housing 92f is pressed against the diagnostic object 1, thereby protecting the audible range sensor units $X_{mp1}$-$X_{mp6}$ and $X_{mq1}$-$X_{mq5}$ from damage. When the seven corresponding sound sensor units $X_{mij}$ in FIG. 24B are respectively slid into the seven through holes, if the sound sensor units $X_{mp1}$-$X_{mp6}$ and $X_{mq1}$-$X_{mq5}$ are subjected to excessive pressure, the pressure absorption layer 44b can absorb the excess pressure. In addition, if excessive pressure is applied to the sound sensor units $X_{mp1}$-$X_{mp6}$ and $X_{mq1}$-$X_{mq5}$, the pressure absorption layer 44b can absorb the excessive pressure because the housing 92f is made of a flexible elastic material.

In addition, a power circuit 96, a signal processing circuit 97, and a communication circuit 98 are also provided in the upper storage chamber of the two-story building, which together with the first semiconductor chip 41b stored in the lower storage chamber form a three-dimensional hybrid integrated circuit. The power circuit 96 supplies necessary power to the seven sound sensor units $X_{mij}$, the signal processing circuit 97, and the communication circuit 98 integrated in the first semiconductor chip 41b. The signal processing circuit 97 receives signals from multiple auditory sensor units $X_{mij}$ of different sizes, converts them into A/D signals, and then performs signal processing using digital techniques, such as smoothing frequency characteristics and eliminating noise. As shown in FIG. 24B, the loop antenna 99 is located in the upper storage cavity of the two-story building. The antenna 99 may be a dipole antenna, a helical antenna, or a planar antenna. The input and output terminals of the communication circuit 98 are connected to the antenna 99, but the connection lines and other details are omitted in the figure.

Through the antenna 99, the communication circuit 98 can transmit the information signal processed by the signal processing circuit 97 to each information system 3 in real time, similar to that shown in FIG. 1A. Through antenna 99 or other wireless communication methods, the information signals processed by signal processing circuit 97 are transmitted in real-time to various information systems for visualization. Various biological information generated by the patient's heartbeat, breathing, blood flow, digestion, etc. can be obtained by the information system of relevant institutions through wireless communication and other methods.

By detecting the characteristic ranging signal through the seven audible range sensor units $X_{mij}$ shown in FIG. 24A, even if the housing 92f is small and the outer diameter is short, the characteristic ranging signal can be detected with sufficient signal strength. By reducing the number of seven audible range sensor units $X_{mij}$ shown in FIG. 24A, the outer diameter of the sound collection portion housing 92f can be further reduced, thereby achieving good audibility even with a smaller size. the material of the housing 92f is not particularly limited. For example, soft plastics (such as polyvinyl chloride), or flexible elastic materials (such as phthalate rubber or silicone rubber) that can be deformed by pressure when the housing 92f is pressed against the object to be diagnosed, can absorb excessive pressure due to the pressure absorbing layer 44b.

According to the electronic stethoscope of the second variation of the fourth embodiment, as shown in FIG. 24B, by directly contacting the tip protective film of a plurality of audible range sensor units $X_{mij}$ with the surface of the diagnostic object 1, characteristic audible frequency signals can be effectively detected as voltage signals, and each audible range sensor unit has a mechanical resonance frequency within the audible frequency range. In addition, if the internal structure of each audible range sensor unit $X_{mij}$ contains an integrated insulated gate semiconductor, the detected characteristic acoustic frequency signal can be self-amplified by the electrostatic induction effect of the internal structure. Therefore, according to the electronic stethoscope of the second variation of the fourth embodiment, even in an environment where the characteristic audible signal as the sound source is weak compared to the noise level, a high SN ratio can be achieved, thereby providing a diagnosis with high objectivity and auditory accuracy.

**Other implementation forms**

As mentioned above, the present disclosure has been described through the first to fourth embodiments, but the discussion and drawings that form part of this disclosure should not be construed as limiting the present disclosure. For those skilled in the art, various alternative embodiments, examples, and operational techniques will be apparent from this disclosure. For example, the electronic stethoscopes of the first to fourth embodiments are mainly described in the case where the planar patterns of the audible range sensors $X_{ij}$ and the audible range sensor unit $X_{mw1}$ are particularly regular hexagons, but the planar patterns of the audible range sensors $X_{ij}$ and the audible range sensor unit $X_{mw1}$ are not limited to regular hexagons. For example, they can also be regular octagons, regular dodecagons, regular tetracagons, or circles. In particular, the wavelength $\lambda$ in the audible range is very long, while the diameter of the audible range sensor $X_{ij}$ and the

audible range sensor unit $X_{mw1}$ is sufficiently small compared to the size of $\lambda/4$. Therefore, the shape of the planar pattern that must be considered in the case of ultrasonic vibration due to distortion caused by harmonics and other factors is not a major issue within the audible range.

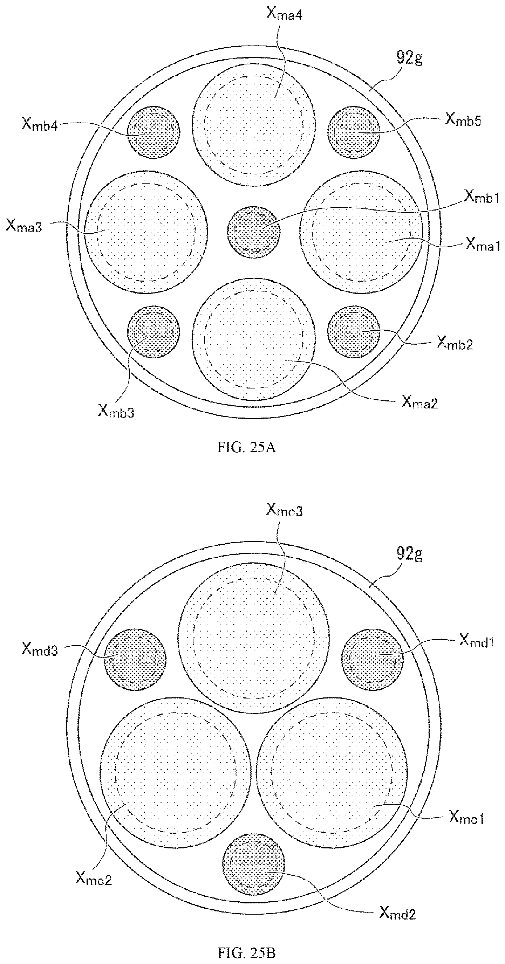

The paragraph on the electronic stethoscope of the fourth embodiment refers to the combined layout of the large-diameter audible range sensor units $X_{mv1}$-$X_{mv4}$ and the small-diameter audible range sensor units $X_{mw1}$-$X_{mw5}$, as shown in FIG. 22A. The planar patterns and planar arrangements shown in FIG. 22A and others are only examples, and are not limited to the planar patterns and planar arrangements shown in FIG. 22A and others. Each planar pattern of the audible range sensor units $X_{mv1}$-$X_{mv4}$ and $X_{mw1}$-$X_{mw5}$ may be circular. However, for the planar layout of arranging multiple drum-shaped detection parts of different diameters within the housing, it should be noted that the diameter of the drum-shaped detection part located in the central portion of the housing is smaller than the diameter of at least one drum-shaped detection part located outside the central portion, as shown in FIG. 25A. Alternatively, it should be noted that, as shown in FIG. 25B, the planar layout of the cabinet is such that there is no drum-shaped detection section in the central part of the cabinet. If the planar layout shown in FIGS. 25A and 25B is adopted, the pressure absorbing layer 44a shown in FIG. 22B and the pressure absorbing layer 44b shown in FIGS. 23B and 24B can be omitted.

In the floor plan shown in FIG. 25A, a plurality of drum-shaped detection portions having different diagonal diameters are arranged inside the housing 92g of the electronic stethoscope, and a first audible range sensor unit $X_{mb1}$ is provided at a central position of the housing 92g, and the first inner diameter thereof includes the drum-shaped detection portions. Although the semiconductor chip is omitted in the planar layout diagram of FIG. 25A, the first audible range sensor unit $X_{mb1}$ is monolithically integrated on the semiconductor chip together with the other audible range sensor units $X_{ma1}$-$X_{ma4}$ and $X_{mb2}$-$X_{mb5}$. As shown in FIG. 25A, the first audible range sensing unit $X_{mb1}$ with a first inner diameter is monolithically integrated with the outer drum-shaped detection portion around the first audible range sensing unit $X_{mp1}$, the second audible range sensing unit $X_{ma1}$, the third audible range sensing unit $X_{ma2}$, the fourth audible range sensing unit $X_{ma3}$, and the fifth audible range sensing unit $X_{ma4}$. The inner diameter of the second unit is smaller than that of the first unit. In the planar layout shown in FIG. 25A, the sixth audible range sensor unit $X_{mb2}$ having a first inner diameter defines the size of the outer drum-shaped detection area, which is monolithically integrated between the second audible range sensor unit $X_{ma1}$ and the third audible range sensor unit $X_{ma2}$.

Similarly, between the third audible range sensor unit $X_{ma2}$ and the fourth audible range sensor unit $X_{ma3}$, there is a seventh audible range sensor unit $X_{mb3}$, whose first inner diameter determines the size of the outer drum-shaped detection area; Between the fourth audible range sensor unit $X_{ma3}$ and the fifth audible range sensor unit $X_{ma4}$, there is an eighth audible range sensor unit $X_{mb4}$, whose first inner diameter determines the size of the outer drum-shaped detection area. In addition, a ninth audible range sensor unit $X_{mb5}$ is also provided between the fifth audible range sensor unit $X_{ma4}$ and the second audible range sensor unit $X_{ma1}$, with its first inner diameter located at the outer drum-shaped detection portion. However, an important feature of the planar layout shown in FIG. 25A is that when a semiconductor chip with multiple drum-shaped detection portions of different diameters is arranged within the housing 92g, the diameter of the central drum-shaped detection portion of the housing 92g is smaller than at least one diameter of the outside drum-shaped detection portion other than the center.

The detection arrays described in the first to fourth embodiments, which include multiple drum-shaped detection sections, each have a thin drum-shaped vibration cavity structure. Therefore, if the central part of the array is subjected to excessive pressure, not only will the vibration cavity collapse and lose its electroacoustic conversion sensitivity, but in the worst case, the vibration cavity will also break apart. The planar layout shown in FIG. 25A has a small diameter for the central audible range sensor unit, so even when the housing 92g is pressed against the surface of the diagnostic object 1, due to the macroscopic stress distribution, stress concentrates at the center of the semiconductor chip, making it difficult for the vibration cavity to be damaged. The planar layouts shown in FIGS. 22A, 23A, and 24A also indicate that when multiple drum-shaped detection portions of different diameters are arranged inside the housings 92d, 92e, and 92f, the diameter of the drum-shaped detection portion located in the middle of the housings 92d, 92e, and 92f is smaller than the diameter of at least one of the outer drum-shaped detection portions outside the central portion.

As shown in FIG. 25A, the drum-shaped detection portions with larger and smaller diameters have different mechanical resonance frequencies from each other. Therefore, in an array consisting of multiple drum-shaped detection parts, by intentionally shifting the respective mechanical resonance frequencies from each other, the frequency band of the electronic stethoscope can be simultaneously expanded, thereby improving the overall receiving sensitivity.

The floor plan shown in FIG. 25B also has a plurality of drum-shaped detection portions with different diagonal diameters arranged inside the housing 92g, but unlike the floor plan shown in FIG. 25A, the housing 92g does not have a drum-shaped detection portion with a smaller inner diameter (first inner diameter) in the middle, leaving a blank space. Then, the first audible range sensor unit $X_{mc1}$, the second audible range sensor unit $X_{mc2}$, and the third audible range sensor unit $X_{mc3}$ are arranged around the blank space, which includes a drum-shaped detection portion with a larger inner diameter (second inner diameter). As shown in FIG. 25A, the semiconductor chip is omitted in FIG. 25B, but the audible range sensor units $X_{mc1}$-$X_{mc3}$ are monolithically integrated on the semiconductor chip together with the audible range sensor units $X_{md1}$-$X_{md3}$. That is to say, between the first audible range sensor unit $X_{mc1}$ and the second audible range sensor unit $X_{mc2}$, the fourth audible range sensor unit $X_{md2}$ is monolithically integrated on the semiconductor chip, with a small drum-shaped detection area as its first inner diameter.

In addition, a fifth audible range sensor unit $X_{md3}$ with a first inner diameter of a small drum-shaped detection area is provided between the second audible range sensor unit $X_{mc2}$ and the third audible range sensor unit $X_{mc3}$, and a sixth audible range sensor unit $X_{md1}$ with a first inner diameter of a small drum-shaped detection area is provided between the third audible range sensor unit $X_{mc3}$ and the first audible range sensor unit $X_{mc1}$. It is located in the middle of the first inner diameter of the small drum-shaped detection area. However, an important feature of the planar layout shown in FIG. 25B is the arrangement of semiconductor chips that integrates multiple drum-shaped detection sections of different diameters on a single chip, so that when placed inside the housing 92g, the drum-shaped detection sections are not located in the middle of the housing 92g.

The planar layout shown in FIG. 25B is designed to prevent damage to the thin drum-shaped vibration cavity when the housing 92g is pressed against the surface of the diagnostic object 1, because even if stress concentration occurs at the center of the semiconductor chip due to macroscopic stress distribution, there is no audible sensor unit at the center. As shown in FIG. 25B, in addition to preventing damage, the drum-shaped detection portions with larger and smaller diameters have different mechanical resonance frequencies from each other. Therefore, in an array consisting of multiple drum-shaped detection parts, by deliberately shifting the mechanical resonance frequencies of each part, the frequency band of the electronic stethoscope can be simultaneously expanded, thereby improving the overall receiving sensitivity.

In the descriptions of the first to fourth embodiments above, the main description is of the case where the diagnostic object is a living body and the biological sound signal is used as a characteristic sound signal for the subject being examined, but this is only an example. The diagnosis object is not limited to living organisms, but can also be mechanical devices or structures such as concrete. If the diagnostic object is a mechanical device, the vibration caused by mechanical device failure or malfunction will become a characteristic sound signal. If the diagnostic object is concrete, the vibration caused by cracks or voids in the concrete will become a characteristic sound signal. If the diagnostic object is a tree, the electronic stethoscope can be attached to the bark to listen to the "heartbeat" of the trunk when it absorbs water. The "heartbeat" of trees emits characteristic audible range signals such as "buzzing", "puffing", or "go-go-go", depending on the type of tree, the point of use of the electronic stethoscope, and the health status of the tree.

In the description of FIG. 19A and other figures, it is explained that the bottom surface of the annular body composed of the housing 92c and the plane of the tip protective film of the audible range sensor $X_{ij}$ are aligned externally. However, the plane of the protective film on the lower part of the annular cover and the tip of the audible range sensor $X_{ij}$ is not strictly aligned with the outer part of the lower part of the annular cover, and the protective film on the tip of the audible range sensor $X_{ij}$ can be retracted from the lower part of the annular cover by a depth of 2mm or less to form a groove. The plane of the protective film on the lower part of the ring and the tip of the audible range sensor $X_{ij}$ is not aligned with the outer bottom surface, and the protective film on the tip of the hearing sensor $X_{ij}$ can protrude from the lower part of the ring by a height of 2mm or less, forming a protrusion.

The electronic stethoscope of the third embodiment describes the structure of the sound collection part 9c, as shown in FIG. 19B, where the adsorption assist device 95 is arranged as a suction pad at the center of the ring structure, but this is only an example. The method of fixing the sound collection component 9c on the diagnostic object 1 is not limited to suction pads, but can also be other methods, such as adhesive tape, to fix the sound collection component 9c on the diagnostic object 1. In this sense, the adsorption assist device 95 of the electronic stethoscope of the third embodiment is not a necessary condition, and if the adsorption assist device 95 is not centrally located, the ring shape shown in FIG. 18 is also not a necessary condition.

In FIG. 5, the structure is illustrated where the central lower electrode 17c of each of the audible range sensors $X_{v1}$-$X_{v4}$ and $X_{w1}$-$X_{w5}$ shares a common area with the respective gate of the corresponding insulated gate semiconductor element. However, the structure shown in FIG. 5 is not limited to the structure shown in FIG. 5. In principle, the technical concept of an audible range sensor with built-in amplification elements is different from ordinary insulated gate semiconductor elements, because even if the structure does not have a gate electrode or gate insulating film, it can operate in the same way as a vacuum MOS transistor. That is, if the upper electrode 25c is set to a first potential and the channel formation region 14 is set to a second potential, and vibration is performed through a characteristic sound signal, the capacitance between the channel formation region 14 and the upper electrode 25c changes, and charges are generated on the surface of the channel formation region 14 through electrostatic induction effect. Therefore, the height of the electron potential barrier that generates electrons in the n-p-n hook structure between the first main electrode region 15b and the second main electrode region 15a can be controlled by the vibration of the upper electrode 25c through the characteristic sound signal.

In other words, even if the structure does not have a gate electrode or a gate insulating film, the gate-to-substrate capacitance $C_{gB}$ will change due to the vibration of the characteristic sound signal through the upper electrode 25c, resulting in a change in the current between the first main electrode region 15b and the second main electrode region 15a. As mentioned above, it goes without saying that the present disclosure is not limited to the first to fourth embodiments described, and various changes are possible, which are also included in the scope of the present disclosure. Therefore, the technical scope of the present disclosure is determined only by the specific matters of the disclosure described in the claims, and it can be seen from the above description that the description of the claims is reasonable.

**Symbol Explanation**

1...diagnostic object, 3...information system, 5...display/operating unit, 6...outer ear wearing part, 7...sound tube, 9a, 9b, 9c, 9d, 9e...sound collection section, 11...common substrate, 12...gate dielectric film (the first gate dielectric film), 13...element isolation dielectric film, 14...channel formation region, 15a...the second main electrode region (drain cell) , 15b...the first main electrode region (source region), 16...the second gate insulating film, 17c...central lower electrode, 17o...peripheral lower electrode, 19...electric field enhancement layer, 20...cavity forming insulating film, 23...cavity vibrating film, 24a...the second contact plug, 24b...the first contact plug, 25a...the second surface wiring layer, 25b...the first surface wiring layer, 25c...upper electrode, 26...upper electrode protective film, 27...liquid introduction hole, 28...vibration cavity, 31c... rigid reinforcement cover, 34...tip protective film, $81_{ij}$...amplifier, 82...main processing circuit, 89...resistance adjustment circuit, 91...buffer film holder, 92a, 92b, 92c, 92d, 92e, 92f...housing, 93...buffer film, 95... adsorption assist device, 96...power circuit, 97...signal processing circuit, 98...communication circuit, 99...antenna, 701a... ear canal, 702...y-shaped tube, 703...speaker, 704...conductor.

**Claims**

1. An electronic stethoscope, comprising:

   a housing;
   an audible range sensor, which is stored inside the storage cavity of the housing, has a mechanical resonance frequency in the audible range, and has a drum shaped detection part for detecting the characteristic audible range signal;
   a signal processing circuit, which is configured inside the storage cavity to process the signal output from the audible range sensor.

2. The electronic stethoscope according to claim 1, wherein the audible range sensor detects a characteristic audible range signal from the diagnostic object in the condition that no air layer between the audible range sensor and the diagnostic object.

3. The electronic stethoscope according to claim 1, wherein

   the audible range sensor comprises
   the upper electrode, which is parallel to the signal input when there is no load, is set to be the first potential;
   the channel formation region, which is composed of a semiconductor region of a first conductivity type, is set to be the second potential, and is located at a distance further from the signal input than the distance between the upper electrode and the signal input, parallel to the signal input;
   the first and second main electrode regions of the second conductivity type, which are arranged opposite and separated from each other on the surface of the channel formation region;
   a cavity formed insulating film. which is configured between the channel forming region and the upper electrode, used for surrounding the vibrating cavity so as to provide the vibrating cavity as a closed region and supporting the perimeter of the upper electrode;
   the displacement of the upper electrode caused by the characteristic audible range signal is detected as a change in current flowing between the first and second main electrode regions.

4. The electronic stethoscope according to claim 3, further comprising:

   the audible range sensor further comprises
   a gate insulating film, which is provided on the first and second main electrode regions and on the channel formation region sandwiched between the first and second main electrode regions;
   a central lower electrode, which is composed of a conductive layer set in at least a pseudo floating state, and is located on the gate insulating film above the channel formation region sandwiched by the first and second main electrode regions.

5. The electronic stethoscope, wherein

   a housing;
   multiple audible range sensors, which are housed in the storage cavity of the housing and have different sizes;
   a signal processing circuit, which is disposed in the storage cavity and performs signal processing on the signals output from multiple audible range sensors respectively; and
   each audible range sensor has a drum-shaped detection portion with a mechanical resonance frequency in the audible range.

6. The electronic stethoscope according to claim 5, wherein in the planar layout of the plurality of audible range sensors, the diameter of the drum-shaped detection portion located at the central portion of the planar layout is at least one diameter smaller than that of the drum-shaped detection portion arranged at a position other than the central portion.

7. The electronic stethoscope according to claim 5, wherein in the planar layout of the plurality of audible range sensors, the pattern of the drum-shaped detection portion is not arranged in the central portion of the planar layout.

FIG. 1A

FIG. 1B

FIG. 2

FIG. 3A

FIG. 3B

FIG. 4

FIG. 5

FIG. 6A

FIG. 6B

FIG. 6C

FIG. 6D

FIG. 7

FIG. 8

FIG. 9A

FIG. 9B

FIG. 10A

FIG. 10B

FIG. 11A

FIG. 11B

FIG. 12

FIG. 13A

FIG. 13B

FIG. 14

FIG. 15

FIG. 16

FIG. 17

FIG. 18

FIG. 19A

| | |
|---|---|
| 96 | Power circuit |
| 97 | Signal processing circuit |
| 98 | Communi cation |

FIG. 19B

FIG. 19C

$X_{q1}$

$X_{r4}$

$X_{r1}$

9d

92c

$X_{q4}$

$X_{q2}$

$X_{r3}$

$X_{r2}$

$X_{q3}$

FIG. 20

$X_{s1}$

$X_{u3}$

$X_{t1}$

9e

92c

$X_{t3}$

$X_{u1}$

$X_{s3}$

$X_{s2}$

$X_{u2}$

$X_{t2}$

FIG. 21

FIG. 22A

FIG. 22B

FIG. 23A

Power circuit

Communication circuit

Signal processing circuit

FIG. 23B

$X_{mp5}$   $X_{mp6}$   $X_{mp1}$   $X_{mq1}$   $X_{mp4}$   $X_{mp3}$   $X_{mp2}$

41b   92f

II XIVB   II XIVB

FIG. 24A

96   98   92f

99   99

| Power circuit | Communication circuit |

Signal processing circuit   97

44b

41b

1   $X_{mp4}$   $X_{mq1}$   $X_{mp1}$

FIG. 24B

FIG. 25A

FIG. 25B

## INTERNATIONAL SEARCH REPORT

| International application No. |
| --- |
| **PCT/CN2024/088985** |

| A. | CLASSIFICATION OF SUBJECT MATTER |
| --- | --- |

A61B 7/04(2006.01)i

According to International Patent Classification (IPC) or to both national classification and IPC

| B. | FIELDS SEARCHED |
| --- | --- |

Minimum documentation searched (classification system followed by classification symbols)

IPC:A61B

Documentation searched other than minimum documentation to the extent that such documents are included in the fields searched

Electronic data base consulted during the international search (name of data base and, where practicable, search terms used)

CNTXT, ENTXTC, CJFD, VEN, CNKI: 传感器, 声, 共振, 鼓, 膜, 电容, 电极, 多, 二, 两, 三, 频率, 半导体, 放大, 麦克风, 皮肤, 接触, sensor?, voice, syntony, drum, membrane, capacitor?, electrode?, two, three, frequency, semiconductor?, amplifi+, microphone, skin, contact+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| E | CN 118177855 A (SILICON SEMICONDUCTOR TECHNOLOGY CO., LTD.) 14 June 2024 (2024-06-14)<br>claims 1-7 | 1-7 |
| X | CN 1846626 A (HEALTH & LIFE CO., LTD.) 18 October 2006 (2006-10-18)<br>description, pages 3-4, and figures 1-4 | 1-2 |
| Y | CN 1846626 A (HEALTH & LIFE CO., LTD.) 18 October 2006 (2006-10-18)<br>description, pages 3-4, and figures 1-4 | 5-7 |
| X | CN 106982399 A (NAZHIYUAN TECHNOLOGY (TANGSHAN), LLC) 25 July 2017 (2017-07-25)<br>description, paragraphs [0058]-[0062] | 1 |
| Y | CN 106982399 A (NAZHIYUAN TECHNOLOGY (TANGSHAN), LLC) 25 July 2017 (2017-07-25)<br>description, paragraphs [0058]-[0062] | 5-7 |
| X | CN 113056234 A (ECHONOUS INC.) 29 June 2021 (2021-06-29)<br>description, paragraphs [0021] and [0047] | 1 |

☑ Further documents are listed in the continuation of Box C. ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 July 2024** | **20 July 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

# EP 4 699 542 A1

## INTERNATIONAL SEARCH REPORT

| | International application No. |
|---|---|
| | **PCT/CN2024/088985** |

### C. DOCUMENTS CONSIDERED TO BE RELEVANT

| Category* | Citation of document, with indication, where appropriate, of the relevant passages | Relevant to claim No. |
|---|---|---|
| Y | CN 113056234 A (ECHONOUS INC.) 29 June 2021 (2021-06-29) description, paragraphs [0021] and [0047] | 2, 5-7 |
| Y | CN 113038870 A (ONIO AS) 25 June 2021 (2021-06-25) description, paragraph [0196] | 2 |
| Y | US 2021345939 A1 (LEVEL 42AI) 11 November 2021 (2021-11-11) description, paragraphs [0125]-[0126] | 5-7 |
| A | US 2005078533 A1 (VYSHEDSKIY, A. et al.) 14 April 2005 (2005-04-14) entire document | 1-7 |

Form PCT/ISA/210 (second sheet) (July 2022)

66

## INTERNATIONAL SEARCH REPORT
### Information on patent family members

| International application No. |
|---|
| **PCT/CN2024/088985** |

| Patent document cited in search report | | | Publication date (day/month/year) | Patent family member(s) | | | Publication date (day/month/year) |
|---|---|---|---|---|---|---|---|
| CN | 118177855 | A | 14 June 2024 | None | | | |
| CN | 1846626 | A | 18 October 2006 | None | | | |
| CN | 106982399 | A | 25 July 2017 | None | | | |
| CN | 113056234 | A | 29 June 2021 | AU | 2019356470 | A1 | 13 May 2021 |
| | | | | US | 2020107800 | A1 | 09 April 2020 |
| | | | | US | 11647977 | B2 | 16 May 2023 |
| | | | | CA | 3115470 | A1 | 16 April 2020 |
| | | | | EP | 3863521 | A1 | 18 August 2021 |
| | | | | EP | 3863521 | A4 | 18 May 2022 |
| | | | | KR | 20210106982 | A | 31 August 2021 |
| | | | | JP | 2022508629 | A | 19 January 2022 |
| | | | | WO | 2020076627 | A1 | 16 April 2020 |
| | | | | IN | 202117020271 | A | 22 October 2021 |
| | | | | HK | 40060075 | A0 | 13 May 2022 |
| | | | | RU | 2021112458 | A | 14 November 2022 |
| CN | 113038870 | A | 25 June 2021 | KR | 20210070324 | A | 14 June 2021 |
| | | | | EP | 3860444 | A1 | 11 August 2021 |
| | | | | EP | 3860444 | B1 | 16 November 2022 |
| | | | | JP | 2022504314 | A | 13 January 2022 |
| | | | | JP | 7315669 | B2 | 26 July 2023 |
| | | | | US | 2022000376 | A1 | 06 January 2022 |
| | | | | WO | 2020071926 | A1 | 09 April 2020 |
| | | | | NO | 201801283 | A | 06 April 2020 |
| | | | | GB | 2577726 | A | 08 April 2020 |
| | | | | GB | 2577726 | B | 11 August 2021 |
| | | | | IN | 202117019729 | A | 21 January 2022 |
| US | 2021345939 | A1 | 11 November 2021 | US | 2022103922 | A1 | 31 March 2022 |
| | | | | CA | 3178244 | A1 | 11 November 2021 |
| | | | | EP | 4146071 | A1 | 15 March 2023 |
| | | | | EP | 4146071 | A4 | 01 May 2024 |
| | | | | WO | 2021224888 | A1 | 11 November 2021 |
| | | | | US | 11240579 | B2 | 01 February 2022 |
| | | | | JP | 2023526564 | A | 21 June 2023 |
| US | 2005078533 | A1 | 14 April 2005 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2024088985 W
- JP 2023070122 B

- JP 53030187 U

**Non-patent literature cited in the description**

- The Transmission Characteristics of Stethoscopes. **HASHIGUCHI et al.** Research Report of the Faculty of Engineering. Yamanashi University, December 1986, 45-49